(19) Europäisches Patentamt

European Patent Office

Office européen des brevets

(11) EP 0 987 035 A2

(12) **EUROPEAN PATENT APPLICATION**

(43) Date of publication:
22.03.2000 Bulletin 2000/12

(51) Int Cl.⁷: **A61M 1/14**, A61M 1/36, A61M 1/16

(21) Application number: 99115092.1

(22) Date of filing: 06.08.1999

| | |
|---|---|
| (84) Designated Contracting States:<br>**AT BE CH CY DE DK ES FI FR GB GR IE IT LI LU MC NL PT SE**<br>Designated Extension States:<br>**AL LT LV MK RO SI**<br><br>(30) Priority: 07.08.1998 JP 23649298<br>14.09.1998 JP 27941898<br>17.11.1998 JP 34489098<br><br>(71) Applicant: **TERUMO KABUSHIKI KAISHA**<br>**Tokyo 151 (JP)** | (72) Inventors:<br>• **Ogihara, Mitsuaki**<br>**Nakai-machi, Ashigarakami-gun, Kanagawa (JP)**<br>• **Takeuchi, Kazuhiko**<br>**Fujinomiya-shi, Shizuoka (JP)**<br><br>(74) Representative: **Casalonga, Axel et al**<br>**BUREAU D.A. CASALONGA - JOSSE**<br>**Morassistrasse 8**<br>**80469 München (DE)** |

(54) **Blood oxygenating apparatus having blood delivery mechanism-equipped blood reservoir, bubble remover for blood oxygenating circuit, bubble remover-equipped blood oxygenating circuit and blood delivery mechanism-equipped blood reservoir**

(57)     A blood oxygenating apparatus (1) comprises a blood delivery mechanism-equipped blood reservoir including a blood storing portion (10); a blood delivery mechanism having blood reserving members (3a,3b) having delivery blood reserving portions (30a,30b) communicating with the blood storing portion (10) and reserving blood in an amount proportional to an amount of blood stored in the blood storing portion (10) when the amount of the blood stored in the blood storing portion (10) becomes equal to or less than a predetermined value and capable of flowing out blood stored in the delivery blood reserving portions (30a,30b) when the delivery blood reserving portions (30a,30b) are pressed from outside; and a heat exchange function-provided oxygenator (5) connected with a blood outlet port of the blood delivery mechanism-equipped blood reservoir (2). The blood outlet port of the oxygenator (5) communicates with the vicinity of an upper end of a blood chamber provided inside the oxygenator (5).

FIG. 1

**Description**

BACKGROUND OF THE INVENTION

The present invention relates to a blood oxygenating apparatus provided with a delivery blood reservoir and an oxygenator having a function of temporarily storing blood in an extracorporeal blood circulation circuit and delivering stored blood to a patient.

In recent years, heart surgery often uses an extracorporeal blood circulation circuit having incorporated therein an oxygenator for removing carbon dioxide from blood and adding oxygen to blood instead of the living lung. The extracorporeal circulation circuit is to drain blood from the patient's vein, subject the blood to gas exchange in the oxygenator, and return the blood to the patient's artery. The extracorporeal circulation circuit also includes a cardiotomy line for sucking in blood from the operation area, removing foreign matter therefrom, and returning the blood.

In general, the extracorporeal circulation circuit with an oxygenator includes a blood reservoir for temporarily storing blood drained from the patient and a cardiotomy reservoir for filtering blood sucked from the operation area and temporarily storing the blood. The blood reservoir and cardiotomy reservoir serve a buffering function of adjusting the amount of blood in the circuit and maintaining a constant amount of blood to be returned to the patient.

Let it be supposed that a considerably large amount of air is introduced into the conventional oxygenator. To prevent the air from flowing to the downstream side thereof, a conventional blood oxygenating apparatus has a blood outlet port provided at a lower portion (downstream from the center of a blood chamber) of the oxygenator to trap the air in the oxygenator.

Although the conventional blood oxygenating apparatus is safe because the blood outlet port is provided at the lower portion (downstream from the center of the blood chamber) of the oxygenator, it is difficult to remove air present upstream from a blood outlet port-connected port in a priming time, which causes the priming time to take long. Another problem is that residual air in the priming operation flows out when blood circulates.

Therefore, it is a first object of the present invention to provide a blood oxygenating apparatus, having a blood delivery mechanism-equipped blood reservoir, which is capable of performing a priming work very easily by connecting a blood outlet port to an upper portion of a blood chamber of an oxygenator and which has a function of not delivering air into the oxygenator during a blood delivery time so that the blood oxygenating apparatus can be used safely even though the oxygenator is not provided with an air trap mechanism.

The present invention also relates to a bubble remover which separate and removes bubbles from blood in a blood oxygenating circuit provided with an oxygenator; and also relates to a blood oxygenating circuit apparatus having the bubble remover.

Heart surgery often uses an extracorporeal blood circulation circuit having incorporated therein an oxygenator for removing carbon dioxide from blood and adding oxygen to blood instead of the living lung. The extracorporeal circulation circuit is to store blood drained from the patient's vein in a vein reservoir, subject the blood to gas exchange in the oxygenator, and return the blood to the patient's artery. A delivery means (pump) is provided between the vein reservoir and the oxygenator.

An air filter is accommodated in the blood oxygenating circuit of this type to remove bubbles or foreign matter which have been contained therein. More specifically, an artery filter (bubble remover) having the function of an air chamber is provided at a position in the downstream side of the blood oxygenating circuit such that the position is immediately before the position at which the blood is returned to the patient's artery. It is necessary for the bubble remover to have the air chamber function, in consideration of the capacity of circulation blood and the amount of bubble which may be contained in the blood oxygenating circuit Therefore, it is necessary for the bubble remover to have an internal capacity of about 200 ml for adults. The use of such a bubble remover causes the entire blood oxygenating circuit to have an increased priming amount (extracorporeal flow-out amount of blood) of about 200 ml. It is demanded that heart surgery is made by using a possible smallest transfusion amount. To this end, the priming amount (extracorporeal flow-out amount of blood) is reduced to a possible smallest transfusion amount.

Therefore, it is a second object of the present invention to provide a bubble remover for use in a blood oxygenating circuit which displays a high air chamber function, has a small pressure loss when it is used for adults, secures a sufficient amount of flow rate, and has a small priming amount and to provide a blood oxygenating circuit apparatus having the bubble remover.

There is a situation that the volume of blood in the blood delivery mechanism-equipped blood reservoir becomes zero during extracorporeal blood circulation. If the blood feed pump continues to operate, air can be fed into the extracorporeal circulation circuit To avoid such inconvenience, the blood delivery mechanism-equipped blood reservoir is provided with a level sensor for monitoring the volume of blood in the tank. When the sensor detects that the volume of blood in the blood delivery mechanism-equipped blood reservoir is below a predetermined value, the feed pump is interrupted to stop blood delivery action until the volume of blood in the blood delivery mechanism-equipped blood reservoir is restored. The temporary interruption of blood delivery, however, is undesirable because it causes blood stagnation throughout the circuit including the oxygenator. Therefore, there is a need to provide a delivery blood storing mechanism-equipped blood reservoir capable of changing the amount of blood to be delivered in accordance with the amount of blood stored in a blood storing portion if the amount of blood stored therein becomes equal to or less than a predetermined value.

Lately, the conveyance of blood into the blood reservoir and the drainage of blood from a patient are often performed by a method using a suction device (negative pressure applying device) connected to the blood reservoir. Suction drainage of blood is used in the case where a sufficient drainage amount cannot be secured, namely, in the case where only a fine drainage catheter such as MICS (thoracotomy minimally invasive surgery) is used or a long head cannot be obtained between a patient and an oxygenator. When this device is operated, the pressure in the delivery blood storing mechanism-equipped blood reservoir becomes negative, and the negative pressure affects the interior of the delivery blood reserving member. That is, the interior of the delivery blood reserving member becomes exposed to a negative pressure due to the operation of the suction device (negative pressure applying device), while receiving the atmospheric pressure on the outside. Therefore, the delivery blood reserving member is pressed by a differential pressure between the inside and outside pressures, so that the liquid surface-sensitive (pressure-sensitive, pre-load-sensitive) function of the delivery blood reserving member may be impeded.

Therefore, it is a third object of the present invention to provide a blood delivery mechanism-equipped blood reservoir that will not impede a liquid surface-sensitive (pressure-sensitive, pre-load-sensitive) function of a delivery blood reserving member even if a suction device (negative pressure applying device) is connected to the delivery blood storing mechanism-equipped blood reservoir.

SUMMARY OF THE INVENTION

In a first aspect, the present invention provides a blood oxygenating apparatus having a blood delivery mechanism-equipped blood reservoir that comprises a blood delivery mechanism-equipped blood reservoir having a blood storing portion and a blood reserving member having a delivery blood reserving portion communicating with said blood storing portion and reserving blood in an amount proportional to an amount of blood stored in said blood storing portion when the amount of said blood stored in blood storing portion becomes equal to or less than a predetermined value and capable of flowing out blood stored in said delivery blood reserving portion when said delivery blood reserving portion is pressed from outside; and an oxygenator connected with a blood outlet port of said blood delivery mechanism-equipped blood reservoir, wherein said blood outlet port of said oxygenator communicates with an upper end or its proximity of a blood chamber provided inside said oxygenator.

In a second aspect, the present invention provides a bubble remover for a blood oxygenating circuit which is used by locating it downstream from an oxygenator of said blood oxygenating circuit comprising a blood delivery mechanism-equipped blood reservoir having a blood storing portion and a blood reserving member having a delivery blood reserving portion communicating with said blood storing portion and reserving blood in an amount proportional to an amount of blood stored in said blood storing portion when the amount of said blood stored in blood storing portion becomes equal to or less than a predetermined value and capable of flowing out blood stored in said delivery blood reserving portion when said delivery blood reserving portion is pressed from outside; and said oxygenator connected with a blood outlet port of said blood delivery mechanism-equipped blood reservoir, wherein said bubble remover has the following relationship between an internal capacity (ml) and a maximum use flow (L/min):

$$\text{Internal capacity (ml)/Maximum use flow (L/min)} \leqq 15.$$

In a second aspect, the present invention provides a blood oxygenating circuit apparatus that comprises a blood delivery mechanism-equipped blood reservoir having a blood storing portion and a blood reserving member having a delivery blood reserving portion communicating with said blood storing portion and reserving blood in an amount proportional to an amount of blood stored in said blood storing portion when the amount of said blood stored in blood storing portion becomes equal to or less than a predetermined value and capable of flowing out blood stored in said delivery blood reserving portion when said delivery blood reserving portion is pressed from outside; an oxygenator connected with a blood outlet port of said blood delivery mechanism-equipped blood reservoir, and a bubble remover located downstream from said oxygenator and having a relationship between an internal capacity (ml) and a maximum use flow (L/min):

$$\text{Internal capacity (ml)/Maximum use flow rate (L/min)} \leqq 15.$$

In a third aspect, the present invention provides a blood delivery mechanism-equipped blood reservoir that comprises a blood storing portion; and a blood delivery mechanism including a delivery blood reserving portion communicating with said blood storing portion and reserving blood in an amount proportional to an amount of blood stored in said blood storing portion when the amount of said blood stored in said blood storing portion becomes equal to or less than a predetermined value and capable of flowing out blood stored in said delivery blood reserving portion when said delivery blood reserving portion is pressed from the outside and a blood delivery drive unit operating when blood in said delivery blood reserving portion is delivered, wherein said blood delivery mechanism-equipped blood reservoir can be connected with a sucking means for sucking an interior of said blood storing portion; said blood delivery mechanism-equipped blood reservoir further comprising: a communication passage communicating an upper portion of an interior of said blood storing portion with a space formed inside said blood delivery mechanism and formed between said delivery blood reserving portion and said blood delivering drive unit; and a communication mode selection function making it possible to select communication between said space of said blood delivery mechanism and said upper portion of said interior of said blood storing portion or communication between said space of said blood delivery mechanism and outside air.

## BRIEF DESCRIPTION OF THE DRAWINGS

The foregoing and further objects, features and advantages of the present invention will become apparent from the following description of preferred embodiments with reference to the accompanying drawings, wherein like numerals are used to represent like elements and wherein:

Fig. 1 is a front elevation of a blood oxygenating apparatus having a blood delivery mechanism-equipped blood reservoir according to the present invention.

Fig. 2 is a side view of the blood oxygenating apparatus shown in Fig. 1.

Fig. 3 is an explanatory view for explaining the internal construction of the blood oxygenating apparatus shown in Figs. 1 and 2.

Fig. 4 is an enlarged sectional view of the neighborhood of the blood delivery mechanism of the blood delivery mechanism-equipped blood reservoir shown in Fig. 3 and the neighborhood of a lower portion of a blood storing portion thereof, wherein a flexible diaphragm 42a, 42b is not pressed (blood is not being delivered).

Fig. 5 is a front elevation of a blood delivery mechanism portion of the blood oxygenating apparatus shown in Figs. 1 and 2.

Fig. 6 is a sectional view of a blood delivery mechanism portion of the blood oxygenating apparatus shown in Fig. 5.

Fig. 7 is a front view of a bubble remover for use in a blood oxygenating circuit according to an embodiment of the present invention.

Fig. 8 is a sectional view of the bubble remover shown in Fig. 7.

Fig. 9 is a cross-sectional view taken along lines B-B in Fig. 8.

Fig. 10 is a concept view of a blood oxygenating circuit apparatus according to an embodiment of the present invention.

Fig. 11 is a right side view of a blood delivery mechanism-equipped blood reservoir according to an embodiment of the present invention.

Fig. 12 is a front elevation of the blood delivery mechanism-equipped blood reservoir shown in Fig. 11.

Fig. 13 is a back elevation view of the blood delivery mechanism-equipped blood reservoir shown in Fig. 11.

Fig. 14 is a plan view of the blood delivery mechanism-equipped blood reservoir shown in Fig. 11.

Fig. 15 is an explanatory view for explaining the internal construction of the blood delivery mechanism-equipped blood reservoir shown in Figs. 11 through 14.

Fig. 16 is an enlarged sectional view of the vicinity of the blood delivery mechanism-equipped blood reservoir shown in Fig. 15 and the vicinity of a blood storing portion.

Fig. 17 is a front view of a blood delivery mechanism portion of the blood delivery mechanism-equipped blood reservoir shown in Figs. 11 through 14.

Fig. 18 is a sectional view of the blood delivery mechanism portion of the blood delivery mechanism-equipped blood reservoir shown in Fig. 17.

Fig. 19 is a front view of a first cap (used in non-sucking time) serving as a communication mode selection function.

Fig. 20 is a bottom view of the first cap (used in non-sucking time) shown in Fig. 19.

Fig. 21 is an enlarged sectional view of the neighborhood of a communication passage of a blood delivery mechanism-equipped blood reservoir in the state where the first cap (used in non-sucking time) is installed.

Fig. 22 is a front view of a second cap (used in sucking time) serving as a communication mode selection function.

Fig. 23 is a plan view of the second cap (used in sucking time) shown in Fig. 22.

Fig. 24 is a sectional view taken along a line D-D of Fig. 23.

Fig. 25 is an enlarged sectional view of the neighborhood of a communication passage of a blood delivery mechanism-equipped blood reservoir in the state where the second cap (used in sucking time) is installed.

Fig. 26 is an enlarged sectional view of the neighborhood of a communication passage of a blood delivery mechanism-equipped blood reservoir in the state where a first cap (used in non-sucking time) according to another embodiment is installed.

Fig. 27 is a sectional view of a communication mode selection function of a blood delivery mechanism-equipped blood reservoir at a non-sucking time according to another embodiment.

Fig. 28 is a sectional view of a communication mode selection function of a blood delivery mechanism-equipped blood reservoir at a sucking time according to another embodiment.

Fig. 29 is a sectional view of a communication mode selection function of a blood delivery mechanism-equipped blood reservoir at a sucking time according to another embodiment.

Fig. 30 is a sectional view of a blood delivery mechanism-equipped blood reservoir according to another embodiment of the present invention.

Fig. 31 shows a state where a communication mode selection function to be used for a blood delivery mechanism-equipped blood reservoir, shown in Fig. 30, at a non-sucking time.

Fig. 32 shows a state where a communication mode selection function to be used for a blood delivery mechanism-equipped blood reservoir, shown in Fig. 30, at a sucking time according to another embodiment

Fig. 33 shows a state where a communication mode selection function to be used for a blood delivery mechanism-equipped blood reservoir, shown in Fig. 30, at a non-sucking time according to another embodiment.

Fig. 34 shows the communication mode selection function, shown in Fig. 33, at a sucking time.

DETAILED DESCRIPTION

A blood oxygenating apparatus having blood delivery mechanism-equipped blood reservoir of the present invention will be described in detail below with reference to the drawings.

A blood oxygenating apparatus (pump oxygenator apparatus)1 of the present invention comprises a blood delivery mechanism-equipped blood reservoir including a blood storing portion 10; a blood delivery mechanism having blood reserving members 3a, 3b having delivery blood reserving portions 30a, 30b, respectively communicating with the blood storing portion 10 and reserving blood in an amount proportional to an amount of blood stored in the blood storing portion 10 when the amount of the blood stored in the blood storing portion 10 becomes equal to or less than a predetermined value and capable of flowing out blood stored in the delivery blood reserving portions 30a, 30b when the delivery blood reserving portions 30a, 30b are pressed from outside; and a heat exchange function-provided oxygenator 5 connected with a blood outlet port of the blood delivery mechanism-equipped blood reservoir 2. The blood outlet port of the oxygenator 5 communicates with the vicinity of an upper end of a blood chamber provided inside the oxygenator 5. Preferably, the blood outlet port communicates with an uppermost end of the blood chamber.

More specifically, the blood oxygenating apparatus 1 includes the blood storing portion 10 storing a specified minimum reserving amount of blood; the blood reservoir 2 including the blood delivery mechanism having the blood reserving members 3a, 3b provided with the delivery blood reserving portions 30a, 30b, respectively communicating with the blood storing portion 10 and reserving blood in the amount proportional to the amount of blood stored in the blood storing portion 10 when the amount of the blood stored in the blood storing portion 10 becomes equal to or less than the predetermined value and capable of flowing out blood stored in the delivery blood reserving portions 30a, 30b when the delivery blood reserving portions 30a, 30b are pressed from outside; and the heat exchange function-provided oxygenator 5 connected with the blood outlet port of the blood delivery mechanism-equipped blood reservoir 2. The upper end of the blood chamber provided inside the oxygenator 5 is located at a position lower than the level of the surface of blood in the state where the blood storing portion 10 reserves the specified minimum amount of blood. The blood outlet port of the oxygenator 5 communicates with the vicinity of the upper end of the blood chamber provided inside the oxygenator 5. Preferably, the blood outlet port communicates with the uppermost end of the blood chamber.

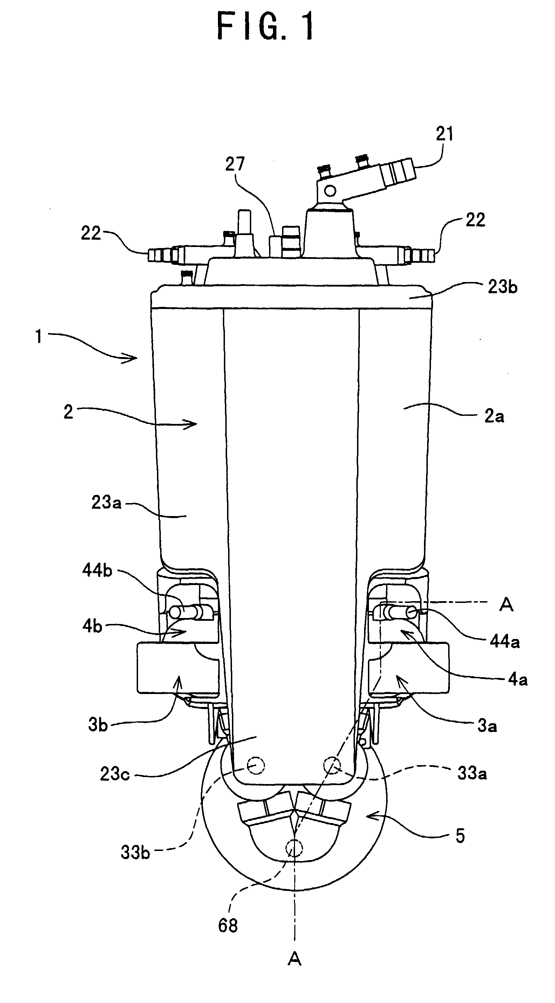

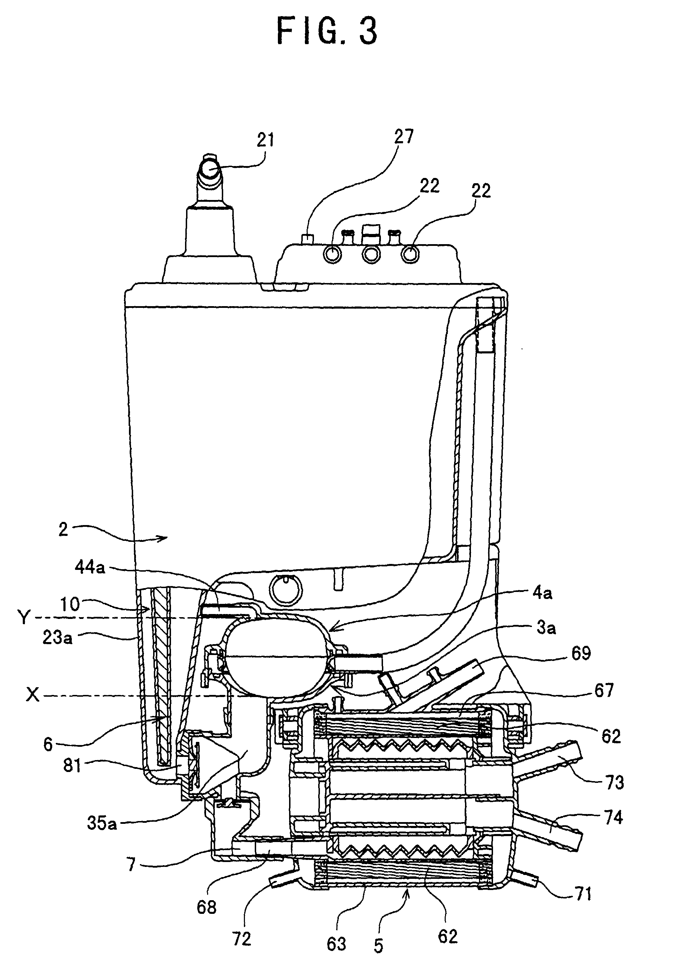

Fig. 3 shows the heat exchange function-provided oxygenator 5 and the blood delivery mechanism by cutting them along a center line and a broken line A-A of Fig. 1, respectively.

The blood delivery mechanism-equipped blood reservoir 2 includes a blood reservoir portion 2a; delivery blood reserving members 3a, 3b; and blood passages 35a, 35b communicating the blood reservoir portion 2a and each of the delivery blood reserving members 3a, 3b with each other. The oxygenator 5 is installed on the blood reservoir portion 2a by hanging the former from the bottom surface of the latter. A blood inlet port 68 of the oxygenator 5 is connected with a blood discharge opening 7 of the blood reservoir 2.

The blood storing portion 10 serves as a blood storing space in the blood reservoir portion 2a. In the blood reservoir 2, when the surface of the blood in the blood reservoir 2 is at a level below the upper end of the diaphragms 42a, 42b as well as a horizontal position (Y), i.e., when the amount of the blood stored in the blood storing portion 10 is less than the predetermined value, the blood reserving members 3a, 3b store an amount of blood proportional to the amount of the blood stored in the blood storing portion 10. The blood in the blood reserving members 3a, 3b can be flowed out intermittently by pressing the delivery blood storing members 3a, 3b at the side of the diaphragms 42a, 42b thereof from the outside. Therefore, when a remaining amount of the blood stored in the blood storing portion 10 becomes small, the blood is fed in a small amount in proportion to the remaining amount of the blood stored in the blood storing portion 10. That is, even though the remaining amount of the blood in the blood storing portion 10 becomes small, the delivery of a small amount of blood is maintained. Thus, even though the remaining amount of the blood in the blood storing portion 10 becomes small, it is unnecessary to suspend the blood delivery operation and possible to prevent the blood from stagnating in the extracorporeal blood circulation circuit The blood reservoir stores the specified minimum reserving blood amount That is, when the surface of the blood in the blood storing portion 10 is located at the lower end of the delivery blood storing members 3a, 3b, namely, at the lowermost end (position shown with X of Fig. 3) of the diaphragms 42a, 42b, the blood is not delivered. In other words, it occurs that the surface of the blood in the blood storing portion 10 drops to the position shown with X of Fig. 3.

The drop of the blood surface to the position shown with X means that the part above the position shown with X forms an air layer. The upper end of the blood chamber inside the oxygenator 5 is lower than the level (X) in the state where the specified minimum reserving blood amount is reserved in the blood storing portion 10. Therefore, air is prevented from flowing into the blood chamber of the oxygenator 5. When the amount of blood in the blood reservoir is restored to the predetermined amount, the delivery of blood can be started immediately without feeding air. A blood outlet port 69 of the oxygenator 5 communicates with the upper end of the blood chamber 67 provided inside the oxygenator 5. That is, the blood outlet port 69 is formed at an upper portion of the oxygenator 5.

Let it be supposed that a considerably large amount of air is introduced into a conventional oxygenator. To prevent the air from flowing to the downstream side thereof, a conventional blood oxygenating apparatus has a blood outlet port provided at a lower portion (downstream from the center of a blood chamber) of the oxygenator to trap the air in the oxygenator. Thus, the conventional blood oxygenating apparatus is safe in this respect But it is difficult to remove air present upstream from a blood outlet port-connected port in a priming time, which causes the priming time to take long. Another problem is that residual air in the priming operation flows out when blood circulates.

Unlike the conventional blood oxygenating apparatus, in the blood oxygenating apparatus 1 of the present invention, the blood outlet port 69 of the oxygenator 5 communicates with the upper end of the blood chamber 67 provided inside the oxygenator 5. That is, in performing priming operation of the blood chamber inside the oxygenator 5, it is very easy to replace air inside the blood chamber with a fluid by using the blood delivery mechanism to be used for an extracorporeal blood circulation. Further, unlike the conventional blood delivery mechanism, the blood delivery mechanism which is used in the blood oxygenating apparatus 1 of the present invention does not deliver air to the oxygenator 5 when the amount of the blood in the blood storing portion decreases. Therefore, it is unnecessary to provide the oxygenator with an air trap mechanism. Moreover, because the blood outlet port 69 is provided on the upper portion of the oxygenator 5, it is easy to perform an operation of connecting tubes of the extracorporeal blood circulation circuit to the blood outlet port 69 and possible to use short tubes, which reduces a priming volume. Furthermore, it is possible to use a small loop consisting of upwardly extended tubes. Thus, the tubes do not interfere with medical appliances or the like in an operation.

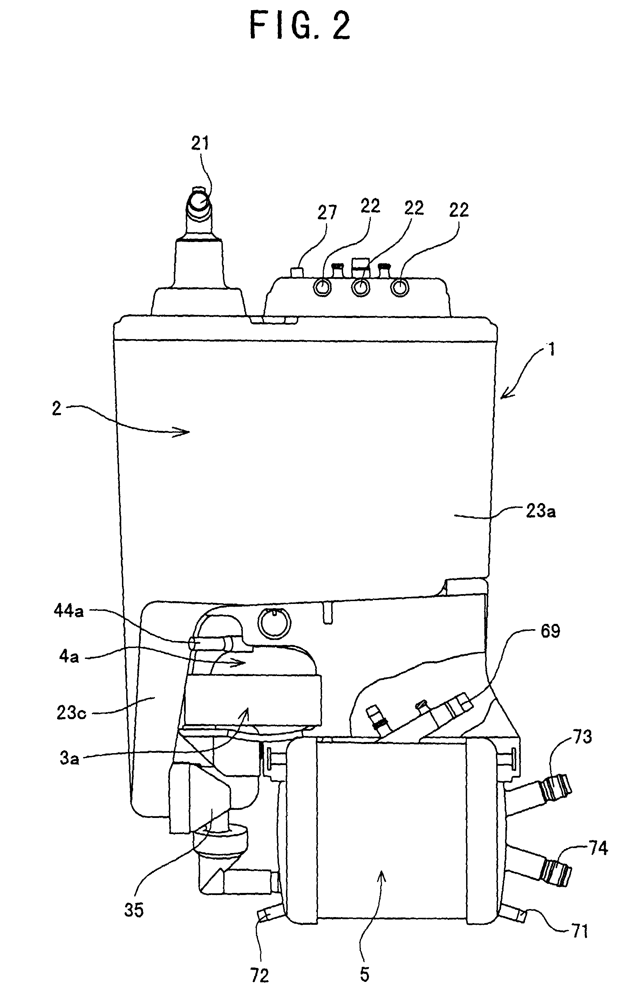

As shown in Figs. 1 through 3, the blood reservoir portion 2a has a housing constructed of a housing body 23a and a lid body 23b both made of hard resin. The lid body 23b covering an opening formed at an upper portion of the housing body 23a is fitted into the upper end of the housing body 23a. As shown in Figs. 1 and 2, the lid body 23b has blood inlet ports 21, 22 and an air discharge opening 27. The blood inlet port 22 is connected with a cardiotomy line delivering blood fed from an operation field. The blood inlet port 21 is connected with a blood drainage line delivering blood fed from a blood drainage cannula inserted into a patient's heart ascending/descending veins.

The housing body 23a of the blood reservoir portion 2a and the lid body 23b thereof may be formed of any desired resin, for example, polycarbonate, acrylic resin, polyethylene terephthalate, polyethylene, polypropylene, polystyrene, polyvinyl chloride, acryl-styrene copolymers, and acryl-butadiene-styrene copolymers. Polycarbonate, acrylic resin, polystyrene, and polyvinyl chloride are especially preferred.

In the housing body 23a, there are provided a blood filter (venous blood filter) 6 serving as an anti-foaming member and a foam-removing member for filtering blood introduced into the housing body 23a from the blood inlet port 21; and a cardiotomy blood filter (not shown) for filtering blood introduced thereinto from the blood inlet port 22. The lower end of the blood inlet port 21 extends into the blood storing portion 10 and is positioned inside the blood filter 6. All blood introduced into the housing body 23a from the blood inlet port 21 flows into the blood storing portion 10 through the blood filter 6. Therefore, it is possible to prevent the blood introduced into the housing body 23a from the blood inlet port 21 from directly dripping to the surface of the blood stored inside the blood storing portion 10. More specifically, the blood filter 6 is fixed to an inner surface of the lid body 23b and extends downward toward the lower end surface of the blood storing portion 10 such that the blood filter 6 encloses a projection portion, of the blood inlet port 21, projecting into the blood storing portion 10.

The housing body 23a has a projection portion 23c projecting downward. The lower end of the blood filter 6 extends to a position in the vicinity of the lower end of the projection portion 23c. In the state in which the specified minimum reserving amount of blood is reserved in the blood storing portion 10, the lower end of the blood filter 6 is positioned below the level of the blood surface shown with X. That is, the lower end of the blood filter 6 is positioned below the lower ends of the delivery blood reserving portions 30a, 30b of the blood reserving members 3a, 3b. In the state in which the specified minimum reserving amount of blood is reserved in the blood storing portion 10, the lower end of the blood filter 6 is positioned below the surface of the blood. Therefore, in the state where the amount of the blood stored in the blood storing portion 10 is reduced to a value below the predetermined value and the blood reserving members 3a, 3b are placed in the state where they display the function of reserving blood in the amount proportional to the amount of the blood stored in the blood storing portion 10, the blood introduced into the blood storing portion 10 does not bubble. This is because the lower end of the blood filter 6 is located below the surface of the blood in the blood storing portion 10. When the amount of the blood stored in the blood storing portion 10 increases and returns to a value above the predetermined value, the blood introduced into the blood storing portion 10 hardly bubbles because the lower end of the blood filter 6 is positioned below the surface of the blood in the blood storing portion 10.

The interior of the blood filter 6 is filled with an anti-foaming member 6a or a bubble-removing member for removing air bubbles that may be contained in incoming blood. To form the bubble eliminating member 6a or the bubble removing member, various porous materials may suitably be used, including, but not limited to, foamed materials such as polyurethane foam, polyethylene foam, polypropylene foam, polystyrene foam and the like, mesh, woven fabrics, non-woven fabrics, porous ceramics, sintered materials of resin and the like. Materials having relatively small air passage resistance (pressure loss) are particularly preferred. If a material having less air passage resistance, for example, a foamed material such as polyurethane foam or the like or other porous materials are used, the pore size thereof is favorably approximately within the range of 20 µm to 10 mm and, more favorably, within the range of 50 µm to 5 mm.

It is preferable that the anti-foaming member 6a carries an anti-foaming agent having a function of breaking bubbles when the anti-foaming agent contacts the bubbles. A preferred bubble eliminating agent is silicone (silica-compounded type, oil type or the like). Examples of the method for the bubble eliminating member 6a to carry the bubble eliminating agent include, but are not limited to, a method wherein a bubble eliminating member is dipped in a liquid containing a bubble eliminating agent and a method wherein a liquid containing a bubble eliminating agent is applied or sprayed to a bubble eliminating member, and then dried (for example, at 30°C for 180 minutes).

The exterior of the anti-foaming member 6a is covered with a filter member 6b. The filter member 6b removes foreign matter or air bubbles from blood. A preferred material for the filter member 6b is a porous material having a sufficiently high blood permeability. Examples of the porous material include mesh (net) materials, woven fabrics, non-woven fabrics and the like. Such materials may be used alone or in any desired combination (particularly, in the form of a laminate). It is preferred to use one or a combination of two or more of macromolecular materials that include polyesters such as PET, PBT or the like, polyolefins such as polyethylene and polypropylene, rayon, Tetron®, nylon (polyamide), and the like. It is also possible to use metallic materials such as aluminum, stainless steel or the like. Particularly preferred are polyesters, polypropylene, polyethylene and stainless steel. The opening size of a mesh used for the filter member 6b is preferably about 15-300 µm and, more favorably, about 20-200 µm. It is also preferred to subject the filter member 6b to a plasma treatment or a hydrophilic treatment such as coating with a hydrophilic macromolecular material or the like, in order to improve blood permeability.

The interior of the blood reservoir portion 2a forms the blood storing portion 10 for temporarily reserving blood, as shown in Fig. 3. The capacity of the blood storing portion 10 is not specified. Normally, the capacity of the blood storing portion 10 is about 3,000 to 5,000 ml for adults and about 1,000 to 2,500 ml for children. The housing main body 23a is preferably substantially transparent or semi-transparent so that the amount and conditions of blood reserved therein may be readily observed. The downward projection portion 23c is a narrow portion with a reduced sectional area, whereby when the amount of blood reserved decreases, the amount of blood reserved or a change in the blood amount can be correctly and readily read. The blood reservoir portion 2a may be a soft type blood reservoir portion formed from a soft resin. In this case, the blood reservoir portion 2a is a closed-type blood reservoir portion.

A blood incoming portion of a body 35 of a blood delivery instrument 3 provided with a joint portion 81 to the blood reservoir portion 2a is connected to a lower portion of the blood reservoir portion 2a, as shown in Fig. 3. As shown in Figs. 3 through 6, the blood delivery instrument 3 includes the blood storing portion 10; the blood reserving members 3a, 3b; the blood flow passage portions 35a, 35b formed inside the body 35 and communicating with the blood reserving members 3a, 3b, respectively; and the blood discharge opening 7.

The first blood flow passage portion 35a formed inside the body 35 communicates with only the first delivery blood reserving member 3a. The second blood flow passage portion 35b formed inside the body 35 communicates with only the second delivery blood reserving member 3b. Disposed at boundary sites between the blood storing portion 10 and the blood flow passage portions 35a, 35b are first check valves 33a, 33b that allow flow of blood from the blood storing portion 10 toward the blood flow passage portions 35a, 35b (namely, toward the blood delivery instrument 3) and restricts (prevents) the reverse flow of blood.

The first check valves 33a, 33b function as passage control members for blocking the communication between the blood storing portion 10 and the delivery blood reserving members 3a, 3b during the operation of the blood delivering drive units 4 as described below. The blood delivery instrument 3 is provided with the blood discharge opening 7 for communication with the blood flow passage portions 35a, 35b. Provided near the blood discharge opening 7 are second check valves 34a, 34b that allow blood flow toward a side downstream from the blood flow passage portions 35a, 35b (that is, the side at the blood reserving member 3) and restricts the reverse flow of blood. The second check valves 34a, 34b function as passage control members for blocking reverse blood flow from the downstream side toward the delivery blood reserving members 3a, 3b (that is, into the blood flow passage portions 35a, 35b) when the blood delivering drive unit 4 is not operated.

Each check valve 33, 34 has a disc-shaped movable valve body a portion of which is secured to the housing. Preferably, the movable valve body of each check valve has a slightly less specific gravity than blood, and a hardness of about 30 to 90 on Shore A scale. The valve bodies are preferably formed of, for example, styrene-based elastomer oil gel, silicone gel, silicone rubber, fluororubber or the like, to a thickness of about 0.5 to 5 mm.

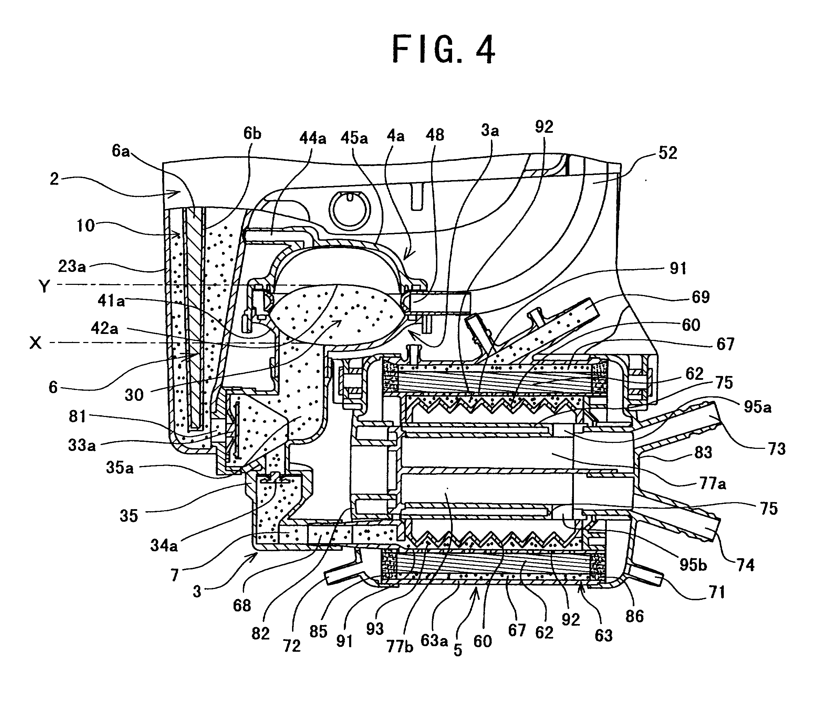

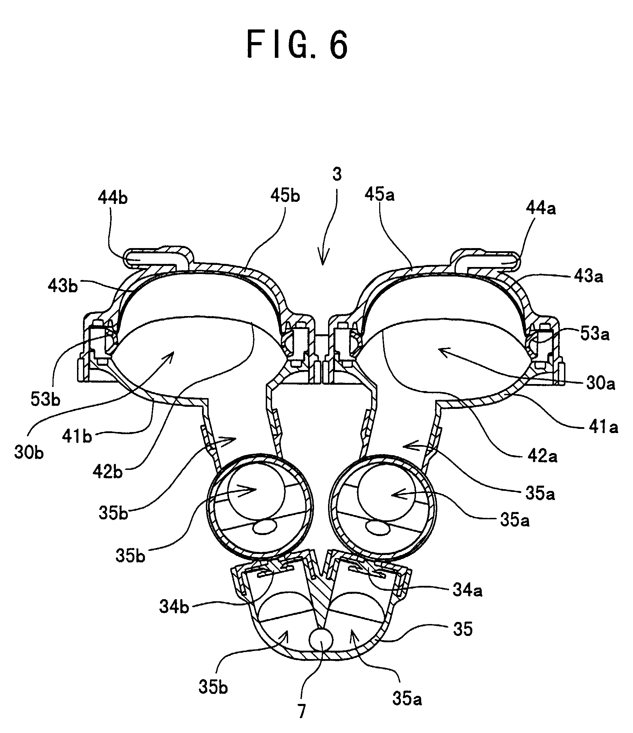

Each of the delivery blood reserving members 3a, 3b includes the delivery blood reserving member body portions 41a, 41b formed from a hard material; the flexible diaphragms 42a, 42b supported or fixed at its peripheral end to the delivery blood reserving member body portions 41a, 41b; and the delivery blood reserving portions 30a, 30b (see Figs. 4 and 6) formed between the delivery blood storing member body portions 41a, 41b and the flexible diaphragms 42a, 42b.

Fig. 4 is an enlarged sectional view of the blood delivery mechanism and surrounding parts of the blood delivery mechanism-equipped blood reservoir shown in Fig.3; and a lower portion of the blood storing portion and their surroundings, wherein the flexible diaphragms 42a, 42b are not pressed (blood is not being delivered).

The blood reserving members 3a, 3b include the flexible diaphragms 42a, 42b, respectively forming a portion of the delivery blood reserving portions 30a, 30b, respectively. The flexible diaphragms 42a, 42b are formed from a flexible material. The blood delivery mechanism-equipped blood reservoir 2 is provided with the blood delivering drive units 4a, 4b that operate, at the time of blood delivery, to deform the flexible diaphragms 42a, 42b, that is, deformable portions, so as to force blood out of the blood reserving members 3a, 3b. The blood delivering drive units 4a, 4b will be described below.

Each of the blood reserving member body portions 41a, 41b is provided with an inwardly concavity surface portion having the shape of a bowl. A peripheral end portion of each of the flexible diaphragms 42a, 42b is retained to a peripheral end portion of the concavity surface portion of the blood reserving member body portions 41a, 41b in such a manner that the peripheral end portion of each of the flexible diaphragms 42a, 42b is inclined toward a central portion of the concavity surface portion. The entire inner surface of each of the blood reserving member body portions 41a, 41b, including the peripheral end portion, is receded in the form of a bowl. This receded or concave surfaces of the blood reserving member body portions 41a, 41b closely contact the flexible diaphragms 42a, 42b, respectively at the time of blood delivery. Thus, the delivery blood reserving portions 30a, 30b formed between the inner surface of the flexible diaphragms 42a, 42b and the concave surfaces of the blood reserving member body portions 41a, 41b is a space that is variable in capacity.

The peripheral end portion of each of the flexible diaphragms 42a, 42b is clamped between the peripheral end portion of the blood reserving member body portions 41a, 41b (a portion slightly inward from the peripheral end) and an outer peripheral portion of diaphragm retaining annular members 53a, 53b.

The maximum amount of blood that can be reserved by the delivery blood reserving members 3a, 3b (the maximum amount that can be contained therein, that is, the capacity thereof) varies depending on the capacity of the blood reservoir portion 2a used. However, the capacity of the delivery blood reserving members 3a, 3b is favorably about 20-500 ml and, particularly, about 50-500 ml. More favorably, the capacity thereof is about 50-300 ml and, particularly, about 80-300 ml.

The flexible diaphragms 42a, 42b have a shape corresponding to the shape of the inner surfaces of the concavity surface portions of the blood reserving member body portions 41a, 41b. It is preferred that the pressure needed to deform the diaphragms 42a, 42b be equal to or less than 100 mmH$_2$O, whereby the flexible diaphragms 42a, 42b become more sensitive in responding to changes in the amount of blood stored in the blood storing portion 10, so that reservation of amounts of blood proportional to the amount of blood stored in the blood storing portion 10 is more reliably ensured. The pressure needed to deform each of the flexible diaphragms 42a, 42b is more favorably equal to or less than 50 mmH$_2$O.

The diaphragms 42a, 42b may be suitably formed from, for example, polyurethane, silicone, polyvinyl chloride, and the like. Particularly preferred materials are polyurethane and silicone, which are presently widely used for members that contact blood.

It is preferred that the diaphragms 42a, 42b be sufficiently flexible. As an index of flexbility, compliance may be employed. The diaphragms 42a, 42b of the blood reserving members 3a, 3b have a compliance of, favorably, 2 ml/sec•mmHg or higher and, more favorably, within the range of 5-30 ml/sec•mmHg, when the blood surface in the blood storing portion 10 of the blood reservoir 2 is lower than the uppermost portion of the inner space of each of the delivery blood reserving members 3a, 3b, that is, when the pressure sensitivity (liquid surface sensitivity) of the delivery blood reserving members 3a, 3b is effective. It is further preferred that the diaphragms 42a, 42b of the delivery blood reserving members 3a, 3b have a compliance that is lower than the aforementioned value, when the blood surface in the blood storing portion 10 of the blood reservoir 2 is higher than the uppermost portion of the inner space of each of the delivery blood reserving members 3a, 3b. The resistance to blood inflow into the delivery blood reserving members 3a, 3b can be expressed by rate of blood inflow to the delivery blood reserving members 3a, 3b. The rate of blood inflow to the delivery blood reserving members 3a, 3b is preferably 20-600 ml/sec.

It is preferred to provide the flexible diaphragms 42a, 42b with a reinforcement (not shown) which covers the outside of the diaphragms and whose peripheral end portion is fixed to the blood reserving member body portions 41a, 41b. Preferably, the diaphragm and the reinforcement combined have the following properties: the pressure needed to deform the diaphragm is equal to or less than 100 mmH$_2$O; the rupture strength is equal to or greater than 5 kg/cm$^2$; and the diaphragm itself produces substantially no self-restoring force against deformation. It is preferred that the diaphragm and the reinforcement of each of the delivery blood reserving members 3a, 3b be not adhered to each other at all or except at the peripheral end portions thereof. The reinforcements may be provided in the form of a woven fabric, a non-woven fabric, a knitted fabric, a sheet material or the like. In view of strength, the form of a woven or knitted fabric is preferred. As for examples of the material of the reinforcements, polyethylene terephthalate, nylon 6, nylon 66, regenerated cellulose, polypropylene, fiber-reinforced plastics (FRP, with aramid fiber or the like), stainless steel, aluminum or the like may be suitably used. Particularly preferred are polyethylene terephthalate, nylon 6, nylon 66, regenerated cellulose and polypropylene.

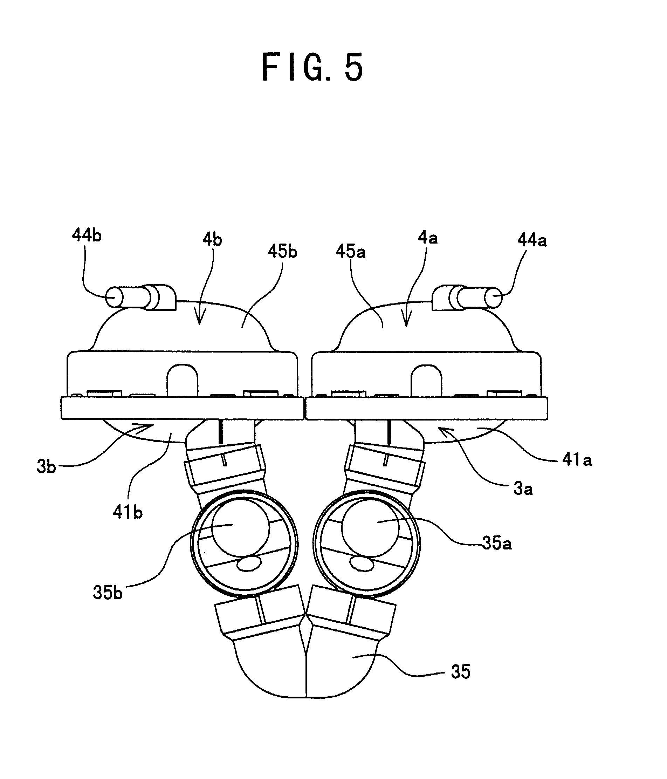

The delivery blood reserving members 3a, 3b have the delivery blood reserving portions 30a, 30b, respectively provided therein and communicate with the blood passages 35a, 35b positioned at a lower portion (lower end) of the blood reserving members 3a, 3b. The delivery blood reserving members 3a, 3b are positioned above the first check valves 33a, 33b defining boundary sites between the blood storing portion 10 and the blood flow passage portions 35a, 35b. The delivery blood reserving members 3a, 3b extend substantially horizontally. If the blood surface in the blood storing portion 10 is below the uppermost end of the delivery blood reserving portions 30a, 30b (inner space) of the delivery blood reserving members 3a, 3b, amounts of blood proportional to the blood surface in the blood storing portion 10 flow into the delivery blood reserving members 3a, 3b. If the blood surface in the blood storing portion 10 is above the uppermost end of the delivery blood reserving portions 30a, 30b (inner space) of the delivery blood reserving members 3a, 3b, the maximum amount of blood flows into the delivery blood reserving members 3a, 3b (as shown in Fig. 5) since the maximum capacity of the delivery blood reserving portions 30a, 30b of the delivery blood reserving members 3a, 3b is fixed.

More specifically, the delivery blood reserving members 3a, 3b receive pressure proportional to the amount of blood stored in the blood storing portion 10, thus forming pressure-sensitive containers. If the amount of blood (liquid surface) in the blood storing portion 10 is equal to or greater than a predetermined amount (in this embodiment, the blood surface in the blood storing portion 10 is above the uppermost end of the inner space of the delivery blood reserving members 3a, 3b), the amount of blood reserved in the delivery blood reserving members 3a, 3b is determined by the maximum capacity thereof, not by the pressure corresponding to the amount of blood stored in the blood storing portion 10. That is, the delivery blood reserving members 3a, 3b reserve the maximum amount of blood. If the amount of blood stored in the blood storing portion 10 is equal to or less than the predetermined amount (in this embodiment, the blood surface in the blood reservoir portion 2a is below the uppermost end of the inner space of the delivery blood reserving members 3a, 3b), the pressure sensitivity of the delivery blood reserving members 3a, 3b becomes effective so that the delivery blood reserving members 3a, 3b reserve amounts of blood proportional to the amount of blood stored in the blood storing portion 10 (that is, the level of the blood surface in the blood reservoir portion 2a). In short, the delivery blood reserving members 3a, 3b function to automatically reserve amounts of blood proportional to the amount of blood stored in the blood storing portion 10 when the amount of blood in the blood storing portion 10 is equal to or less than the predetermined value.

The delivery blood reserving members 3a, 3b do not spontaneously draw in blood, that is, do not substantially have self-shape-restoring characteristic. If the delivery blood reserving members 3a, 3b were formed so that the members have a preset shape and self-shape-restoring characteristic, the delivery blood reserving members 3a, 3b would restore their preset shape when the load from the drive members for forcing blood out of the members 3a, 3b is removed after blood is thereby forced out Then the self-restoring force of the delivery blood reserving members 3a, 3b would cause a suction force, whereby blood would be drawn back into the delivery blood reserving members 3a, 3b from the side of the blood reservoir portion 2a. In short, the delivery blood reserving members 3a, 3b would retain a certain minimum level of blood, below which the pressure sensitivity could not be effective.

The capacity of each of the blood flow passage portions 35a, 35b (that is, a blood passage partially defined by the blood inlet of the delivery blood reserving portions 30a, 30b and the first and second check valves 33a, 34a or 33b, 34b) of the blood delivery instrument 3 is preferably equal to or less than 150 ml. Each of the blood flow passage portions 35a, 35b represents a blood passage partially defined by the first and second check valves 33a, 34a or 33b, 34b and the blood inlet of the delivery blood reserving portions 30a, 30b of the blood reserving members 3a, 3b (the inner surface of the diaphragms 42a, 42b) when the flexible diaphragms 42a, 42b are closed or depressed.

The minimum sectional area of each of the blood flow passage portions 35a, 35b is equal to or greater than 1.0 cm$^2$. With such a sectional area of each of the blood flow passage portions 35a, 35b, the pressure sensitivity of the blood reserving members 3a, 3b will not be impeded, but can be made sufficiently high so that the blood reserving members 3a, 3b will reserve amounts of blood proportional to the amount of blood in the blood storing portion 10 (that is, the blood surface in the blood reservoir portion 2a) when the amount of blood stored is equal to or less than a predetermined value. It is preferred that the pressure loss of each of the blood flow passage portions 35a, 35b (the pressure loss of a blood passage) be as small as possible. In present invention, the pressure loss is preferably 130 mmH$_2$O or less and, particularly, 110 mmH$_2$O and, more favorably, 100 mmH$_2$O. With such a pressure loss, a sufficiently high pressure sensitivity and good operation can be achieved. The minimum sectional area of each of the blood flow passage portions 35a, 35b is more favorably 1.5 cm$^2$ or greater, whereby a favorable pressure sensitivity of the blood reserving members can be ensured.

It is also preferred that the capacity of each of the blood flow passage portions 35a, 35b be smaller than the maximum amount of blood that can be reserved in the blood reserving members 3a, 3b (the maximum amount that can be contained therein), whereby the entire volume of blood in the blood flow passage portions 35a, 35b can be forced out thereof by blood discharged from the blood reserving members 3a, 3b, thereby minimizing blood stagnation in the blood flow passage portions 35a, 35b.

The blood reserving member body portions 41a, 41b may suitably be formed from, for example, polycarbonate, acrylic resin, polyethylene terephthalate, polyethylene, polypropylene, polystyrene, polyvinyl chloride, acryl-styrene copolymers, and acryl-butadiene-styrene copolymers, and the like. Particularly preferred materials are polycarbonate, acrylic resin, polystyrene, and polyvinyl chloride. In this embodiment, the blood reserving member body portion 41a and the blood flow passage portion 35a are unitarily formed as a single member. Similarly, the blood reserving member body portion 41b and the blood flow passage portion 35b are unitarily formed. However, it is also possible to separately provide a blood reserving member body portion and a blood flow passage part and then connect them.

The blood delivering drive units 4a, 4b are disposed outside the blood reserving members 3a, 3b in such a manner as to cover the blood reserving members 3a, 3b, respectively. The blood delivering drive units 4a, 4b and the blood delivery instrument 3 form a blood delivery mechanism.

Each of the blood delivering drive units 4a, 4b is provided with the blood reserving member-pressurizing portions 43a, 43b formed of an expandable sheet material and operating to deliver blood out of the corresponding blood reserving members 3a, 3b. More specifically, each of the blood delivering drive units 4a, 4b includes headers 45a, 45b provided with the blood delivering fluid flow ports 44a, 44b extending horizontally outwardly from a central portion thereof.

In each of the blood reserving member-pressurizing portions 43a, 43b, an annular portion slightly inward from a peripheral end portion thereof is clamped between the headers 45a, 45b and a peripheral end portion of the diaphragm retaining annular members 53a, 53b, and thereby substantially air-tightly retained. Each of the diaphragm retaining annular members 53a, 53b retains both the diaphragms 42a, 42b and the blood reserving member-pressurizing portions 43a, 43b, and controls excessive expansion of the diaphragms 42a, 42b. Since the blood reserving member-pressurizing portions 43a, 43b of the blood delivering drive units 4a, 4b are expandable or stretchable sheet members for expansion and contraction achieved by a fluid, the incidence of damaging or breaking the diaphragm by pressurization thereon is minimum. Furthermore, a relatively inward peripheral end portion of each of the blood reserving member-pressurizing portions 43a, 43b is restricted by the inclined annular surface of the diaphragm retaining annular members 53a, 53b when the blood reserving member-pressurizing portions 43a, 43b are expanded.

For use, a blood delivering fluid supply machine (not shown) is connected to the blood delivering fluid flow ports 44a, 44b. By supplying and discharging a fluid (liquid or gas) by a compressor provided in the blood delivering fluid supply machine, the expanding and contracting driving of the blood reserving member-pressurizing portions 43a, 43b is repeated. The blood reserving member-pressurizing portions 43a, 43b of each of the blood delivering drive units 4a, 4b do not contact the diaphragms 42a, 42b of the blood reserving members 3a, 3b when not expanded (contracted) as shown in Fig. 5. When expanded, the blood reserving member-pressurizing portions 43a, 43b of the blood delivering drive units 4a, 4b pressurize the diaphragms 42a, 42b of the blood reserving members 3a, 3b in cooperation with the blood reserving member body portions 41a, 41b, thereby discharging blood out of the interior of the blood reserving members 3a, 3b (the interior of the delivery blood reserving portions 30a, 30b).

The spaces between the blood reserving members 3a, 3b (the diaphragms 42a, 42b) and the blood reserving member-pressurizing portions 43a, 43b (namely, spaces between the blood reserving members 3a, 3b and the blood delivering drive units 4a, 4b) are in communication with the interior of the blood storing portion 10. More specifically, in each space formed between the blood reserving members 3a, 3b and the blood reserving member-pressurizing portions 43a, 43b, one end of a tubular body 52 is connected with openings 48a, 48b formed on the diaphragm retaining annular members 53a, 53b which are component members of the blood delivering drive units 4a, 4b, and the other end of the tubular body 52 communicates with an upper portion of the interior of the blood storing portion 10.

Preferred examples of elastic materials used to form the blood reserving member-pressurizing portions 43a, 43b include synthetic rubbers such as urethane rubber, silicone rubber, butadiene rubber and the like, natural rubbers such as latex rubber and the like, and elastomers such as olefin-based elastomer, amide-based elastomer, styrene-based elastomer (for example, styrene-butadiene-styrene copolymers, styrene-isoprene-styrene copolymers, styrene-ethylenebutylene-styrene copolymers), polyurethane elastomer, and the like.

The blood delivering drive unit may adjust the amount of blood forced out of the blood reserving member by adjusting the amount or pressure of the fluid fed into the drive unit. The amount of blood delivered may be easily changed by adjusting the amount or pressure of the fluid fed into the blood delivering drive unit while fixing the number of times of driving the blood delivering drive unit per predetermined length of time.

The blood delivery mechanism-equipped blood reservoir 2 of this embodiment is equipped with the two sets of the delivery blood storing member and the blood delivering drive unit 4, as described above. Further, the two separate blood flow passage portions 35 (35a, 35b) are provided without communication therebetween, as shown in Figs. 5 and 6. By providing two or more sets thereof in this manner, the capacity of each of the delivery blood reserving members 3a, 3b and each of the blood delivering drive units 4a, 4b can be reduced, so that the blood inflow and discharge response improves. Furthermore, if two or more sets of the delivery blood storing member and the blood delivering drive unit 4 are provided, good blood flow may be achieved by shifting the expanding strokes of the blood delivering drive units 4a, 4b in timing from each other. If the expanding timing of the blood delivering drive units 4a, 4b is thus shifted, flow of blood from one blood reserving member to the other in this embodiment never occurs since the individual blood reserving members are provided with the separate blood flow passage portions 35a, 35b. The construction of the invention is not limited by this embodiment The number of sets of the delivery blood reserving member and the blood delivering drive unit may also be one, or three or more. In addition, although the blood delivering drive units 4a, 4b in this embodiment repeat expansion and contraction through fluid operation, the blood delivering drive unit is not restricted by this driving manner. For example, the blood delivering drive unit may mechanically drive a pressurizing plate to pressurize the blood reserving member.

If two sets of the delivery blood reserving member and the blood delivering drive unit are provided as in blood delivery mechanism-equipped blood reservoir 2, and furthermore, the individual blood delivering drive units can be separately controlled by a fluid supply machine for blood delivery, it becomes possible to select the form of blood flow for delivery, more specifically, select a pulse flow or a constant flow, considering the conditions of a patient, the type of the oxygenator used or the like. It becomes also possible to change the form of delivery blood flow while in use. More specifically, a substantially constant blood flow can be achieved by shifting the phases of the blood flow out of the individual delivery blood reserving members 3a, 3b by substantially 180° from each other, that is, by shifting the phases of fluid flowing into and out of the blood delivering drive units 4a, 4b by substantially 180° from each other. A good pulse flow can be achieved by setting the phases of blood flow out of the individual blood reserving members 3a, 3b to substantially the same phase or within ±30°, that is, by setting the phases of fluid flowing into and out of the blood delivering drive units 4a, 4b to substantially the same phase or within ±30°. If three or more sets of the delivery blood storing member and the blood delivering drive unit are provided, the form of blood flow will become a substantially constant flow by shifting the phases of blood flow out of the individual delivery blood reserving members by an angle obtained by dividing 360° by the number of the sets provided.

In the blood delivery mechanism-equipped blood reservoir 2, the diaphragms 42a, 42b of the blood reserving members 3a, 3b are surrounded with the delivery blood reserving member body portions 41a, 41b, and are not exposed to the outside. Thus, the function of protective covers for the diaphragms are provided.

Next, the heat exchange function-equipped oxygenator 5 will be described below.

The heat exchange function-provided oxygenator 5 includes a cylindrical heat exchanger portion 60; a hollow fiber bundle 62 composed of a large number of gas exchange hollow fibers and accommodating the cylindrical heat exchanger portion 60 therein; a housing 63 accommodating the cylindrical hollow fiber bundle 62 and the cylindrical heat exchanger portion 60 therein; partitioning walls 85, 86 for fixing either end of the cylindrical hollow fiber bundle 62 to the housing 63 and to the cylindrical heat exchanger portion 60, with either end of the hollow fiber open; a blood chamber 67 formed of an inner surface of the housing 63, an outer surface of the hollow fiber, an outer surface of the cylindrical heat exchanger portion 60, and the partitioning walls 85, 86; and a blood inlet port 68 and a blood outlet port 69 both formed on the housing 63 and communicating with the blood chamber 67; a gas inlet port 71 and a gas outlet port 72 both communicating with an interior of the hollow fiber; and a heating medium inlet port 73 and a heating medium outlet port 74 both communicating with an interior of the cylindrical heat exchanger portion 60.

The housing 63 of the oxygenator 5 of the embodiment shown in Fig. 3 includes a cylindrical housing body 63a; a first header 83 having the gas inlet port 71, the heating medium inlet port 73, and the heating medium outlet port 74; and a second header 82 having the gas outlet port 72. The blood inlet port 68 is formed at a position in the vicinity of the upper end of the side surface of the cylindrical housing body 63a.

The cylindrical hollow fiber bundle 62 is wound on the outer surface of a core 91 having a large number of openings 92 formed thereon. The cylindrical heat exchanger portion 60 is accommodated inside the core 91. A blood communication portion 93 (a part of the blood chamber 67) communicating with the blood inlet port 68 and the blood outlet port 69 is formed between an inner surface of the core 91 and an outer surface of the cylindrical heat exchanger portion 60. The blood chamber 67 is formed between an outer surface of the hollow fiber and an inner surface of the cylindrical housing body 63a. Formed inside the cylindrical heat exchanger portion 60 is a heating medium communication chamber 76 communicating with the heating medium inlet port 73 and the heating medium outlet port 74.

In the oxygenator 5, blood introduced into the oxygenator 5 from the blood inlet port 68 is heat-exchanged while it is flowing between the inner surface of the core 91 and the outer surface of the cylindrical heat exchanger portion 60. Then, the blood flows into the hollow fiber bundle 62 (into the blood chamber 67) from the opening 92 of the core 91. Upon contact between the blood and the hollow fiber, gases contained in the blood are exchanged with each other. The blood which has passed through the cylindrical hollow fiber bundle 62 flows into an annular space (a part of the blood chamber 67) formed between the cylindrical hollow fiber bundle 62 and the inner surface of the cylindrical housing body 63a and then flows out from the blood outlet port 69. The annular space may not be provided.

As described above, the upper end of the blood chamber inside the oxygenator 5 is lower than the level (X) in the state where the specified minimum reserving amount of blood is reserved inside the blood storing portion 10. Further, as described above, the blood inlet port 68 is formed at the position in the vicinity of the upper end of the side surface of the cylindrical housing body 63a. The blood outlet port 69 of the oxygenator 5 communicates with an upper end or its proximity of a blood chamber provided inside the oxygenator 5. It is to be noted that in the blood oxygenating apparatus 1 of the embodiment, the oxygenator 5 includes the cylindrical housing 63 extending substantially horizontally; and the blood chamber 67 formed inside the cylindrical housing 63. It is to be also noted that the blood outlet port 69 is formed at the upper end of the side surface of the cylindrical housing 63. That is, the blood outlet port 69 is so constructed that it extends upward from the upper end of the blood chamber 67 formed inside the cylindrical housing 63. In the oxygenator 5 of the embodiment, the blood outlet port 69 extends obliquely upward. More specifically, the blood outlet port 69 extends obliquely upward in a direction opposite to the location of the blood delivery mechanism. Therefore, it is easy to perform a circuit-tube connection operation and connected tubes seldom interfere with medical appliances or the like.

Preferably, the outer diameter of the cylindrical hollow fiber bundle 62 is 30 - 144 mm. Preferably, the filling factor of the cylindrical hollow fiber bundle 62 is 45 - 75%.

A porous is used to make the gas exchange hollow fiber. Favorably, the inner diameter of the porous hollow fiber is favorably, 50 - 1000 μm, and more favorably, 54.0 - 84.0 μm. Favorably, the thickness of the porous hollow fiber is 5 - 200 μm, and more favorably, 10 - 100 μm. The outer diameter of the porous hollow fiber is favorably, 66.0 - 106.0 μm. The porosity of the porous hollow fiber is favorably, 20 - 80%, and more favorably, 30 - 60%. The diameter of the pore of the porous hollow fiber is favorably, 0.01 - 5 μm, and more favorably, 0.01 - 1 μm. Hydrophobic polymers are used to form the porous hollow fiber. Exemplary hydrophobic polymers include polypropylene, polyethylene, polysulfone, polyacrylonitrile, polytetrafluoroethylene, and cellulose acetate. Preferred among others are polyolefin resins, especially polypropylene. Porous hollow fibers of polypropylene having micropores formed thereon by drawing method or solid-liquid phase separation method are desirable. Preferably, the thickness of the cylindrical hollow fiber bundle 62 is in the range of 5 - 25 mm.

The partitioning walls 85 and 86 are formed of an adhesive and elastic potting material made of such as polyurethane, silicone rubber, and the like. The cylindrical hollow fiber bundle 62, the cylindrical heat exchanger portion 60, and the housing 63 are not necessarily coaxial with one another.

The cylindrical heat exchanger portion 60 of bellows type is preferable. A cylindrical member of bellows type which is a component part of the outer surface of the cylindrical heat exchanger portion 60 is formed of metal such as stainless steel, aluminum or the like or a resinous material such as polyethylene, polycarbonate or the like. These materials are formed in the shape of bellows having fine pleats formed thereon. As the material of the cylindrical member of bellows type, metal such as stainless steel or aluminum is more favorable in consideration of strength and heat exchange effectiveness thereof. The cylindrical heat exchanger portion 60 of the embodiment is formed as a bellow pipe having a large number of convex and concave portions formed repeatedly and substantially perpendicularly to the axial direction thereof. In consideration of the heat exchange effectiveness of the cylindrical heat exchanger portion 60, the difference in height between a mountain of the bellows and a valley thereof is favorably 5.0 - 15.0mm and more favorably 9.0 - 12.0 mm.

The cylindrical heat exchanger portion 60 having an axial length of 70.0 - 150 mm is used, although the axial length thereof is different according to a patient.

The cylindrical heat exchanger portion 60 is constructed of a cylindrical member of bellows type forming the outer surface thereof and an inner cylindrical member 75 accommodated inside the cylindrical member of bellows type. A heating medium chamber 76 is formed between the inner cylindrical member 75 and the cylindrical member of bellows type. The inner cylindrical member 75 is closed on a bottom surface thereof. The inner cylindrical member 75 has at the side surface thereof two openings 95a, 95b; the heating medium inlet port 73; and the heating medium outlet port 74. The interior of the inner cylindrical member 75 is divided into first and second inner chambers 77a, 77b. The heating medium inlet port 73 communicates with the first inner chamber 77a. The heating medium outlet port 74 communicates with the second inner chamber 77b. A heat exchange medium introduced into the cylindrical heat exchanger portion 60 from the heating medium inlet port 73 flows into the first inner chamber 77a, passes through the opening 95a, and flows through the space between the cylindrical member of bellows type and the inner cylindrical member 75, flows into the second inner chamber 77b from the opening 95b, and flows out from the heating medium outlet port 74.

The inner cylindrical member 75 is formed of synthetic resin or metal such as stainless steel, aluminum or the like. It is preferable that the heat exchanger portion 60 is cylindrical; that the outer diameter thereof is 20 - 100 mm; and that the length (effective length) thereof is 15 - 765 mm. The cylindrical member of bellow type and the inner cylindrical member constituting the cylindrical heat exchanger portion 60 may be integral with each other. In this case, preferably, the cylindrical heat exchanger portion 60 is formed of metal.

The housing body 63a may be cylindrical, polygonally cylindrical or elliptic in section. Preferably, the cylindrical housing body 63a is cylindrical. Preferably, the inner diameter of the cylindrical housing body 63a is 30 - 150 mm, and the length (effective length) thereof is 30 - 750 mm.

The cylindrical housing body 63a, the inner cylindrical member 75, a lid body, the first header 83, and the second header 82 can be formed of polyolefin (for example, polyethylene, polypropylene), ester resin (for example, polyethylene terephthalate), styrene resin (for example, polystyrene, MS resin, MBS resin), polycarbonate or the like.

It is preferable that the blood contact surface of the blood oxygenating apparatus is formed as an antithrombic surface. In particular, it is preferable that the delivery blood reserving members 3a, 3b, the blood passages 35a, 35b, the first check valves 33a, 33b, the second check valves 34a, 34b have a blood contact surface, respectively.

The antithrombic surface may be formed by applying and fixing an antithrombin to the surface. Exemplary antithrombins are heparin, urokinase, HEMA-St-HEMA copolymers, and poly-HEMA.

Preferably, the antithrombic surface is formed by treating a substrate with ozone to form functional group-bearing oxides on the substrate surface and applying heparin to the surface so that an amino group of heparin forms a covalent bond with the functional group directly or through a coupling agent This method permits heparin to be fixed on the blood wetting surface without the use of a solvent, minimizing a change of physical properties (e.g., flexibility, elasticity and strength) of the substrate presenting the blood wetting surface.

Through ozone treatment, oxides are formed on the substrate surface and high reactive functional groups such as aldehyde, ketone, and epoxy groups are generated in the oxides.

Amino groups of heparin can directly bond with these functional groups. For the reason of steric hindrance or other, introducing a spacer or coupling agent into these functional groups prior to fixation of heparin is easy and useful from the standpoint that the surface allows heparin to develop its activity. The coupling agents may be used alone or in admixture of two or more. Compounds having at least two aldehyde or epoxy groups are preferred.

Where two or more coupling agents are used, the preferred sequence is by first bonding a coupling agent (spacer coupling agent) in the form of a compound having at least two amino groups with the functional groups previously introduced in the substrate, to thereby introduce amino acid into the substrate, and thereafter bonding heparin to the substrate with the aid of a coupling agent (heparin-fixing coupling agent) in the form of a compound having at least two aldehyde or epoxy groups. In bonding heparin, the coupling agent is preferably admitted into the reaction system at the same time as or subsequent to heparin admission.

Especially when an amino group is introduced using a spacer coupling agent, it displays substantially the same reactivity as the amino group of heparin in the reaction system so that subsequent fixation of heparin to the substrate by the heparin-fixing coupling agent may take place more effectively.

Where the functional group of a coupling agent to directly bond with heparin or the functional group introduced into the substrate is an aldehyde group, it is preferable to use heparin in which some N-sulfate groups are desulfurized into primary amino groups.

It is preferable that the spacer coupling agent is one that forms a bond (covalent bond) with the functional group introduced on the substrate by ozone treatment and has at least two primary amino groups. Examples of the spacer coupling agent having at least two amino groups include polyethylene imine (PEI), polyethylene glycol diamine, ethylene diamine, and tetramethylene diamine.

Aldehyde and epoxy compounds are preferable as the coupling agent used for fixing heparin to the substrate. Exemplary of the aldehyde compound are glutaraldehyde, glyoxal, and succindialdehyde. Exemplary of the epoxy compound are polyethylene glycol diglycidyl ether, 1,4-butanediol diglycidyl ether, sorbitol diglycidyl ether, and glycerol diglycidyl ether.

Illustrative examples are Denacol EX-421, 521, 611, 612, 614, and 614B where the epoxy compound is sorbitol diglycidyl ether; Denacol EX-313 where the diepoxy compound is glycerol diglycidyl ether; Denacol EX-810, 811, 851, 821, 830, 832, 841, and 861 where the diepoxy compound is polyethylene glycol diglycidyl ether, all commercially available from Nagase Chemicals K.K. Denacol EX-313, 421, 512, 521, 810, 811, 821, and 851 are preferred when

13

the difference of epoxy reactivity is considered. In the above-mentioned heparin fixation, coupling-off of heparin is minimized since the bond between polyethylene imine fixed to the substrate and glutaraldehyde and the bond between glutaraldehyde and heparin are both covalent bonds.

According to the blood oxygenating apparatus of the present invention having the blood delivery mechanism-equipped blood reservoir, in performing priming operation of the blood chamber inside the oxygenator, it is very easy to replace air inside the blood chamber with a fluid by using the blood delivery mechanism used for an extracorporeal blood circulation. Further, the blood delivery mechanism does not deliver air to the oxygenator in normal blood delivery and when the amount of blood in the blood storing portion decreases, unlike the conventional blood delivery mechanism. Therefore, it is unnecessary to provide the oxygenator with an air trap mechanism which is formed by locating the blood outlet port of the oxygenator at a position downstream from the center of the blood chamber in the oxygenator. Moreover, even though such an air trap mechanism is not provided, it does not occur that air is not delivered to the oxygenator. Furthermore, it is easy to perform an operation of connecting tubes of the extracorporeal blood circulation circuit to the blood outlet port, and possible to use short tubes, which reduces a priming volume. Furthermore, it is possible to use a short loop consisting of upwardly extended tubes. Thus, the tubes do not interfere with medical appliances or the like in an operation.

The bubble remover for blood oxygenating circuit of the present invention and the blood oxygenating circuit apparatus having the bubble remover will be described in detail below with reference to drawings.

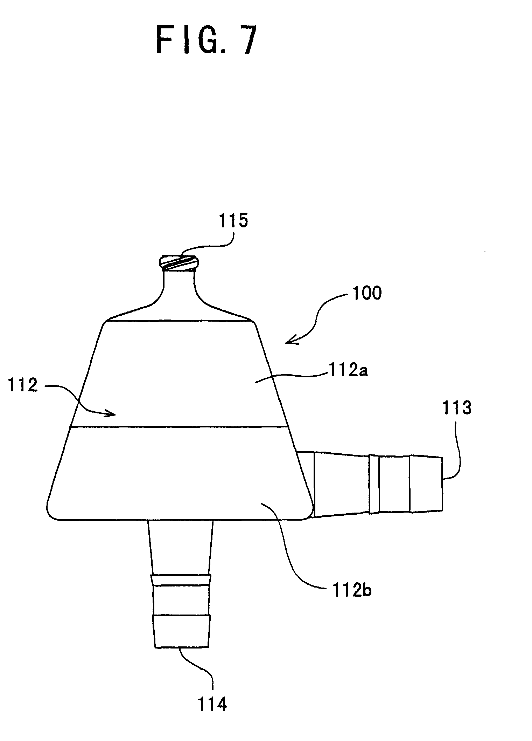

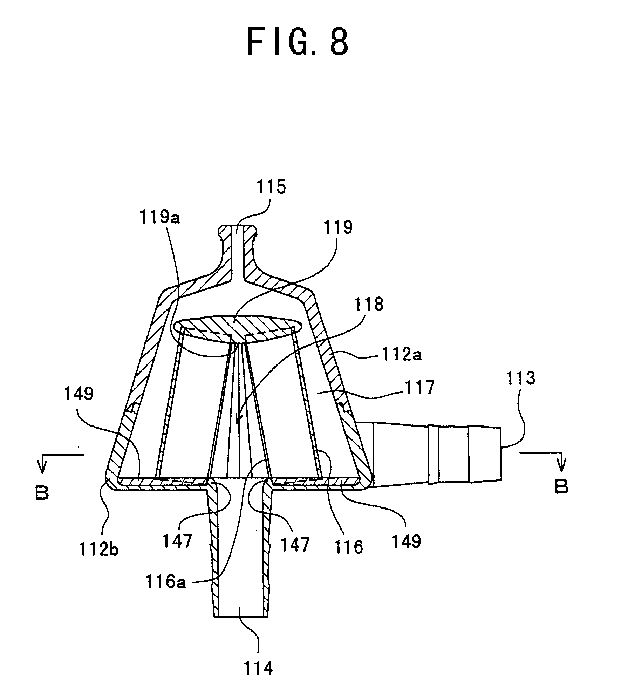

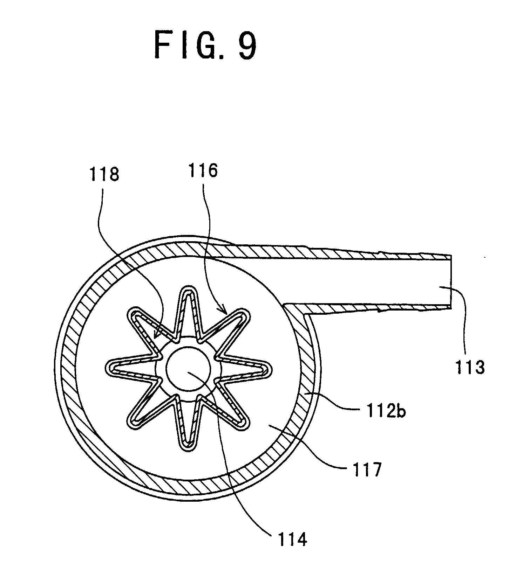

Fig. 7 is a front view showing the bubble remover for a blood oxygenating circuit according to an embodiment of the present invention. Fig. 8 is a sectional view of the bubble remover shown in Fig. 7. Fig. 9 is a sectional view taken along a line B-B of Fig. 8.

A bubble remover 100 for a blood oxygenating circuit of the present invention is used by locating it downstream from an oxygenator 5 of the blood oxygenating circuit comprising a blood delivery mechanism-equipped blood reservoir having a blood storing portion and a blood delivery mechanism having a blood reserving member having a delivery blood reserving portion communicating with the blood storing portion and reserving blood in an amount proportional to an amount of blood stored in the blood storing portion when the amount of the blood stored in the blood storing portion becomes equal to or less than a predetermined value and capable of flowing out blood stored in the delivery blood reserving portion when the delivery blood reserving portion is pressed from outside; and the oxygenator 5 connected with a blood outlet port of the blood delivery mechanism. The bubble remover 100 has the following relationship between an internal capacity (ml) and a maximum use flow (L/min).

$$\text{Internal capacity (ml)/maximum use flow (L/min)} \leqq 15$$

In the embodiment which will be described below, the blood reservoir 2 having the blood storing portion and the blood delivery mechanism is used. The present invention is not limited to the embodiment, but it is possible to adopt a blood reservoir having a construction in which the blood storing portion (blood reservoir) and the blood delivery mechanism are separately provided and connected with each other with a tube.

As will be described later, the blood reservoir 2 for use in the blood oxygenating circuit apparatus provided with the bubble remover 100 of the embodiment includes a blood storing portion 10; blood reserving members 3a, 3b provided with a delivery blood reserving portion, respectively communicating with the blood storing portion 10 and reserving blood in an amount proportional to an amount of blood stored in the blood storing portion 10 when the amount of the blood stored in the blood storing portion 10 becomes equal to or less than a predetermined value and capable of flowing out blood stored in the delivery blood reserving portion when the delivery blood reserving portion is pressed from the outside.

Figs. 1 through 4 show the state in which the heat exchange function-equipped oxygenator is installed on the blood delivery mechanism-equipped blood reservoir. Fig. 3 shows the oxygenator 5 and the blood delivery mechanism by cutting them along a center line and a broken line A-A of Fig. 1, respectively.

In the blood delivery mechanism-equipped blood reservoir 2, when the surface of the blood in the blood reservoir 2 is at a level below the upper end of the diaphragms 42a, 42b as well as a horizontal position (Y), i.e., when the amount of blood stored in the blood storing portion 10 is less than a predetermined value, the blood reserving members 3a, 3b store an amount of blood proportional to the amount of the blood stored in the blood storing portion 10. The blood in the blood reserving members 3a, 3b can be discharged intermittently by pressing the delivery blood storing members 3a, 3b at the side of the diaphragms 42a, 42b thereof from the outside. Therefore, when a remaining amount of the blood stored in the blood storing portion 10 becomes small, blood is fed in a small amount in proportion to the remaining amount of the blood stored in the blood storing portion 10. That is, even though the remaining amount of the blood in the blood storing portion 10 becomes small, the delivery of a small amount of blood is maintained. Thus, even though the remaining amount of the blood in the blood storing portion 10 becomes small, it is unnecessary to suspend the blood delivery and possible to prevent blood from stagnating in the extracorporeal blood circulation circuit The blood reservoir stores the specified minimum reserving amount of blood. That is, when the surface of the blood in the blood storing portion 10 is located at the lower end of the delivery blood storing members 3a, 3b, namely, at the lowermost end (position shown with X of Fig. 3) of the diaphragms 42a, 42b, the blood is not delivered. In other words, it occurs that the surface of the blood in the blood storing portion 10 drops to the position shown with X of Fig. 3.

Because in the blood delivery mechanism, air does not flow into the delivery blood storing members 3a, 3b, air is not delivered to the oxygenator side, regardless of whether the amount of the blood in the blood storing portion 10 is more than the predetermined value. Because the blood delivery mechanism-provided blood reservoir 2 having the construction is used, in the use of the bubble remover, it is unnecessary to consider a possibility that a large amount of air flows into the circuit while blood is circulating extracorporeally, unlike a conventional blood delivery means. Further, it is necessary to consider only removal of air which has not been removed in a priming work, a filtering capacity, and a pressure loss.

The bubble remover 100 of the present invention has a housing 112 constructed of an upper housing 112a and a lower housing 112b. The upper housing 112a and the lower housing 112b are joined with each other after a filtering member 116 is accommodated in the housing 112.

The bubble remover 100 includes the housing 112 having a blood inlet port 113, a blood outlet port 114, and a air vent (deaeration opening) 115; and the filtering member 116 accommodated in the housing 112 such that the filtering member 116 divides the interior of the housing 112 into a blood inlet-side space 117 communicating with the blood inlet port 113 and a blood outlet-side space 118 communicating with the blood outlet port 114. The internal capacity of the bubble remover 100 is 120 ml or less. The diameter of the opening of each of the blood inlet port 113 and the blood outlet port 114 is set to 8 mm or more.

The housing 112 is in the shape of a rotary body having a shaft. That is, as shown in Figs. 7 and 9, the sectional shape of the housing 112 is substantially circular. The blood inlet port 113 is formed on the peripheral surface of the upper housing 112a of the housing 112 such that the blood inlet port 113 projects from the upper housing 112a in a direction substantially tangent to the inner peripheral surface of the lower housing 112b. Therefore, blood introduced into the housing 112 from the blood inlet port 113 forms a spiral current flow along the inner peripheral surface of the housing 112.

The upper housing 112a of the housing 112 is truncated cone-shaped. That is, outer and inner diameters of the upper housing 112a become gradually smaller from the lower end thereof toward the upper end thereof. The air vent 115 for exhausting bubbles removed from blood projects from the upper end of the upper housing 112a. Although not shown, a valve such as a three-way stop cock is connected to the air vent opening 115 when the bubble remover 100 is used.

The blood outlet port 114 extending vertically downward is located at the center of the lower housing 112b of the housing 112. The blood outlet port 114 and the air vent 115 are substantially coaxial with each other.

Formed at the bottom of the lower housing 112b is an annular groove-shaped filtering member-fixing portion for fixing the lower portion of the filtering member 116 which will be described later. A ring-shaped rib 147 projecting into the housing 112 is formed at the boundary between the filtering member-fixing portion and the blood outlet-side space. The rib 147 locks thereto one end (front end) of a pleat 116a projecting into the filtering member 116 and prevents a filling agent 149 injected into the filtering member-fixing portion from flowing out toward the blood outlet port 114.

The housing 112 (the lower housing 112b and the upper housing 112a) is formed of any desired resin, for example, polycarbonate, acrylic resin, polyethylene terephthalate, polyethylene, polypropylene, polystyrene, polyvinyl chloride, acryl-styrene copolymers, and acryl-butadiene-styrene copolymers. It is preferable that the housing 112 is transparent or semi-transparent so that the inside thereof can be readily observed.

The filtering member 116 for filtering bubbles and foreign matter which have mixed into blood is accommodated in the housing 112. The filtering member 116 partitions the housing 112 into the blood inlet-side space 117 formed outside the filtering member 116 and the blood outlet-side space 118 formed inside the filtering member 116. The blood inlet port 113 and the air vent 115 communicate with the blood inlet-side space 117, while the blood outlet port 114 communicates with the blood outlet-side space 118.

The internal capacity of the bubble remover 100 means the capacity of a fluid accommodated inside the housing 112 in the state where the housing 112 accommodates the filtering member 116. The relationship between the internal capacity (ml) and the maximum use flow rate (L/min) is as shown below:

$$\text{Internal capacity (ml)/maximum use flow rate (L/min)} \leqq 15.$$

Because the internal capacity is small as compared with the maximum use flow rate, a bubble remover-caused increase of a blood filling amount (dead volume) is very small. More favorably, $4 \leqq$ internal capacity (ml) / maximum use flow rate (L/min) $\leqq 15$. If internal capacity (ml) / maximum use flow rate (L/min) $\geqq 4$, it is possible to form a spiral current reliably inside the bubble remover 100 of internal spiral current type which is used in the embodiment and thus separate bubbles from blood.

Preferably, the internal capacity of the bubble remover 100 is less than 120 ml. In this case, a bubble remover-caused increase of the blood filling amount (dead volume) is very small. Favorably, the diameter of each of the inlet port 113 and the blood outlet port 114 is 8 mm or more to accomplish a preferable extracorporeal circulation, namely, to prevent a pressure loss from being great when the flow rate of blood is 0.5 - 7 L/min necessary for performing an extracorporeal circulation for adults. In some conventional bubble removers for children having an internal capacity of 35 ml or less, the diameter of each of its inlet port 113 and its blood outlet port 114 is not large enough to flow blood at a rate of about 7 L/min necessary for performing an extracorporeal circulation for adults. Favorably, the internal capacity of the bubble remover is 30 - 105 ml. When the internal capacity thereof is 30 ml or more, it is possible to form a spiral current reliably inside the bubble remover of internal spiral current type which is used in the embodiment When the internal capacity thereof is 105 ml or less, it is possible to reduce the blood filling amount (dead volume). More favorably, the internal capacity of the bubble remover is 40 - 100 ml.

Favorably, the diameter of each of the blood inlet port 113 and the blood outlet port 114 is 8 - 12 mm. When it is 8 mm or more, blood can be flowed at a rate of about 7 L/min necessary for performing an extracorporeal circulation for adults. When it is 12 mm or less, a pressure loss is low. More favorably, the diameter of each of the blood inlet port 113 and the blood outlet port 114 is 9.5 - 10.5 mm. The pressure loss (hematcrit (Hct) value: 35%, when blood of 37°C is circulated at a flow rate 8 L/min) is favorably 60 mmHg or less. More favorably, the pressure loss (hematcrit (Hct) value: 35%, when blood of 37°C is circulated at a flow rate 8 L/min) is favorably 40 mmHg or less. As the filtering capability of the bubble remover, it is preferable to catch bubbles/particles of 40µm at 98% or more and catch bubbles/particles of 30 µm at 50% or more.

Next, the construction of the filtering member 116 will be described in detail below. The filtering member 116 is formed cylindrically such that a plurality of pleats 116a of a folded porous material are arranged radially. An opening of the filtering member 116 at the apex side thereof is sealed with a filler material member 119. The outer diameter of the filtering member 116 becomes gradually larger from the upper end thereof toward the lower end thereof. That is, the filtering member 116 is hollow truncated cone-shaped. Because the filtering member 116 has the above-described construction, it is possible to introduce blood into the blood inlet-side space 117 and form a spiral current therein smoothly and preferably. Consequently, the separation/removal efficiency of bubbles can be enhanced and the pressure loss can be reduced. Further, hemolysis can be suppressed.

The following various porous materials can be used to form the filtering member 116: mesh, net, foamed materials, woven fabrics, non-woven fabrics and a combination thereof. For example, it is preferable to sandwich both screen mesh surfaces made of polypropylene or polyester with a net made of polypropylene or polyester. The minimum diameter of a pore of a porous material represented by the mesh and the net is favorably 20 - 200 µm, and more favorably, 20 - 60 µm.

As shown in Fig. 8, the center of the surface (hereinafter referred to as lower surface 119a) of the filling material 119 at the side of the blood outlet-side space 118 projects toward the blood outlet-side space 118. The region from the center of the lower surface 119a to the periphery of the filling material 119, in particular, the portion traversing the pleats 116a forms a predetermined angle with respect to a horizontal line.

Because the lower surface 119a of the filling material 119 has such a shape, it is possible to remove bubbles in the blood outlet-side space 118 in priming operation efficiently and remove bubbles from the valley of each pleat 116a at a blood treatment operation efficiently. The average inclination of the lower surface 119a of the pleat-traverse portion with respect to the horizontal line is not specified, but favorably, 5° or more and more favorably, 10 - 60° to remove bubble more efficiently.

The filling material 119 can be formed of the following materials: silicone, epoxy resin, polyolefins such as polyurethane, polyethylene, polypropylene and ethylene-vinyl acetate copolymers; and macromolecular materials such as rubber materials. Polyurethane, silicone, and epoxy resin are particularly favorable.

The filtering member 116 having the construction is produced by the following method.

First, a porous material folded in the shape of a pleat is molded cylindrically such that pleats are radially arranged. Then, the filling material 119 is injected into a cylindrical material thus prepared from the apex thereof. At this time, the lower surface 119a of the filling material 119 may be horizontal.

Before the filling material 119 is hardened completely, a die is inserted into the cylindrical material thus prepared to open the bottom portion of the filtering member 116. As a result, the filtering member 116 is formed in the shape of a truncated cone, and the lower surface 119a of the filling material 119 has the above-described shape. Then, the filling material 119 is hardened, and the die is removed. In this manner, the filtering member 116 as shown in Fig. 8 is formed.

The above-described method allows the filtering member 116 to be truncated cone-shaped easily and the lower surface 119a of the filling material 119 to have the above-described shape easily. Further, this method has superior reproducibility in mass production.

The filtering member 116 is fixed to the housing 112 as follows: the filling agent 149 is injected into the filtering material-fixing portion of the lower housing 112b. Then, the bottom portion of the filtering member 116 is located at the filtering material-fixing portion and immersed in the filling agent 149. Then, the filling agent 149 is hardened, with the front end of each pleat projecting into the inner side of the filtering member 116 locked to the rib 147. In this case, preferably, the filtering member 116 is fixed to the housing 112, with the axis of the filtering member 116 coincident with or proximate to that of the housing 112. A material similar to the filling agent 149 can be used.

In the bubble remover 100, blood introduced into the blood inlet-side space 117 in the housing 112 from the blood inlet port 1134 forms a spiral current therein. If the blood contains bubbles, the bubbles float upward in the upper housing 112a owing to buoyancy while they are whirling, with bubbles of small masses collect to the center of the spiral current owing to a centrifugal force. Then, the bubbles float upward along the inner surface of the upper part of the upper housing 112a and discharged to the outside from the air vent 115.

At this time, some bubbles which have not been removed may penetrate into the valley (gap between adjacent pleats 116a) of the pleats 116a. Bubbles float upward in a deep portion of the valley and attach to the lower surface 119a of the pleat-traverse portion of the filling material 119. But as described previously, the lower surface 119a inclines. Thus, the bubbles float upward along the inclined surface, thus escaping the valley. Thus, it is possible to prevent the bubbles from remaining the valley.

The blood from which the bubbles have been removed flows into the blood outlet-side space 118 through innumerable pores of the filtering member 116. Foreign matter contained in the blood cannot pass through the pores and filtered.

Even though tiny bubbles pass through pores of the filtering member 116 and flow into the blood outlet-side space 118, they float upward therein and arrive at the lower surface 119a of the filling material 119. Then, they float upward along the inclined lower surface 119a and move to the inner side of the mountain of each pleat 116a. A part thereof pass through pores of the filtering member 116 and return to the blood inlet-side space 117, thus floating upward and discharged to the outside from the air vent 115.

Next, a blood oxygenating circuit apparatus 150 of the present invention will be described below.

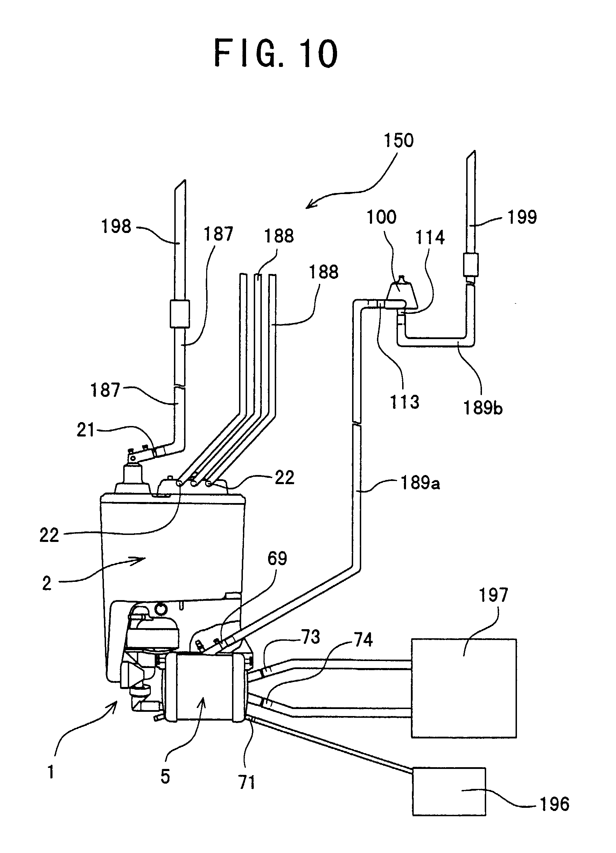

Fig. 10 is a conceptual view (circuit diagram) of an embodiment of the blood oxygenating circuit apparatus of the present invention.

The blood oxygenating circuit apparatus 150 comprises a blood reservoir 2 having a blood storing portion and a blood delivery mechanism including a blood reserving member having a delivery blood reserving portion communicating with the blood storing portion and reserving blood in an amount proportional to an amount of blood stored in the blood storing portion when the amount of the blood stored in the blood storing portion becomes equal to or less than a predetermined value and capable of flowing out blood stored in the delivery blood reserving portion when the delivery blood reserving portion is pressed from the outside; and an oxygenator 5 connected with a blood outlet port of the blood reservoir 2; and a bubble remover 100 located downstream from the oxygenator 5 and having a relationship between an internal capacity (ml) and a maximum use flow(L/min): internal capacity (ml)/maximum use flow (L/min) $\leqq 15$.

More specifically, the blood oxygenating circuit apparatus 150 includes the blood delivery mechanism-equipped blood reservoir 2 of the blood oxygenating apparatus 1; a vein line 187 connected with a venous blood inlet port (blood inlet port) 21 of the blood reservoir 2; a cardiotomy line 188 connected with a blood inlet port 22 of the blood delivery mechanism-equipped blood reservoir 2; a first artery line 189a connecting the blood outlet port 69 of the heat exchange function-equipped oxygenator 5 and the blood inlet port 113 of the bubble remover 100 with each other; and a second artery line 189b connected with the blood outlet port 114 of the bubble remover 100. The gas inlet port 71 of the oxygenator 5 is connected with an oxygen-containing gas feeder 196. A heating medium feeder 197 is connected with the heating medium inlet port 73 and the heating medium outlet port 74 of the oxygenator 5.

As the vein line 187, the cardiotomy line 188, and the artery lines 189a, 189b, a tube made of soft synthetic resin (for example, soft polyvinyl chloride tube, silicone rubber tube or the like) is used. A blood drainage catheter 198 is connected with an end (front end) of the vein line 187. A blood delivery catheter 199 is connected with an end (front end) of the second artery line 189b. As the oxygen-containing gas feeder 196, the heating medium feeder 197, the blood drainage catheter 198, and the blood delivery catheter 199, those known can be used.

The bubble remover 100 having the above-described construction is used.

As described previously, the blood oxygenating apparatus 1 having the blood delivery mechanism-equipped blood reservoir and used for the blood oxygenating circuit apparatus 150 includes the blood storing portion 10; the blood reservoir 2 having a blood delivery mechanism including the blood reserving members 3a, 3b provided with the delivery blood reserving portions 30a, 30b, respectively communicating with the blood storing portion 10 and reserving blood in an amount proportional to an amount of blood stored in the blood storing portion 10 when the amount of the blood stored in the blood storing portion 10 becomes equal to or less than a predetermined value and capable of flowing out blood stored in the delivery blood reserving portions 30a, 30b when the delivery blood reserving portions 30a, 30b are pressed from outside; and the heat exchange function-provided oxygenator 5 connected with the blood outlet port of the blood delivery mechanism-equipped blood reservoir 2. The blood outlet port 69 of the oxygenator 5 communicates with the upper end or its proximity of the blood chamber provided inside the oxygenator 5. Preferably, the blood outlet port communicates with the uppermost end of the blood chamber.

The blood oxygenating apparatus 1 having the blood delivery mechanism-equipped blood reservoir and used for the blood oxygenating circuit apparatus 150 includes the blood storing portion 10 storing a specified minimum reserving amount of blood; the blood reservoir 2 having the blood delivery mechanism including the blood reserving members 3a, 3b provided with the delivery blood reserving portions 30a, 30b, respectively communicating with the blood storing portion 10 and reserving blood in the amount proportional to the amount of blood stored in the blood storing portion 10 when the amount of the blood stored in the blood storing portion 10 becomes equal to or less than the predetermined value and capable of flowing out blood stored in the delivery blood reserving portions 30a, 30b when the delivery blood reserving portions 30a, 30b are pressed from outside; and the heat exchange function-provided oxygenator 5 connected with the blood outlet port of the blood delivery mechanism-equipped blood reservoir 2. The upper end of the blood chamber provided inside the oxygenator 5 is located at a position lower than the level of the surface of blood in the state where the blood storing portion 10 reserves the specified minimum amount of blood. The blood outlet port of the oxygenator 5 communicates with the upper end or its proximity of the blood chamber provided inside the oxygenator 5. Preferably, the blood outlet port communicates with the uppermost end of the blood chamber.

The blood oxygenating apparatus 1 having the blood delivery mechanism-equipped blood reservoir and used for the blood oxygenating circuit apparatus of the present invention is as described above and as shown in Figs.1 to 6.

According to the bubble remover for a blood oxygenating circuit, the bubble remover can display a high air chamber performance and has a very small internal capacity. Thus, priming capacity is small. Further, the bubble remover has the sufficiently large blood inlet and outlet ports, which secures a flow rate required for adults and reduces a pressure loss. Thus, the use of the bubble remover allows an amount of extracorporeal outflow blood to be small and a transfusion amount to be also small.

Further, the blood oxygenating circuit apparatus has an amount of extracorporeal outflow blood to be small and a transfusion amount to be also small.

The blood delivery mechanism-equipped blood reservoir of the present invention will be described below in detail with reference the drawings.

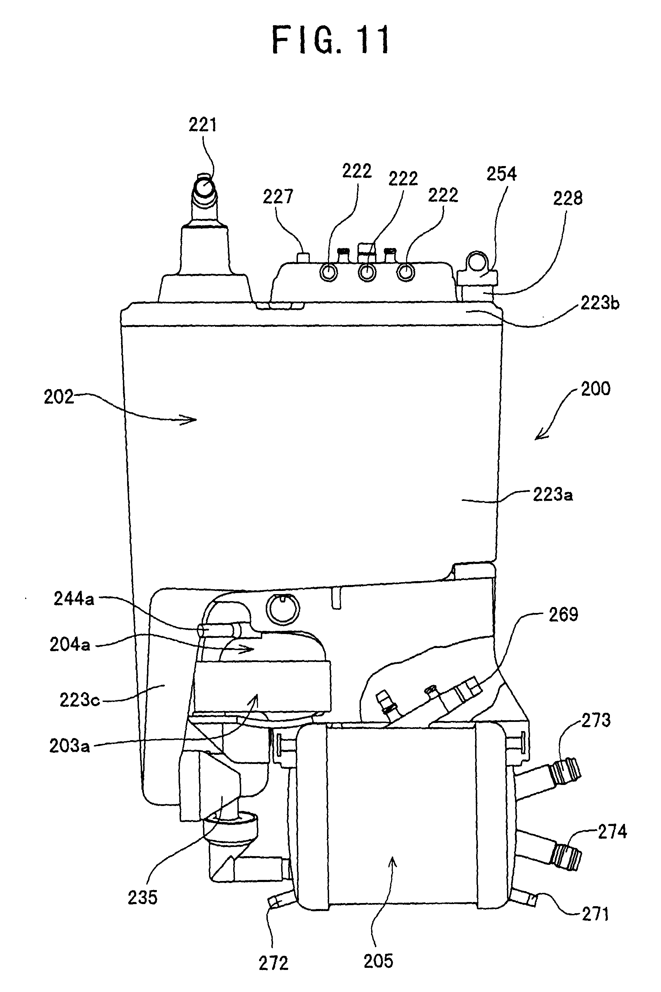

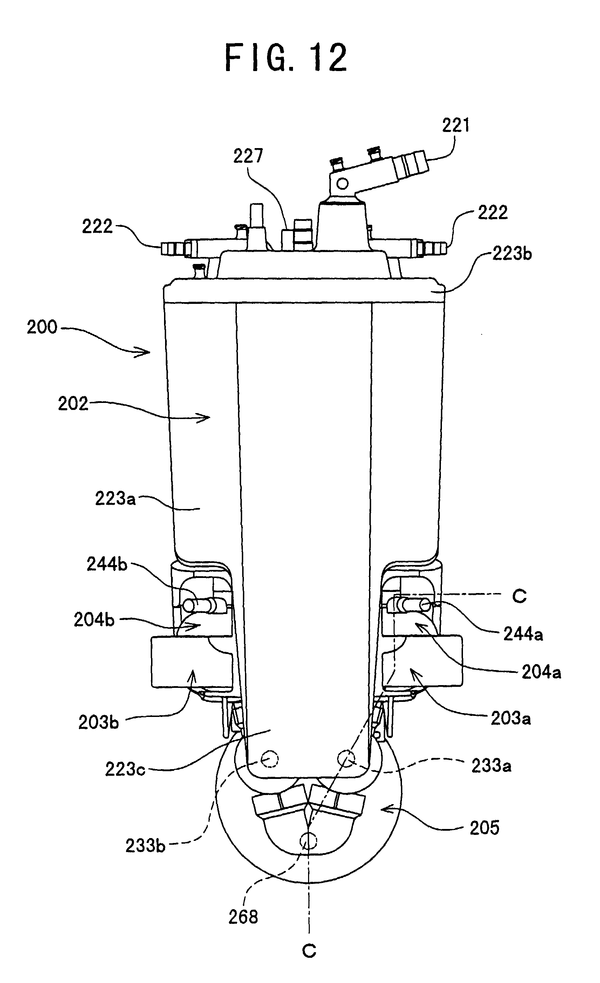

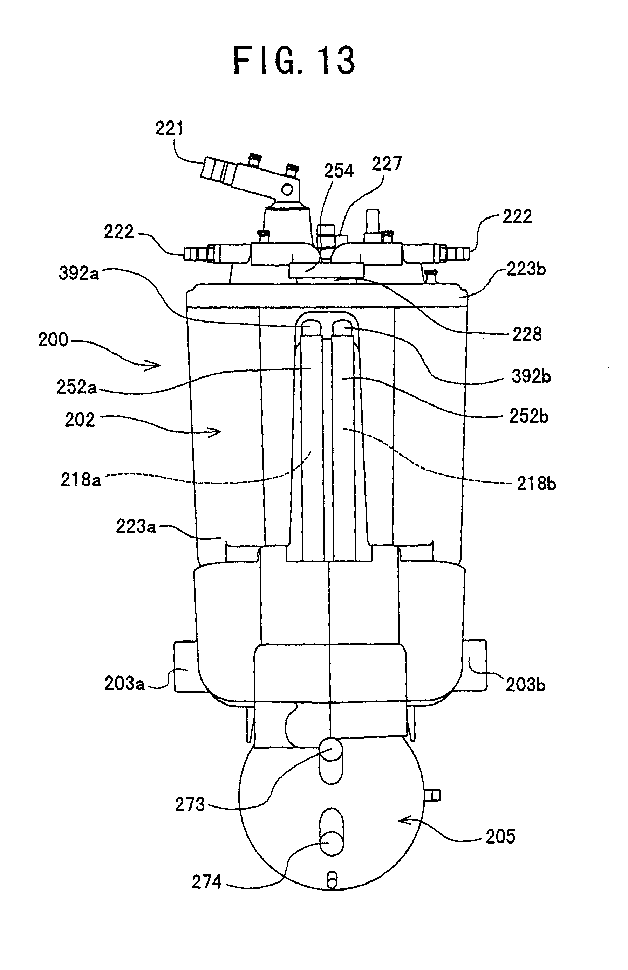

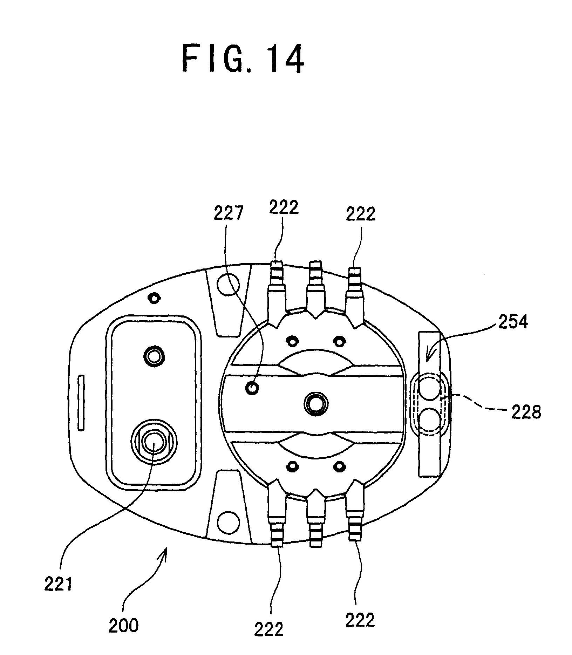

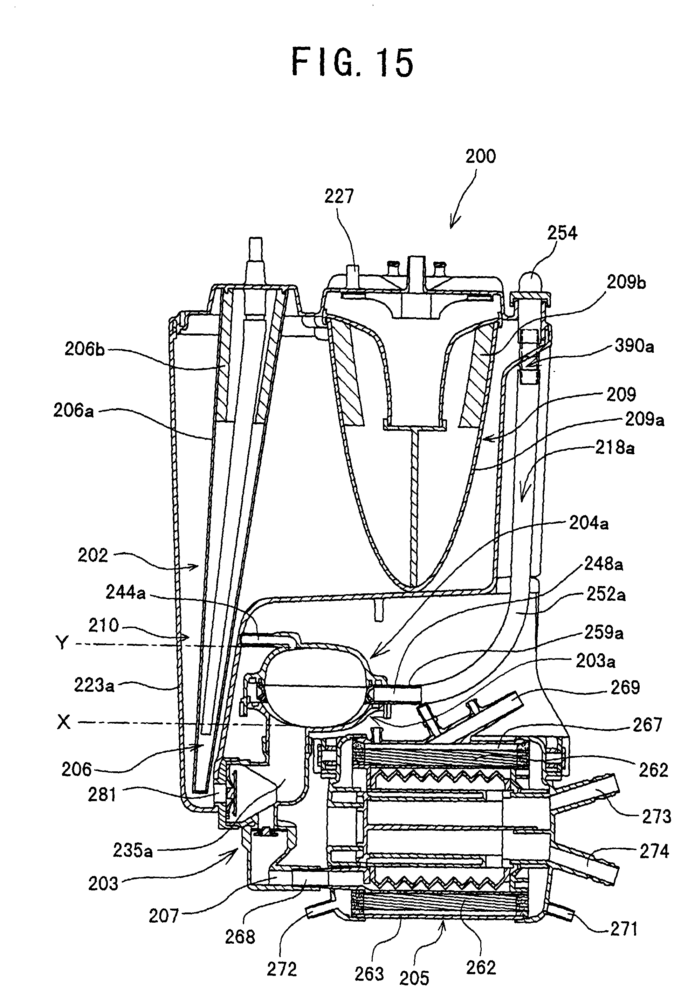

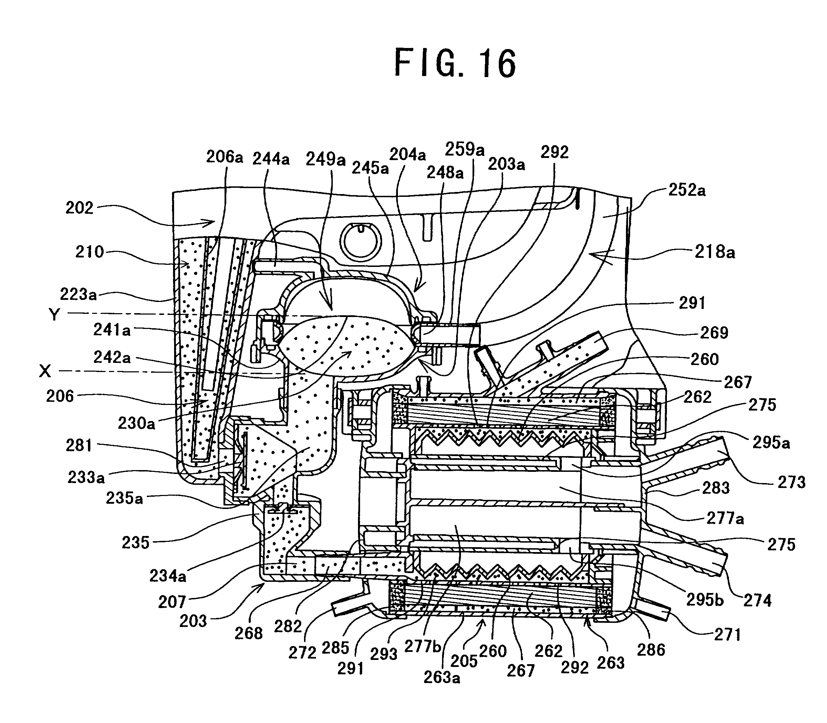

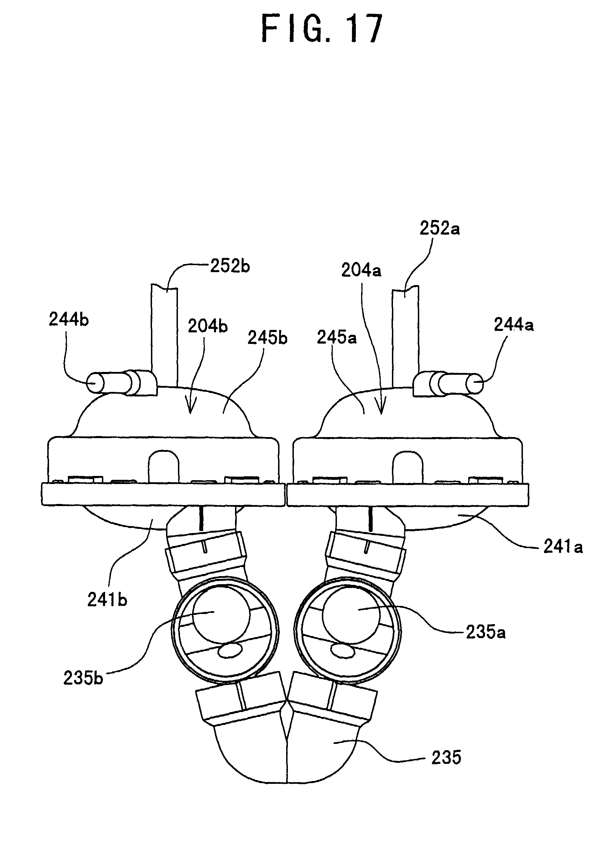

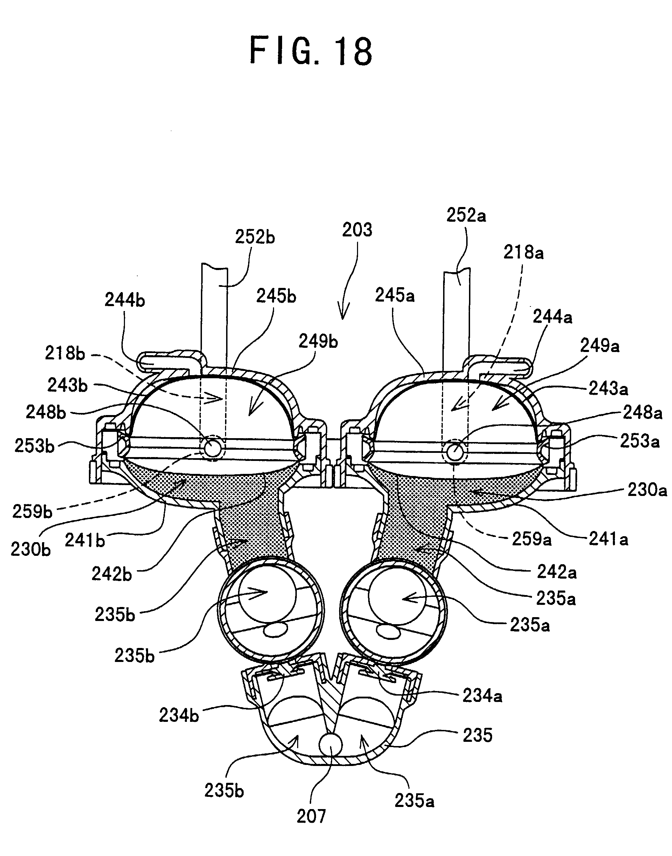

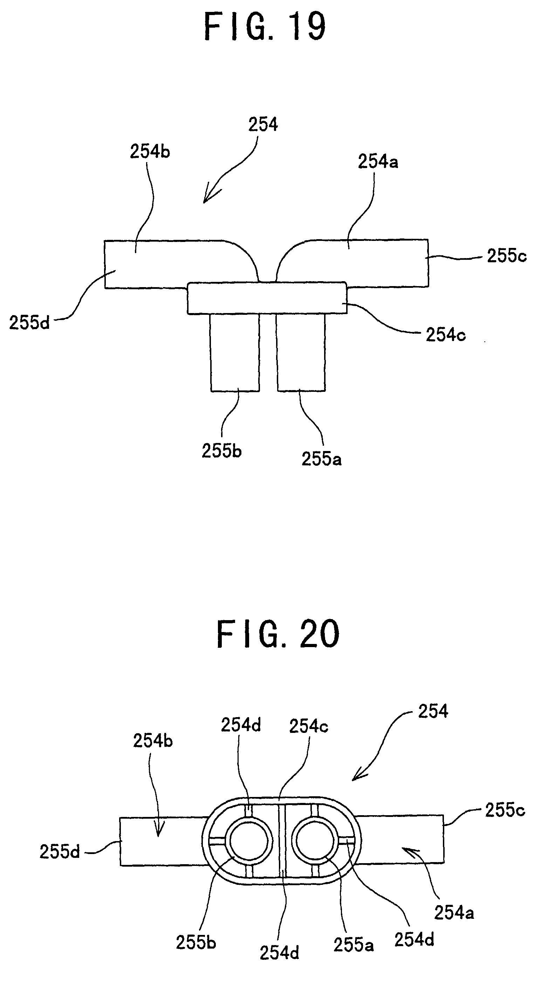

Fig. 11 is a front elevation of a blood delivery mechanism-equipped blood reservoir according to an embodiment of the present invention. Fig. 12 is a left side view of the blood delivery mechanism-equipped blood reservoir shown in Fig. 11. Fig. 13 is a right side view of the blood delivery mechanism-equipped blood reservoir shown in Fig. 11. Fig. 14 is a plan view of the blood delivery mechanism-equipped blood reservoir shown in Fig. 11. Fig. 15 is an explanatory view for explaining the internal construction of the blood delivery mechanism-equipped blood reservoir shown in Figs. 11 through 14. That is, Fig. 15 shows the blood reservoir and the heat exchange function-provided oxygenator and the blood delivery mechanism by cutting them along a center line of Fig. 12 and a broken line C-C of Fig. 12, respectively. Fig. 16 is an enlarged sectional view of the vicinity of the blood delivery mechanism-equipped blood reservoir shown in Fig. 15 and the vicinity of a blood storing portion. Fig. 17 is a front view of a blood delivery mechanism portion of the blood delivery mechanism-equipped blood reservoir shown in Figs. 11 through 14. Fig. 18 is a sectional view showing the blood delivery mechanism portion of the blood delivery mechanism-equipped blood reservoir shown in Fig. 17 and also showing the state of the flexible diaphragm at a non-pressing time (when blood is reserved in the delivery blood reserving portion at a non-delivery time). Fig. 19 is a front view of a first cap (used in non-sucking time) serving as a communication mode selection function. Fig. 20 is a bottom view of the first cap (used in non-sucking time) shown in Fig. 19.

Figs. 11, 14, and 15 show the state in which a cap (first cap 254) which is used at non-sucking time and which constitutes the communication mode selection function is installed.

A blood delivery mechanism-equipped blood reservoir 200 of the present invention includes a blood storing portion 210; and a blood delivery mechanism 203 including delivery blood reserving portions 203a, 203b communicating with the blood storing portion 210 and reserving blood in an amount proportional to an amount of blood stored in the blood storing portion 210 when the amount of the blood stored in the blood storing portion 210 becomes equal to or less than a predetermined value and capable of flowing out blood stored in the delivery blood reserving portions 203a, 203b when the delivery blood reserving portions 203a, 203b are pressed from the outside and blood delivery drive units 204a, 204b operating when the blood in the delivery blood reserving portions 203a, 203b is delivered. The blood delivery mechanism-equipped blood reservoir 200 can be connected with a sucking means (not shown) for sucking an interior of the blood storing portion 210. The blood delivery mechanism-equipped blood reservoir 200 includes communication passages 218a, 218b communicating an upper portion of the interior of the blood storing portion 210 with spaces (closed space) 249a, 249b formed between the delivery blood reserving portion 203a, 203b formed inside the blood delivery mechanism 203 and the blood delivering drive units 204a, 204b; and a communication mode selection function making it possible to select communication between the spaces 249a, 249b of the blood delivery mechanism 203 and the upper portion of the interior of the blood storing portion 210 or communication between the spaces 249a, 249b of the blood delivery mechanism 203 and the outside air.

The blood delivery mechanism-equipped blood reservoir 200 includes a blood reservoir portion 202; a blood delivery mechanism 203; and a heat exchange function-equipped oxygenator 205 installed on the blood reservoir portion 202 by hanging the former from the bottom surface of the latter. A blood inlet port 268 of the heat exchange function-equipped oxygenator 205 is connected with a blood discharge opening 207 of the blood reservoir 200. The blood storing portion 210 is a blood storing space of the blood reservoir portion 202.

As shown in Figs. 11 through 15, the blood reservoir portion 202 has a housing formed of a housing body 223a and a lid body 223b both made of hard resin. The lid body 223b covering an opening formed at an upper portion of the housing body 223a is fitted into an upper end of the housing body 223a. As shown in Figs. 11 through 15, the lid body 23b has blood inlet ports 221, 222, a rapid priming port 227, and a cap portion installing portion 228 on which a first cap member (used in non-sucking time) 254 and a second cap member (used in sucking time) 256 can be installed arbitrarily. The first cap member 254 and the second cap member constitute the communication mode selection function which will be described later. The blood inlet port 222 is connected with the cardiotomy line feeding blood fed from an operation field. The blood inlet port 221 is connected with a blood drainage line feeding blood delivered from a blood drainage cannula inserted into a patient's heart ascending/descending veins.

The housing body 223a of the blood reservoir portion 202 and the lid body 223b may be formed of any desired resin, for example, polycarbonate, acrylic resin, polyethylene terephthalate, polyethylene, polypropylene, polystyrene, polyvinyl chloride, acryl-styrene copolymers, and acryl-butadiene-styrene copolymers. Polycarbonate, acrylic resin, polystyrene, and polyvinyl chloride are especially preferred.

In the housing body 223a, there are provided a blood filter (venous blood filter) 206 serving as an anti-foaming member and a foam-removing member for filtering blood introduced into the housing body 223a from the blood inlet port 221; and a cardiotomy blood filter 209 for filtering blood introduced into the housing body 223a from the blood inlet port 222. The lower end of the blood inlet port 221 extends into the blood storing portion 10 and is positioned inside the blood filter 206. All blood introduced into the housing body 223a from the blood inlet port 221 flows into the blood storing portion 210 through the blood filter 206. Therefore, it is possible to prevent the blood introduced into the housing body 223a from the blood inlet port 221 from directly dripping to the surface of the blood inside the blood storing portion 210. More specifically, the blood filter 206 is fixed to an inner surface of the lid body 223b and extends toward a lower portion of the blood storing portion 210 such that the blood filter 206 encloses a projection portion of the blood inlet port 221 projecting into the blood storing portion 210.

The housing body 223a has a projection portion 223c projecting downward. The lower end of the blood filter 206 extends to a position in the vicinity of the projection portion 223c. In the state in which the specified minimum reserving blood amount is reserved in the blood storing portion 210, the lower end of the blood filter 206 is positioned below the level of the blood surface shown with X. That is, the lower end of the blood filter 206 is positioned below the lower end of the delivery blood reserving portions 230a and 230b of the blood reserving members 203a and 203b. In the state in which the specified minimum reserving blood amount is reserved in the blood storing portion 210, the lower end of the blood filter 206 is positioned below the surface of blood. Therefore, in the state where the storage blood amount in the blood storing portion 210 is reduced to a value below a predetermined value and the blood reserving members 203a and 203b are placed in the state where they display the function of reserving blood in an amount proportional to the amount of blood stored in the blood storing portion 210, the blood introduced into the blood storing portion 210 does not bubble. This is because the lower end of the blood filter 206 is located below the surface of the blood in the blood storing portion 210. When the amount of blood stored in the blood storing portion 210 increases and returns to a value above the predetermined value, the blood which flows into the blood storing portion 210 hardly bubbles because the lower end of the blood filter 206 is positioned below the surface of the blood in the blood storing portion 210.

The blood filter 206 has a filter member 206a and an anti-foaming member 206a installed on the entire periphery of the inner surface of an upper portion of the filter member 206a. Similarly, a cardiotomy blood filter 209 has a filter member 209b and an anti-foaming member 209a installed on the entire periphery of the inner surface of an upper portion of the filter member 209a.

To form the bubble eliminating members 206b, 209b or a bubble-removing member, various porous materials may suitably be used, including, but not limited to, foamed materials such as polyurethane foam, polyethylene foam, polypropylene foam, polystyrene foam and the like, mesh, woven fabrics, non-woven fabrics, porous ceramics, sintered materials of resin and the like. Materials having relatively small air passage resistance (pressure loss) are particularly preferred. If a material having less air passage resistance, for example, a foamed material such as polyurethane foam or the like or other porous material, is used, the pore size is preferably approximately within the range of 20 μm to 10 mm and, more favorably, approximately within the range of 50 μm to 5 mm.

It is preferable that the anti-foaming members 206b, 209b carry an anti-foaming agent having a function of breaking bubbles when bubbles contact them. As an anti-foaming agent, silicone (silica-compounded type, oil type or the like) is preferable. Examples of the method for allowing the anti-foaming members 206b, 209b to carry the anti-foaming agent include a method wherein the anti-foaming members 206b, 209b are dipped in a liquid containing the anti-foaming agent, and a method wherein a liquid containing the anti-foaming agent is applied or sprayed to the anti-foaming members 206b, 209b, and then dried (for example, at 30°C for 180 minutes).

The filter members 206a, 209b remove foreign matter or air bubbles from blood. A preferred material for the filter members 206a, 209b is preferably a porous material having a sufficiently high blood permeability. Examples of the porous material include mesh (net) materials, woven fabrics, non-woven fabrics and the like. Such materials may be used alone or in any desired combination (particularly, in the form of a laminate). It is preferred to use one or a combination of two or more of macromolecular materials that include polyesters such as PET, PBT or the like, rayon, Tetron®, nylon (polyamide), polyolefins such as polyethylene, polypropylene and the like. It is also possible to use metallic materials such as aluminum, stainless steel or the like. Particularly preferred are polyesters, polypropylene, polyethylene and stainless steel. The opening size of a mesh used for the filter members 206a, 209a is preferably about 15-300 μm and, more favorably, about 20-200 μm. It is also preferred to subject the filter members 206a, 209a to a plasma treatment or a hydrophilic treatment such as coating with a hydrophilic macromolecular material or the like, in order to improve blood permeability.

The blood storing portion 210 for temporarily reserving blood is formed in the blood reservoir portion 202, as shown in Fig. 11 and other figures. The capacity of the blood storing portion 210 is not specified. But normally, the capacity of the blood storing portion 210 is about 3,000 to 5,000 ml for adults and about 1,000 to 2,500 ml for children. The housing main body 223a is preferably substantially transparent or semi-transparent so that the amount and conditions of blood reserved therein may be readily observed. The downward projected portion 223c is a narrow portion with a reduced sectional area, whereby when the amount of blood reserved decreases, the amount of blood reserved or a change in the blood amount can be correctly and readily read. The blood reservoir portion 202 may be a soft type blood reservoir portion formed of a soft resin. In this case, the blood delivery mechanism-equipped blood reservoir portion 202 becomes a closed-type blood delivery mechanism-equipped blood reservoir portion.

As shown in Fig. 15, the blood delivery mechanism 203 is installed below the blood reservoir portion 202. The blood delivery mechanism 203 includes delivery blood reserving portions 203a, 203b; delivery blood reserving portion-pressurizing portion 243a, 243b for deforming a deformable portion, formed of a flexible material, of the delivery blood reserving portions 203a, 203b to force out blood in the delivery blood reserving portions 203a, 203b; a blood flow passage-forming portion 235 for connecting the delivery blood reserving portions 203a, 203b to the blood reservoir portion 202 and the oxygenator 205. The blood flow passage-forming portion 235 has a connection portion 281 connected with the blood reservoir portion 202. As shown in Figs. 15 through 18, the blood flow passage-forming portion 235 includes first and second blood flow passage portions 235a, 235b communicating with the blood storing portion 210 and the delivery blood reserving portions 203a, 203b; and a blood discharge opening 207.

The first blood flow passage portion 235a formed inside the blood flow passage-forming portion 235 communicates with only the first delivery blood reserving portion 203a. The second blood flow passage portion 235b formed inside the blood flow passage-forming portion 235 communicates with only the second delivery blood reserving portion 203b. Disposed at boundary sites (namely, inner side of the connection portion 281) between the blood storing portion 210 and the blood flow passage portions 235a, 235b are first check valves 233a, 233b that allow flow of blood from the blood storing portion 210 toward the blood flow passage portions 235a, 235b (namely, toward the delivery blood reserving portion 203a, 203b) and restricts (prevents) the reverse flow of blood.

The first check valves 233a, 233b function as passage control members for blocking the communication between the blood storing portion 210 and the delivery blood reserving members 3a, 3b during the operation of the blood delivering drive units 204 as described below. The blood delivery mechanism 203 is provided with the blood discharge opening 207 for communication with the blood flow passage portions 235a, 235b. Provided near the blood discharge opening 207 are second check valves 234a, 234b that allow blood flow toward a side downstream from the blood flow passage portions 235a, 235b (namely, the side of the delivery blood reserving portion 203a, 203b) and restricts the reverse flow of blood. The second check valves 234a, 234b function as passage control members for blocking reverse blood flow from the downstream side toward the delivery blood reserving members 203a, 203b (that is, into the blood flow passage portions 235a, 235b) when the blood delivering drive unit 204 is not operated.

Each of the check valves 233, 234 has a disc-shaped movable valve body a portion of which is secured to the housing. Preferably, the movable valve body of each check valve has a slightly less specific gravity than blood, and a hardness of about 30 to 90 on Shore A scale. The valve bodies are preferably formed of, for example, styrene-based elastomer oil gel, silicone gel, silicone rubber, fluororubber or the like, to a thickness of about 0.5 to 5 mm.

The blood delivery mechanism 203 includes a body portion; flexible diaphragms 242a, 242b which are accommodated in the body portion, with a periphery thereof retained; flexible diaphragm pressurizing portions 243a, 243b formed of an elastic sheet material; and blood delivering fluid flow ports 244a, 244b provided on the body portion and used to expand and contract the flexible diaphragm pressurizing portions 243a, 243b. The spaces 249a and 249b of the blood delivery mechanism 203 are formed of the body portion of the blood delivery mechanism 203 and a space between the diaphragm 242a and the diaphragm pressurizing portion 243a and between the diaphragm 242b and the diaphragm pressurizing portion 243b. The body portion of the blood delivery mechanism 203 includes delivery blood reserving body portions 241a, 241b both formed of a hard material; and blood delivering drive unit body portions 245a, 245b provided with the blood delivering fluid flow ports 244a, 244b, respectively. The delivery blood reserving portions 203a, 203b are constructed of the blood reserving body portions 241a, 241b, respectively and the flexible diaphragms 242a, 242b, the peripheries of which are held by the delivery blood reserving body portions 241a, 241b, respectively. The blood delivering drive units 204a, 204b are constructed of the flexible diaphragm pressurizing portions 243a, 243b and the blood delivering drive unit body portions 245a, 245b, respectively.

More specifically, the delivery blood reserving portions 203a, 203b include the blood reserving body portions 241a, 241b formed of a hard material; the flexible diaphragms 242a, 242b whose peripheries are held by the blood reserving body portions 241a, 241b; and an interior 230a (see Figs. 16 and 18) of the delivery blood reserving portion 203a formed between the blood reserving body portion 241a and the flexible diaphragm 242a and an interior 230b of the delivery blood reserving portion 203b formed between the blood reserving member body portion 241b and the flexible diaphragm 242b. The flexible diaphragms 242a, 242b are formed of a flexible material constituting a part of the delivery blood reserving portions 203a, 203b, respectively. The blood delivery mechanism-equipped blood reservoir 200 has the blood delivering drive units 204a, 204b which are operated in forcing out blood from the delivery blood reserving portions 203a, 203b by deforming the flexible diaphragm 242a, 242b and delivering the blood. The blood delivering drive units 204a, 204b will be described later.

Each of the blood reserving body portions 241a, 241b is provided with an inwardly concavity surface portion having the shape of a bowl. A peripheral end portion of each of the flexible diaphragms 242a, 242b is retained to a peripheral end portion of the concavity surface portion of the blood reserving body portions 241a, 241b in such a manner that the peripheral end portion of the flexible diaphragms 242a, 242b is inclined toward a central portion of the concavity surface portion. The entire inner surface of each of the blood reserving body portions 241a, 241b, including the peripheral end portion, is receded in the form of a bowl. This receded or concave surface of each of the blood reserving body portions 241a, 241b closely contacts the flexible diaphragms 242a, 242b at the time blood delivery. Thus, the interiors 230a, 230b of the delivery blood reserving portions 203a, 203b formed between the inner surface of the flexible diaphragms 242a, 242b and the concave surface of the blood reserving body portions 241a, 241b are spaces that are variable in capacity.

The peripheral end portion of the flexible diaphragm 242a, 242b is clamped between the peripheral end portion of the blood reserving body portions 241a, 241b (portion slightly inward from the peripheral end) and the outer peripheral portion of the diaphragm retaining annular members 253a, 253b.

The maximum amount of blood that can be reserved by the delivery blood reserving portions 203a, 203b (the maximum amount that can be contained therein, that is, the capacity thereof) varies depending on the capacity of the blood reservoir portion 202 used. However, the capacity of the delivery blood reserving portions 203a, 203b is preferably about 20-500 ml and, particularly, about 50-500 ml. More favorably, the capacity thereof is about 50-300 ml and, particularly, about 80-300 ml.

The flexible diaphragms 242a, 242b have a shape corresponding to the shape of the inner surfaces of the concavity surface portions of the blood reserving body portions 241a, 241b. It is preferred that the pressure needed to deform the diaphragms 242a, 242b be equal to or less than 100 mmH$_2$O, whereby the flexible diaphragms 242a, 242b become more sensitive in responding to changes in the amount of blood stored in the blood storing portion 210, so that reservation of amounts of blood proportional to the amount of blood stored in the blood storing portion 210 is more reliably ensured. The pressure needed to deform the flexible diaphragms 242a, 242b is more favorably equal to or less than 50 mmH$_2$O.

The diaphragms 242a, 242b may be suitably formed from, for example, polyurethane, silicone, polyvinyl chloride, and the like. Particularly preferred materials are polyurethane and silicone, which are presently widely used for members that contact blood.

It is preferred that the diaphragms 242a, 242b be sufficiently soft. As an index of softness, compliance may be employed. The diaphragms 242a, 242b of the delivery blood reserving portions 203a, 203b have a compliance of, preferably, 2 ml/sec•mmHg or higher and, more favorably, within the range of 5-30 ml/sec•mmHg, when the blood surface in the blood storing portion 210 of the blood reservoir 200 is lower than the uppermost portion (Y of Fig. 16) of the inner space of each of the delivery blood reserving portions 203a, 203b, that is, when the pressure sensitivity (liquid surface sensitivity) of the delivery blood reserving portions 203a, 203b is effective. It is further preferred that the diaphragms 242a, 242b of the delivery blood reserving portions 203a, 203b have a compliance that is lower than the aforementioned value, when the blood surface in the blood storing portion 210 of the blood reservoir 200 is higher than the uppermost portion (Y of Fig. 16) of the inner space of each of the delivery blood reserving portions 203a, 203b. The resistance against blood inlet into the delivery blood reserving portions 203a, 203b can be expressed by rate of blood inlet to the delivery blood reserving portions 203a, 203b. The rate of blood inlet to the delivery blood reserving portions 203a, 203b is preferably 20-600 ml/sec.

It is preferred to provide the flexible diaphragms 242a, 242b with a reinforcement (not shown) which covers the outside of the diaphragm and whose peripheral end portion is fixed to the blood reserving body portions 241a, 241b. Preferably, the diaphragm and the reinforcement combined have the following properties: the pressure needed to deform the diaphragm is equal to or less than 100 mmH$_2$O; the rupture strength is equal to or greater than 5 kg/cm$^2$; and the diaphragm itself produces substantially no self-restoring force against deformation. It is preferred that the diaphragm and the reinforcement of the delivery blood reserving portions 203a, 203b be not adhered to each other at all or except at the peripheral end portions thereof. The reinforcements may be provided in the form of a woven fabric, a non-woven fabric, a knitted fabric, a sheet material or the like. In view of strength, the form of a woven or knitted fabric is preferred. As for examples of the material of the reinforcements, polyethylene terephthalate, nylon 6, nylon 66, regenerated cellulose, polypropylene, fiber-reinforced plastics (FRP, with aramid fiber or the like), stainless steel, aluminum or the like may be suitably used. Particularly preferred are polyethylene terephthalate, nylon 6, nylon 66, regenerated cellulose and polypropylene.

Each interiors 230a, 230b of the delivery blood reserving portions 203a, 203b communicate with the blood passages 235a, 235b positioned at a lower portion (lower end) thereof. The delivery blood reserving portions 203a, 203b are positioned above the first check valves 233a, 233b defining boundary sites between the blood storing portion 210 and the blood flow passage portions 235a, 235b. The delivery blood reserving portions 203a, 203b extend substantially horizontally. If the blood surface in the blood storing portion 210 is below the uppermost end of the interiors 230a, 230b (inner space) of the delivery blood reserving portions 203a, 203b, amounts of blood proportional to the blood surface in the blood storing portion 210 flow into the delivery blood reserving portions 203a, 203b. If the blood surface in the blood storing portion 210 is above the uppermost end (Y of Fig. 16) of the 230a, 230b of the delivery blood reserving portion 203a, 203b at the time when the interiors 230a, 230b of the delivery blood reserving portions 203a, 203b expands to the maximum, the maximum amount of blood (state shown in Fig. 16) flows into the delivery blood reserving portions 203a, 203b, because the maximum capacity of the interiors 230a, 230b of the delivery blood reserving portions 203a, 203b is fixed.

More specifically, the delivery blood reserving portions 203a, 203b receive pressure proportional to the amount of blood stored in the blood storing portion 210, thus forming pressure-sensitive containers. If the amount of blood (liquid surface) in the blood storing portion 210 is equal to or greater than a predetermined amount (in this embodiment, the blood surface in the blood reservoir portion 2a is above the uppermost end of the inner space of the delivery blood reserving portions 203a, 203b, and the blood surface is above Y), the amount of blood reserved in the delivery blood reserving portions 203a, 203b is determined by the maximum capacity thereof, not by the pressure corresponding to the amount of blood stored in the blood storing portion 210. That is, the delivery blood reserving portions 203a, 203b reserve the maximum amount of blood. If the amount of blood stored in the blood reservoir portion 2a is equal to or less than the predetermined amount (in this embodiment, the blood surface in the blood storing portion 210 is below the uppermost end of the inner space of the delivery blood reserving portions 203a, 203b, and the blood surface is above Y), the pressure sensitivity of the delivery blood reserving portions 203a, 203b becomes effective so that the delivery blood reserving portions 203a, 203b reserve amounts of blood proportional to the amount of blood stored in the blood storing portion 210 (namely, the level of the blood surface in the blood storing portion 210). In short, the delivery blood reserving portions 203a, 203b function to automatically reserve amounts of blood proportional to the amount of blood stored in the blood storing portion 210 when the amount of blood in the blood storing portion 210 is equal to or less than the predetermined value.

The delivery blood reserving portions 203a, 203b do not spontaneously draw in blood, that is, do not substantially have self-shape-restoring characteristic. If the delivery blood reserving portions 203a, 203b were formed so that the members have a preset shape and self-shape-restoring characteristic, the delivery blood reserving portions 203a, 203b would restore their preset shape when the load from the drive members for forcing blood out of the delivery blood reserving portions 203a, 203b is removed after blood is thereby forced out. Then the self-restoring force of the delivery blood reserving portions 203a, 203b would cause a suction force, whereby blood would be drawn back into the delivery blood reserving portions 203a, 203b from the side of the blood reservoir portion 202. In short, the delivery blood reserving portions 203a, 203b would retain a certain minimum level of blood, below which the pressure sensitivity could not be effective.

The capacity of the blood flow passage portions 235a, 235b (that is, a blood passage partially defined by the blood inlet of the interiors 230a, 230b of the delivery blood reserving portions 203a, 203b and the first and second check valves 233a, 234a or 233b, 234b) of the blood delivery mechanism 203 is preferably equal to or less than 150 ml. Each of the blood flow passage portions 235a, 235b of the blood delivery mechanism 203 represents a blood passage partially defined by the first and second check valves 233a, 234a or 233b, 234b and the blood inlet of the interiors 230a, 230b of the delivery blood reserving portions 203a, 203b (the inner surface of the diaphragm 242a, 242b) when the flexible diaphragms 242a, 242b are closed or depressed.

The minimum sectional area of the blood flow passage portion 235a, 235bs is equal to or greater than 1.0 cm$^2$. With such a sectional area of the blood flow passage portions 235a, 235b, the pressure sensitivity of the delivery blood reserving portions 203a, 203b will not be impeded, but can be made sufficiently high so that the delivery blood reserving portions 203a, 203b will reserve amounts of blood proportional to the amount of blood in the blood storing portion 210 (that is, the blood surface in the blood reservoir portion 202) when the amount of blood stored is equal to or less than a predetermined value. It is preferred that the pressure loss of the blood flow passage portions 235a, 235b (the pressure loss of a blood passage) be as small as possible. In the present invention, the pressure loss is preferably 130 mmH$_2$O or less and, particularly, 110 mmH$_2$O and, more favorably, 100 mmH$_2$O. With such a pressure loss, a sufficiently high pressure sensitivity and good operation can be achieved. The minimum sectional area of the blood flow passage portions 235a, 235b is more favorably 1.5 cm$^2$ or greater, whereby a favorable pressure sensitivity of the blood reserving members can be ensured.

It is also preferred that the capacity of the blood flow passage portions 235a, 235b be smaller than the maximum amount of blood that can be reserved in the delivery blood reserving portions 203a, 203b (the maximum amount that can be contained therein), whereby the entire volume of blood in the blood flow passage portions 235a, 235b can be forced out thereof by blood discharged from the delivery blood reserving portions 203a, 203b, and the same blood is not reserved in the blood delivery mechanism 203.

The blood reserving body portions 241a, 241b may suitably be formed from, for example, polycarbonate, acrylic resin, polyethylene terephthalate, polyethylene, polypropylene, polystyrene, polyvinyl chloride, acryl-styrene copolymers, and acryl-butadiene-styrene copolymers, and the like. Particularly preferred materials are polycarbonate, acrylic resin, polystyrene, and polyvinyl chloride. In this embodiment, the blood reserving body portion 241a and the blood flow passage portion-forming portion 235 are separately provided and connected with each other. Similarly, the blood reserving body portion 241b and the blood flow passage portion-forming portion 235 are separately provided and connected with each other. However, it is possible to form the blood reserving body portion and the blood flow passage portion-forming portion by integrating them with each other.

The blood delivering drive units 204a, 204b are disposed outside the delivery blood reserving portions 203a, 203b in such a manner as to cover the deformable diaphragms of the delivery blood reserving portions 203a, 203b, respectively. The blood delivering drive units 204a, 204b and the delivery blood reserving portions 203a, 203b form a blood delivery mechanism.

The blood delivering drive units 204a, 204b are provided with the delivery blood reserving portion-pressurizing portions 243a, 243b (diaphragm-pressurizing portion), respectively formed of an expandable sheet material and operating to deliver blood out of the corresponding delivery blood reserving portions 203a, 203b. More specifically, the blood delivering drive units 204a, 204b include the blood delivering fluid flow ports 244a, 244b, respectively extending horizontally outwardly from a central portion thereof and blood delivering drive unit body portions 245a, 245b formed of a hard material.

In each of the delivery blood reserving portion-pressurizing portion 243a, 243b, an annular portion slightly inward from a peripheral end portion thereof is clamped between the blood delivering drive unit body portions 245a, 245b and a peripheral end portion of the diaphragm retaining annular members 253a, 253b, and thereby substantially air-tightly retained. Each of the diaphragm retaining annular members 253a, 253b retains both the diaphragms 242a, 242b and the delivery blood reserving portion-pressurizing portions 243a, 243b, and controls excessive expansion of the diaphragms 242a, 242b. Since the delivery blood reserving portion-pressurizing portions 243a, 243b of the blood delivering drive units 204a, 204b are expandable or stretchable sheet members for expansion and contraction achieved by a fluid, the incidence of damaging or breaking the diaphragm by pressurization thereon is minimum. Furthermore, a relatively inward peripheral end portion of each of the delivery blood reserving portion-pressurizing portions 243a, 243b is restricted by the inclined annular surface of the diaphragm retaining annular members 253a, 253b when the delivery blood reserving portion-pressurizing portions 243a, 243b are expanded.

For use, a blood delivering fluid supply machine (not shown) is connected to the blood delivering fluid flow ports 244a, 244b. By supplying and discharging a fluid (liquid or gas) by a compressor provided in the blood delivering fluid supply machine, the expanding and contracting driving of the delivery blood reserving portion-pressurizing portions 243a, 243b is repeated. The delivery blood reserving portion-pressurizing portions 243a, 243b of the blood delivering drive units 204a, 204b do not contact the diaphragms 242a, 242b of the delivery blood reserving portions 203a, 203b when not expanded (contracted) as shown in Fig. 15. When expanded, the delivery blood reserving portion-pressurizing portions 243a, 243b of the blood delivering drive units 204a, 204b pressurize the diaphragms 242a, 242b of the delivery blood reserving portions 203a, 203b in cooperation with the blood reserving body portions 241a, 241b, thereby discharging blood out of the interior of the delivery blood reserving portion 203a, 203b (the interior of the interiors 230a, 230b of the delivery blood reserving portions 203a, 203b).

Preferred examples of the elastic material used to form the delivery blood reserving portion-pressurizing portions 243a, 243b include synthetic rubbers such as urethane rubber, silicone rubber, butadiene rubber and the like, natural rubbers such as latex rubber and the like, and elastomers such as olefin-based elastomer, amide-based elastomer, styrene-based elastomer (for example, styrene-butadiene-styrene copolymers, styrene-isoprene-styrene copolymers, styrene-ethylenebutylene-styrene copolymers), polyurethane elastomer and the like.

In the blood delivery mechanism-equipped blood reservoir 200 of the embodiment, the diaphragms 242a, 242b of the delivery blood reserving portions 203a, 203b are surrounded with the delivery blood reserving body portions 241a, 241b, and are not exposed to the outside. Thus, the function of protective covers for the diaphragms are provided.

The blood delivery mechanism-equipped blood reservoir 200 has communication passages 218a, 218b which allow the communication between the upper portion of the interior of the blood storing portion 210 and the spaces 249a, 249b formed between the delivery blood reserving portions 203a, 203b formed inside the blood delivery mechanism 203 and the blood delivering drive units 204a, 204b.

In the embodiment, the communication passages 218a, 218b is constructed of openings 248a, 248b of the blood delivery mechanism 203; ports 259a, 259b communicating with the openings 248a, 148b, respectively; tubular bodies 252a, 252b, one end of each of which is connected with the ports 259a, 259b, respectively; and communication passage forming ports 390a, 390b formed on the blood reservoir portion housing body 223a and connected with the other end of the tubular bodies 252a, 252b, respectively.

The blood delivery mechanism 203 includes the openings 248a, 248b communicating with the spaces 249a, 249b formed between the delivery blood reserving portions 203a, 203b (diaphragms 242a, 242b) and the blood reserving member pressurizing portions 243a, 243b, namely, the space between the delivery blood reserving portions 203a, 203b and the blood delivering drive units 204a, 204b); and the ports 259a, 259b communicating with the openings 248a, 248b, respectively. More specifically, the openings 248a, 248b are formed at a portion at which the blood delivering drive unit body portions 245a, 245b and the blood reserving member body portions 241a, 241b are connected with each other. The ports 259a, 259b are airtightly fixed to the openings 248a, 248b, respectively. An opening is formed on the diaphragm retaining annular members 253a, 253b at a position thereof corresponding to the openings 248a, 248b.

The blood delivery mechanism-equipped blood reservoir 200 has the communication mode selection function making it possible to select the communication between the upper portion of the interior of the blood storing portion 210 and the spaces 249a, 249b formed between the delivery blood reserving portions 203a, 203b formed inside the blood delivery mechanism 203 and the blood delivering drive units 204a, 204b or the communication between the spaces 249a, 249b of the blood delivery mechanism 203 and the outside air.

The communication mode selection function of the blood delivery mechanism-equipped blood reservoir 200 is constructed of the first cap 254 (used in non-sucking time); the second cap (used in sucking time) 256; and a cap portion installing portion 228 of the lid body 223b.

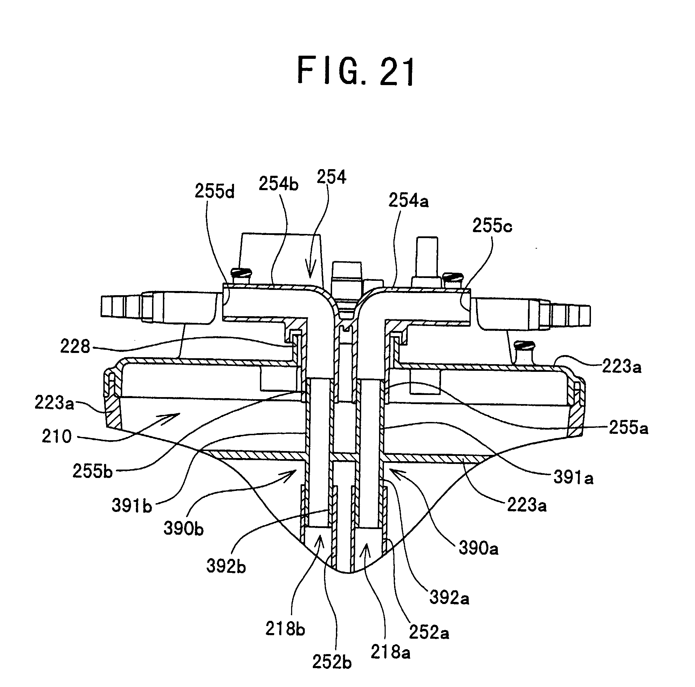

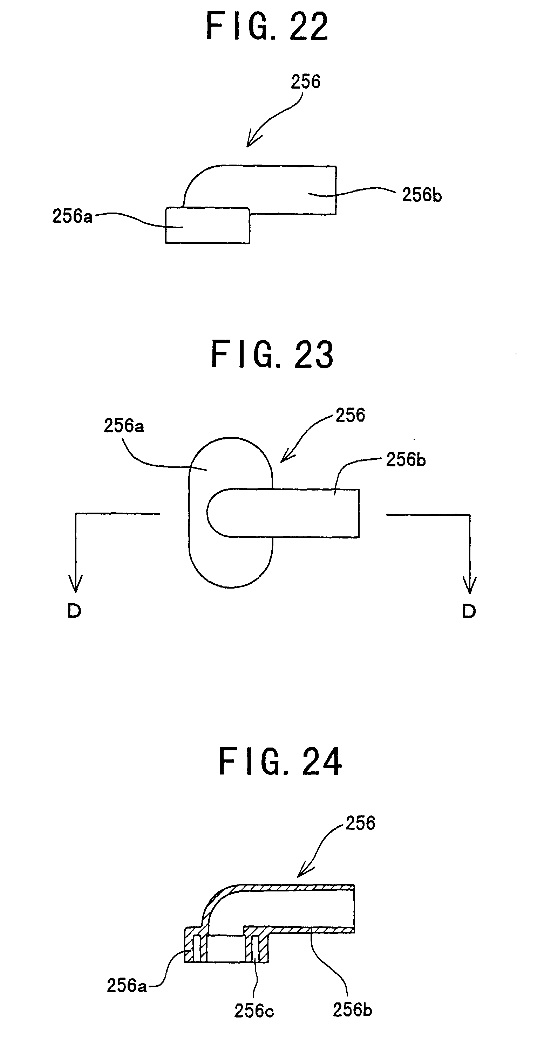

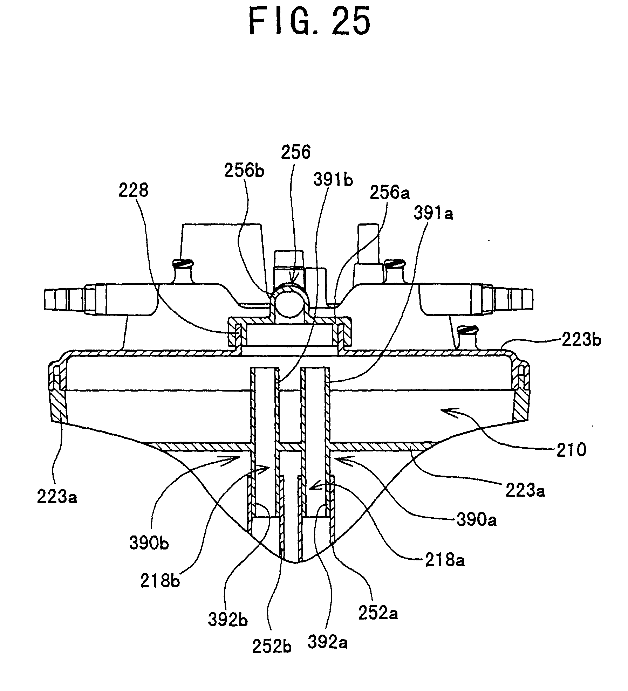

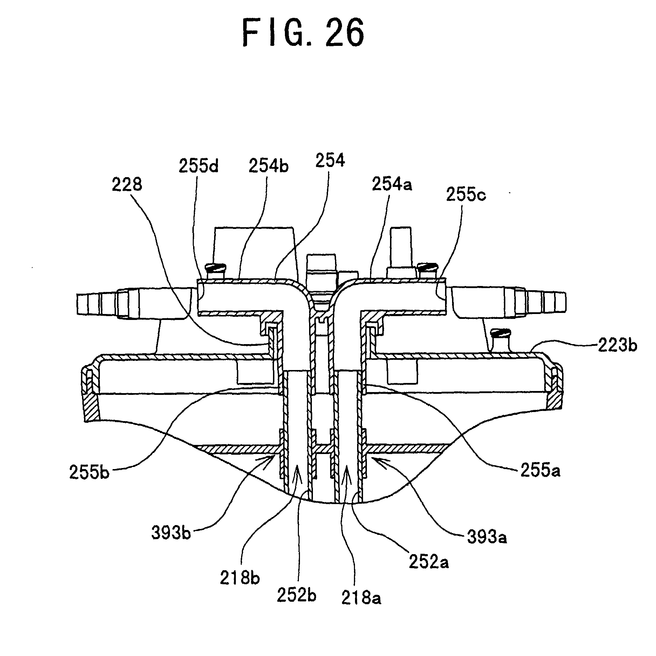

Fig. 19 is a front view of a first cap (used in non-sucking time) serving as a communication mode selection function. Fig. 20 is a bottom view of the first cap (used in non-sucking time) shown in Fig. 19. Fig. 21 is an enlarged sectional view of the neighborhood of a communication passage of a blood delivery mechanism-equipped blood reservoir in the state where the first cap (used in non-sucking time) is installed. Fig. 22 is a front view of a second cap (used in sucking time) serving as a communication mode selection function. Fig. 23 is a plan view of the second cap (used in sucking time) shown in Fig. 22. Fig. 24 is a sectional view taken along a line D-D of Fig. 23. Fig. 25 is an enlarged sectional view of the neighborhood of a communication passage of a blood delivery mechanism-equipped blood reservoir in the state where the second cap (used in sucking time) is installed. Fig. 26 is an enlarged sectional view of the neighborhood of a communication passage of a blood delivery mechanism-equipped blood reservoir in the state where a first cap (used in non-sucking time) according to another embodiment is installed.

As shown in Figs. 15 and 21, in the housing body 223a of the blood reservoir portion, a region positioned below the cap portion installing portion 228 is formed as a proximate region to the lid body 223b. The communication passage forming ports 390a, 390b are formed in the proximate portion located below the cap portion installing portion 228. The communication passage forming ports 390a, 390b have tubular body connection ports 392a, 392b; and installing ports 391a, 391b located inside the housing body 223a and used to install thereon lower ends 255a, 255b of tubular portions 254a, 254b of the first cap 254 extending toward the cap portion installing portion 228 of the lid body 223b.

The mode of connecting the housing body 223a and the tubular bodies 252a, 252b with each other is not limited to the above-described mode, but the construction shown in Fig. 26 may be adopted. In the construction shown in Fig. 26, a region, of the housing body 223a, located below the cap portion installing portion 228 of the lid body 223b is formed as the proximate region to the lid body 223b, similarly to the above-described construction. The communication passage forming ports 392a, 392b connecting the tubular bodies 252a, 252b with each other substantially airtightly are formed below the cap portion installing portion 228 of the proximate region. Inside the housing body 223a, the communication passage forming ports 392a, 392b guide the front end of the tubular bodies 252a, 252b toward the cap portion installing portion 228 of the lid body 223b. The front end of the tubular bodies 252a, 252b can be installed on lower ends 255a, 255b of the tubular portions 254a, 254b of the first cap 254.

The first cap 254 (used in non-sucking time) has the tubular portions 254a, 254b whose one end can be connected with one end of the communication passages 218a, 218b and whose other end is open and located outside the blood storing portion 210 when the first cap member 254 is installed. The second cap 256 (used in sucking time) has an installation portion 256a which is installed on the cap portion installing portion 228 substantially airtightly and a tubular portion 256b which extends outward and an end of which constitutes a sucking means connection portion.

More specifically, as shown in Figs. 19, 20, and 21, the first cap 254 (used in non-sucking time) has a body portion 254c formed at the center thereof; and the tubular portions 254a, 254b whose one end extends into the interior (below the installing portion 254c) of the housing body 223a and whose other end is curved and extends outward in parallel with the body portion 254c. Fig. 21 is an enlarged sectional view showing an end portion of the communication passage of the blood delivery mechanism-equipped blood reservoir and the neighborhood thereof in the state where the first cap 254 (used in non-sucking time) is installed. The lower ends (one end) 255a, 255b of the tubular portions 254a, 254b are open and can be connected with the installing ports 391a, 391b substantially airtightly. The upper ends (other end) 255c, 255d of the tubular portions 254a, 254b are also open. In the embodiment, the body portion 254c of the first cap 254 covers the cap portion installing portion 228, with the body portion 254c not in contact therewith.

The interior of the blood storing portion 210 communicates with the outside air through the space between the first cap 254 and the cap portion installing portion 228. As shown in Fig. 20, in the embodiment, a plurality of ribs 254d are formed on the bottom surface of the body portion 254c of the first cap 254. Thus, even though the ribs 254d contact the end surface of the cap portion installing portion 228, except the region of the ribs 254d, the interior of the blood storing portion 210 communicates with the outside air through the space between the first cap 254 and the cap portion installing portion 228.

In the state where the first cap 254(used in non-sucking time) is installed on the blood delivery mechanism-equipped blood reservoir 200, the spaces 249a, 249b (space between the diaphragms 242a, 242b and the blood reserving member pressurizing portions 243a, 243b) of the blood delivery mechanism 203 communicate with the outside air directly through the communication passages 218a, 218b. Therefore, the interior of the blood storing portion 210 is not affected by the movement of gas from the spaces 249a, 249b caused by the operation of the diaphragms 242a, 242b and that of the blood reserving member pressurizing portions 243a, 243b. When the blood reserving member pressurizing portions 243a, 243b are elastically deformed after delivery fluid (gas) is introduced into the blood reserving member pressurizing portions 243a, 243b to deliver blood, the gas flows into the spaces 249a, 249b. If the spaces 249a, 249b were in direct communication with the interior of the blood storing portion 210, the blood storing portion 210 would suck the outside air in correspondence to an amount of the gas which flows into the spaces 249a, 249b. Therefore, the blood storing portion 210 would suck bacteria floating in the air present in the neighborhood of the air port. In the blood delivery mechanism-equipped blood reservoir of the present invention, the spaces 249a, 249b do not communicate directly with the interior of the blood storing portion 210. Therefore, the blood storing portion 210 does not suck the outside air despite the movement of the gas in the spaces 249a, 249b.

Because the tubular portions 254a, 254b of the first cap 254 are bent, the open front ends (other end) 255c, 255d of the tubular portions 254a, 254b of the first cap 254 is not directed upward but sideways, thus preventing foreign matter from penetrating into the tubular body.

In sucking blood when necessary, the first cap (used in non-sucking time) 254 is removed, and the second cap (used in sucking time) 256 shown in Figs. 22 through 25 is installed on the cap portion installing portion 228. Fig. 25 is an enlarged sectional view of the neighborhood of the communication passage of the blood delivery mechanism-equipped blood reservoir in the state where the second cap (used in sucking time) is installed. The second cap (used in sucking time) 256 has the installing portion 256a to be installed on the cap portion installing portion 228 substantially airtightly and the sucking means connection portion 256b which is curved and extends outward from the center of the installing portion 256a. As shown in Fig. 24, the installing portion 256a has an annular groove 256c accommodating an end of the cap portion installing portion 228. A sucking means not shown is connected with the sucking means connection portion 256b.

In the state where the second cap (used in sucking time) 256 is installed on the blood delivery mechanism-equipped blood reservoir 200, the spaces 249a, 249b (space between the diaphragms 242a, 242b and the blood reserving member pressurizing portions 243a, 243b) of the blood delivery mechanism 203 are sucked by the sucking means through the communication passages 218a, 218b and the blood storing portion 210. In other words, the spaces 249a, 249b of the blood delivery mechanism 203 and the blood storing portion 210 are sucked. Consequently, the pressure condition (pressure-reduced condition) inside the spaces 249a, 249b becomes the same as that in the blood storing portion 210. When the interior of the blood delivery mechanism-equipped blood reservoir portion is sucked and is in a negative-pressure state, the spaces 249a, 249b between the delivery blood reserving portions 203a, 203b (the diaphragms 242a, 242b) and the blood reserving member pressurizing portions 243a, 243b are in a negative-pressure state at the same time. Thus, no pressure difference is generated between the interior of the blood storing portion 210 and the spaces 249a, 249b. Consequently, the blood delivery mechanism 203 is not impeded in its fluid surface sensitivity (pressure sensitivity, pre-load sensitivity) function. Thus, the delivery blood reserving portions 203a, 203b can display the liquid surface-sensitive performance, according to the amount (level of fluid) of blood stored in the blood storing portion 210.

It is preferable to form the first and second caps of a soft material or a flexible material to allow the first cap to have high airtight performance for the communication passage and the second cap to have high airtight performance for the cap portion installing portion 228. The flexible material or the elastic material includes polyurethane (polyester-based polyurethane, polyether-based polyurethane) soft polyvinyl chloride, polyester, synthetic rubbers such as urethane rubber, silicone rubber, butadiene rubber and the like, and elastomers such as olefin-based elastomer (polyethylene elastomer, polypropylene elastomer), amide-based elastomer (polyamide elastomer), styrene-based elastomer (for example, styrene-butadiene-styrene copolymers, styrene-isoprene-styrene copolymers, styrene-ethylenebutylene-styrene copolymers).

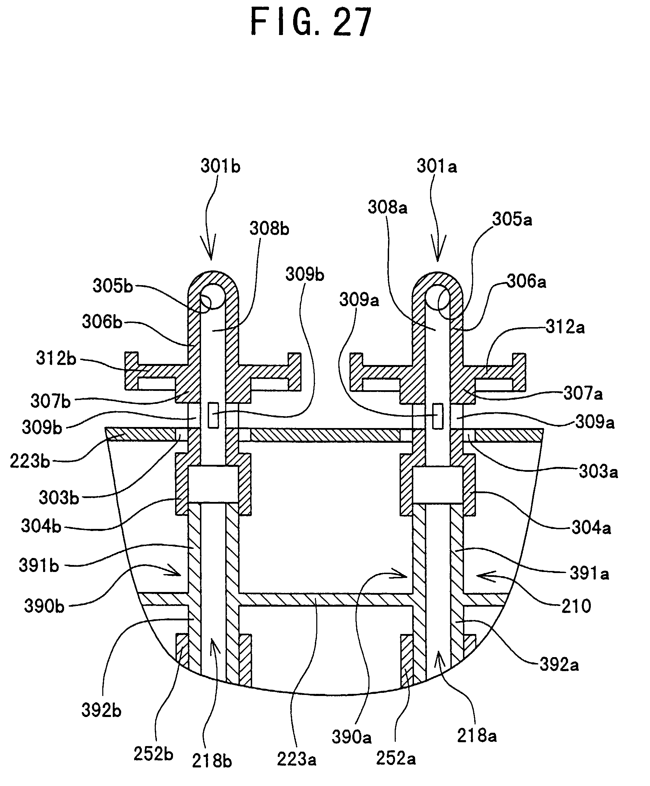

The communication mode selection function of an embodiment shown in Figs. 27 through 29 will be described below.

In the embodiment, the blood delivery mechanism-equipped blood reservoir has communication mode selection openings 303a, 303b formed on the lid body 223b. As the communication mode selection function, the blood delivery mechanism-equipped blood reservoir has two communication mode selection members 301a, 301b. The communication mode selection members 301a, 301b may be formed integrally with each other. As shown in Figs. 27 through 29, a region of the housing body 223a located below the communication mode selection openings 303a, 303b of the lid body 223b is formed as a proximate region to the lid body 223b. Similarly to the embodiment shown in Fig. 25, communication passage forming ports 390a, 390b are formed in the portion located below the communication mode selection openings 303a, 303b. The communication passage forming ports 390a, 390b have tubular body connection ports 392a, 392b extending outwardly downwardly of the housing 223a to connect the tubular bodies 252a, 252b thereto; and installing ports 391a, 391b located inside the housing body 223a and extending to the communication mode selection openings 303a, 303b to install thereon the communication mode selection members 301a, 301b. The mode of connecting the housing body 223a and the tubular bodies 252a, 252b with each other is not limited to the above-described mode, but the construction shown in Fig. 26 may be adopted.

The communication mode selection members 301a, 301b have one ends (lower ends) 304a, 304b which can be connected with the installing ports 391a, 391b substantially airtightly. The one ends (port installing portions) 304a, 304b are large-diameter portions. The communication mode selection members 301a, 301b have tubular bodies 306a, 306b located outside the blood storing portion 210 when they are installed on the installing ports 391a, 391b and having the other end constituting sucking means connection portions 305a, 305b; and fittable portions 307a, 307b provided on the tubular bodies 306a, 306b and airtightly fittable in the communication mode selection openings 303a, 303b. The fittable portions 307a, 307b are large-diameter portions. The communication mode selection members 301a, 301b has through-passages 308a, 308b extending from the one ends (port installing portions) 304a, 304b thereof to the other ends (sucking means connection portions) 305a, 305b. The communication mode selection members 301a, 301b have a plurality of side holes 309a, 309b located at the side (port installing portion) of one end (port installing portions) 304a, 304b with respect to the fittable portions 307a, 307b and communicating the tubular portion (passages 308a, 308b) and the outside with each other. Disk-shaped portions 312a, 312b are formed above the fittable portions 307a, 307b to prevent foreign matter from penetrating into the blood storing portion 210 through the communication mode selection openings 303a, 303b. A cylindrical rib is formed at an end of the disk-shaped portions 312a, 312b to use the cylindrical rib as a gripping portion in operating the communication mode selection members 301a, 301b.

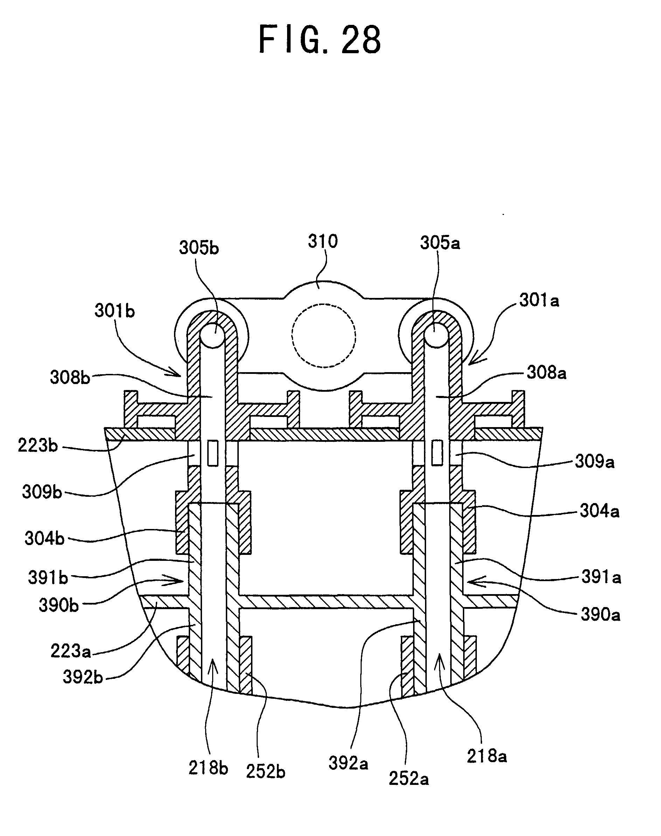

The communication mode selection members 301a, 301b can be moved from the state shown in Fig. 27 to the state shown in Fig. 28 and from the state shown in Fig. 28 to the state shown in Fig. 27, with the communication mode selection members 301a, 301b in connection with the communication passages (port installing portions 304a, 304b).

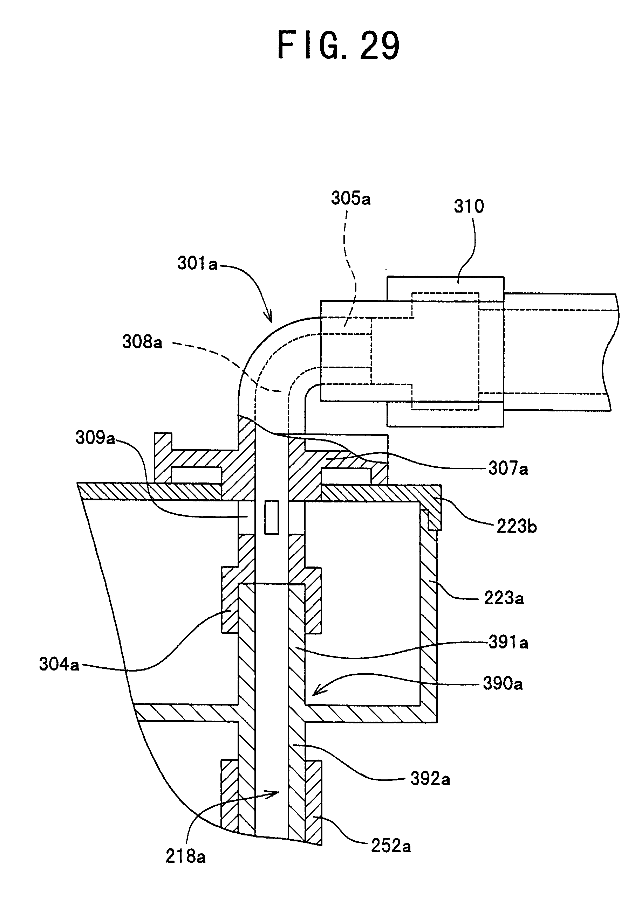

As shown in Fig. 27, the interior of the blood storing portion 210 communicates directly with the outside air through the communication mode selection openings 303a, 303b in the state where the fittable portions 307a, 307b of the communication mode selection members 301a, 301b are not fitted in the communication mode selection openings 303a, 303b. In this state, the sucking means is not connected with the sucking means connection portions 305a, 305b. Thus, the spaces 249a, 249b (the space between the diaphragm 242a, 242b and the delivery blood reserving portion-pressurizing portion 243a, 243b) of the blood delivery mechanism 203 communicate directly with the outside air through the communication passages 218a, 218b, the sucking means connection portions(open ends) 305a, 305b, and the side holes 309a, 309b. Therefore, the interior of the blood storing portion 210 is not affected by the movement of gas from the spaces 249a, 249b caused by the operation of the diaphragms 242a, 242b and that of the blood reserving member pressurizing portion 243a, 243b. In the communication mode selection members 301a, 301b, the tubular bodies 306a, 306b are bent toward the sucking means connection portions 305a, 305b, as shown in Fig. 29. Thus, the sucking means connection portions 305a, 305b which are the open end are directed not upward but sideways, thus preventing foreign matter from penetrating into the tubular body.

In sucking blood when necessary, as shown in Figs. 28 and 29, the communication mode selection members 301a, 301b are pressed downward to fit the fittable portions 307a, 307b thereof into the communication mode selection openings 303a, 303b airtightly. As a result, the side holes 309a, 309b are situated inside the blood storing portion 210, and a connector 310 (shown in Fig. 29) constituting a part of the sucking means is connected with the sucking means connection portions 305a, 305b. In this state, the interior of the blood storing portion 210 communicates with the passages 308a, 308b formed in the tubular bodies 306a, 306b of the communication mode selection members 301a, 301b through the side holes 309a, 309b and is prevented from communicating with the outside. Through the communication passages 218a, 218b, the spaces 249a, 249b (space between the diaphragm 242a, 242b and the blood reserving member pressurizing portion 243a, 243b) of the blood delivery mechanism 203 and the blood storing portion 210 can be sucked by the sucking means. Consequently, the pressure condition (pressure-reduced condition) inside the spaces 249a, 249b becomes the same as that in the blood storing portion 210. When the interior of the blood delivery mechanism-equipped blood reservoir portion is sucked and is placed in a negative-pressure state, the spaces 249a, 249b between the delivery blood reserving portions 203a, 203b (the diaphragms 242a, 242b) and the blood reserving member pressurizing portions 243a, 243b is placed in a negative-pressure state at the same time. Thus, no pressure difference is generated between the interior of the blood storing portion 210 and the spaces 249a, 249b. Consequently, the blood delivery mechanism 203 is not impeded in its fluid surface sensitivity (pressure sensitivity, pre-load sensitivity) function. Thus, the delivery blood reserving portions 203a, 203b can display the liquid surface-sensitive performance, according to the amount (level of fluid) of blood stored in the blood storing portion 210.

It is preferable to form the communication mode selection members 301a, 301b of a soft material or a flexible material to allow it to have high airtight performance for the communication passage and allow the fittable portions 307a, 307b to have high airtight performance for the communication mode selection openings 303a, 303b.

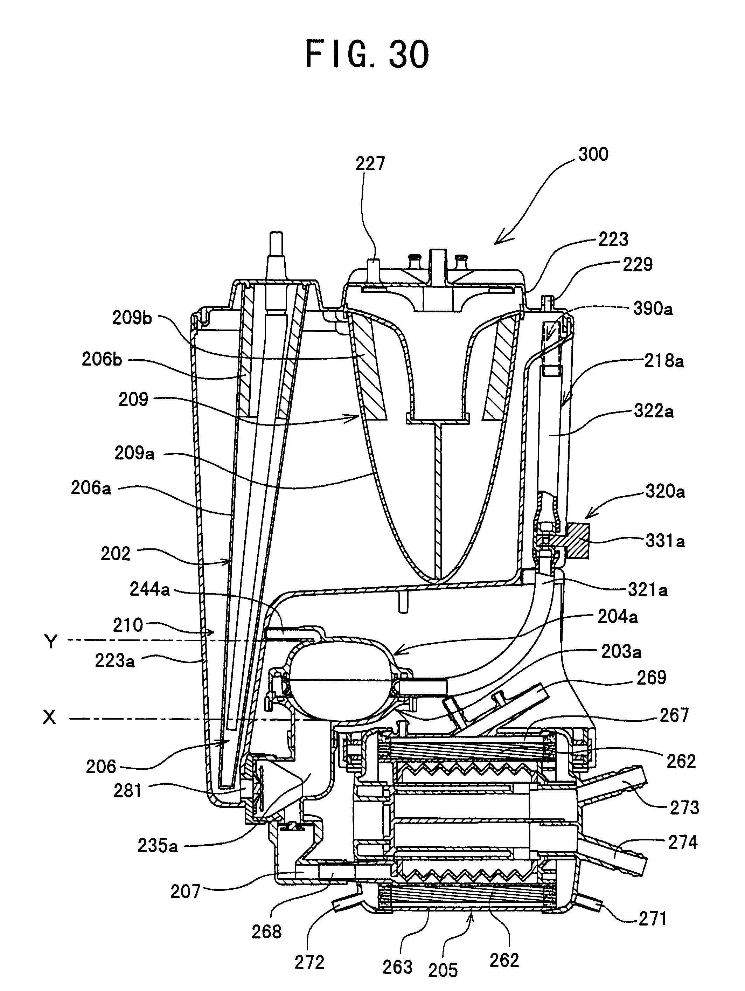

The communication mode selection function of an embodiment shown in Figs. 30 through 32 will be described below.

A blood reservoir 300 of the embodiment has communication mode change-over members 320a, 320b formed on the communication passages 218a, 218b. The blood reservoir 300 has a sucking means connection portion. An air port serves as the sucking means connection portion. The communication mode change-over members 320a, 320b have operation portions 331a, 331b and outside air communication ports 326a, 326b. By operating the operation portions 331a, 331b, it is possible to change the state in which the spaces 249a, 249b of the blood delivery mechanism 203 are in communication with the outside air to the state in which the spaces 249a, 249b are in communication with only the upper portion of the blood storing portion 210 and vice versa.

More specifically, a tubular body constituting a part of the communication passage 218a is constructed of two tubular bodies 321a (at the side of the blood storing portion 210), 322a (at the side of the space 249a of the blood delivery mechanism 203). The communication mode change-over member 320a is installed between the two tubular bodies 321a, 322a. Similarly, a tubular body constituting a part of the communication passage 218b is constructed of two tubular bodies 321b (at the side of the blood storing portion 210), 322b (at the side of the space 249b of the blood delivery mechanism 203). The communication mode change-over member 320b is installed between the two tubular bodies 321b, 322b.

The communication mode change-over member 320a includes a first port 324a connected with the tubular body 321a located at the side of the blood storing portion 210; a second port 325a connected with the tubular body 322a located at the side of the space 249a of the blood delivery mechanism 203; a body portion 330a having the outside air communication port (port open to outside air) 326a; and the change-over cock (operation portion) 331a rotatably accommodated in the body portion 330a. The change-over cock 331a serves as the operation portion of the communication mode change-over member 320a. The body portion 330a has a circular arc rib 332a consisting of 1/4 of the inner circumference of the open end thereof and projecting inward. The change-over cock 331a has a change-over lever; ribs 333a, 334a contacting the rib 332a of the body portion 330a; and a T-shaped passage 327a formed therein.

Similarly, the communication mode change-over member 320b includes a first port 324b connected with the tubular body 321b located at the side of the blood storing portion 210; a second port 325b connected with the tubular body 322b located at the side of the space 249b of the blood delivery mechanism 203; a body portion 330b having the outside air communication port (port open to outside air) 326b; and the change-over cock (operation portion) 331b rotatably accommodated in the body portion 330b. The body portion 330b has a circular arc rib 332a consisting of 1/4 of the inner circumference of the open end thereof and projecting inward. The change-over cock 331b has a change-over lever; ribs 333b, 334b contacting the rib 332b of the body portion 330b; and a T-shaped passage 327b formed therein.

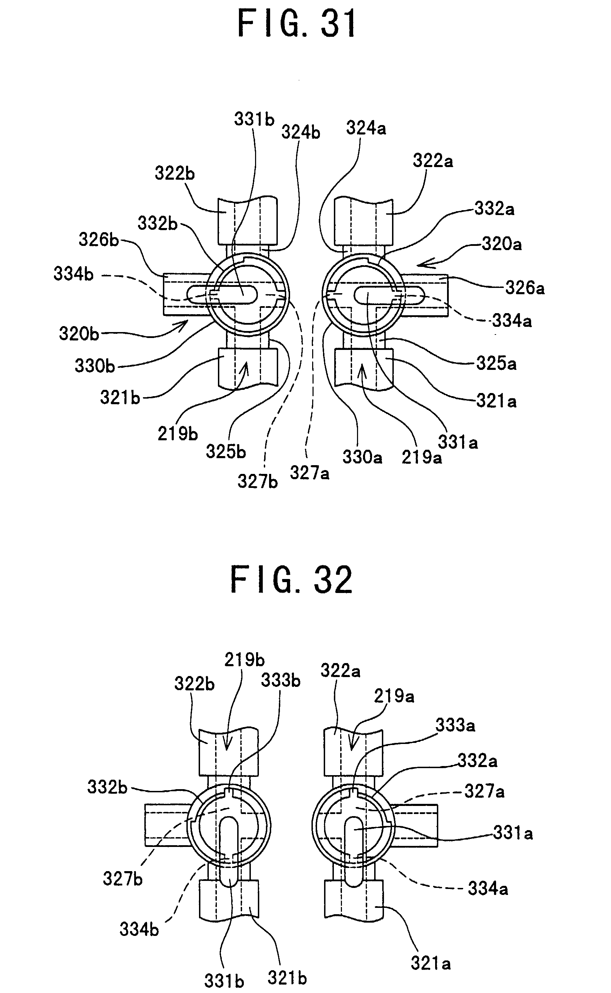

At a non-sucking time, the communication mode change-over members 320a, 320b are placed in the state shown in Fig. 31. In this state, the tubular bodies 321a, 321b at the side of the blood storing portion 210 do not communicate with the tubular bodies 322a, 322b at the side of the spaces 249a, 294b of the blood delivery mechanism 203; and the tubular bodies 322a, 322b at the side of the space 249 of the blood delivery mechanism 203 communicate with only the outside air through the passages 327a, 327b and the outside air communication ports 326a, 326b. In this state, the ribs 334a, 334b of the change-over cocks 331a, 331b contact the rib 332b of the body portions 330a, 330b. Therefore, the change-over cocks 331a, 331b are incapable of rotating upward.

At a sucking time, the sucking means (not shown) is connected with the sucking means connection portion (in the embodiment, air port 229). The sucking means connection portion may be provided separately from the air port 229. At the sucking time, a sealing cap is installed on the rapid priming port 227. The change-over cocks 331a, 331b of the communication mode change-over members 320a, 320b are rotated downward (counterclockwise) until the ribs 333a, 333b contact the rib 332b of the body portions 330a, 330b. As a result, as shown in Fig. 32, the tubular bodies 321a, 321b at the side of the blood storing portion 210 and the tubular bodies 322a, 322b at the side of the space 249 of the blood delivery mechanism 203 communicate with the sucking means connection portion through the passages 327a, 327b; and the outside air communication ports 326a, 326b do not communicate with the tubular bodies 321a, 321b nor the tubular bodies 322a, 322b. Accordingly, through the communication passages 218a, 218b, the spaces 249a, 249b (space between the diaphragm 242a, 242b and the blood reserving member pressurizing portion 243a, 243b) of the blood delivery mechanism 203 and the blood storing portion 210 can be sucked by the sucking means.

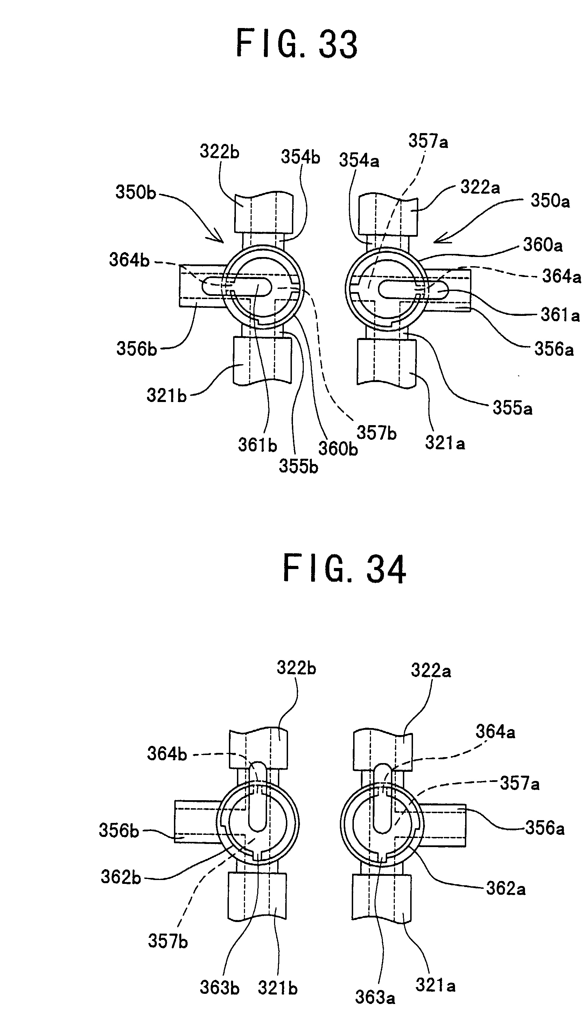

The communication mode selection function of an embodiment shown in Figs. 33 and 34 will be described below.

The basic construction thereof is similar to that of the blood delivery mechanism-equipped blood reservoir 300 shown in Fig. 30. The blood reservoir of the embodiment has also communication mode change-over members 350a, 350b formed on the communication passages 218a, 218b. The blood reservoir does not have a particular sucking means connection portion. The communication mode change-over members 350a, 350b have operation portions 361a, 361b and outside air communication port/sucking portion connection portions 356a, 356b. By operating the operation portions 361a, 361b, it is possible to change the state in which the spaces 249a, 249b of the blood delivery mechanism 203 are in communication with the outside air to the state in which the spaces 249a, 249b and the upper portion of the blood storing portion 210 communicate with the outside air communication port/sucking portion connection portions 356a, 356b.

More specifically, a tubular body constituting a part of the communication passage 218a is constructed of two tubular bodies 321a (at the side of the blood storing portion 210), 322a (at the side of the space 249a of the blood delivery mechanism 203). The communication mode change-over member 350a is installed between the two tubular bodies 321a, 322a. Similarly, a tubular body constituting a part of the communication passage 218b is constructed of two tubular bodies 321b (at the side of the blood storing portion 210), 322b (at the side of the space 249b of the blood delivery mechanism 203). The communication mode change-over member 350b is installed between the two tubular bodies 321b, 322b.

The communication mode change-over member 350a includes a first port 344a connected with the tubular body 351a located at the side of the blood storing portion 210; a second port 355a connected with the tubular body 352a located at the side of the space 249a of the blood delivery mechanism 203; a body portion 360a having the outside air communication port/sucking portion connection portion 356a; and a change-over cock (operation portion) 361a rotatably accommodated in the body portion 360a. The change-over cock 361a serves as the operation portion of the communication mode change-over member 350a. The body portion 360a has a circular arc rib 362a consisting of 1/4 of the inner circumference of the open end thereof and projecting inward. The change-over cock 361a has a change-over lever; ribs 363a, 364a contacting the rib 362a of the body portion 360a; and a T-shaped passage 357a formed therein.

Similarly, the communication mode change-over member 350b includes a first port 344b connected with the tubular body 351b located at the side of the blood storing portion 210; a second port 355b connected with the tubular body 352b located at the side of the space 249b of the blood delivery mechanism 203; a body portion 360b having the outside air communication port/sucking portion connection portion 356b; and a change-over cock (operation portion) 361b rotatably accommodated in the body portion 360b. The body portion 360b has a rib 362b consisting of 1/4 of the inner circumference of the open end thereof and projecting inward. The change-over cock 361b has a change-over lever; ribs 363b, 364b contacting the rib 362a of the body portion 360b; and a T-shaped passage 357b formed therein.

At a non-sucking time, the communication mode change-over members 350a, 350b are placed in the state shown in Fig. 33. In this state, the tubular bodies 321a, 321b at the side of the blood storing portion 210 do not communicate with the tubular bodies 322a, 322b at the side of the spaces 249a, 249b of the blood delivery mechanism 203; and the tubular bodies 322a, 322b at the side of the space 249a, 249b of the blood delivery mechanism 203 communicate with only the outside air through the passages 357a, 357b and the outside air communication ports/ sucking portion connection portions 356a, 356b. In this state, the ribs 364a, 364b of the change-over cocks 361a, 361b contact the rib 362b of the body portions 360a, 360b. Therefore, the change-over cocks 361a, 361b is incapable of rotating downward.

At a sucking time, the sucking means is connected with the sucking means connection portion 356a, 356b, and a sealing cap is installed on the rapid priming port 227 and the air port 229. The change-over cocks 361a, 361b of the communication mode change-over members 320a, 320b are rotated downward until the ribs 363a, 363b contact the rib 362b of the body portions 360a, 360b. As a result, as shown in Fig. 34, the tubular bodies 321a, 321b at the side of the blood storing portion 210 and the tubular bodies 322a, 322b at the side of the spaces 249a, 249b of the blood delivery mechanism 203 communicate with sucking means connection portion 356a, 356b through the passages 357a, 357b. Accordingly, through the communication passages 218a, 218b (the tubular bodies 321a, 321b and the tubular bodies 322a, 322b), the spaces 249a, 249b (space between the diaphragm 242a, 242b and the blood reserving member pressurizing portion 243a, 243b) of the blood delivery mechanism 203 and the blood storing portion 210 can be sucked by the sucking means.

In the blood delivery mechanism-equipped blood reservoirs of the above-described embodiments, it is possible to adjust the amount of blood which flows out from the delivery blood reserving portions 203a, 203b by adjusting the amount or pressure of a fluid to be introduced into the blood delivery drive unit 204a, 204b. The amount of blood to be delivered may be easily changed by adjusting the amount or pressure of the fluid to be fed into the blood delivering drive unit while fixing the number of times of driving the blood delivering drive unit per predetermined length of time.

As described above, the blood delivery mechanism-equipped blood reservoir 200 of this embodiment is equipped with the two sets of the delivery blood reserving portions 203 and the blood delivering drive units 204. Further, the two separate blood flow passage portions 235 (235a, 235b) are provided without communication therebetween, as shown in Figs. 15 and 16. By providing two or more sets thereof in this manner, the capacity of each of the delivery blood reserving portions 203a, 203b and each of the blood delivering drive units 204a, 204b can be reduced, so that the blood inflow and discharge response improves. Furthermore, if two or more sets of the delivery blood reserving portions 203 and the blood delivering drive unit 204 are provided, good blood flow may be achieved by shifting the expanding strokes of the blood delivering drive units 204a, 204b in timing from each other. If the expanding timing of the blood delivering drive units 204a, 204b is thus shifted, flow of blood from one delivery blood reserving portion to the other in this embodiment never occurs since the individual delivery blood reserving portions are provided with the separate blood flow passage portions 235a, 235b. The construction of the present invention is not limited by this embodiment. The number of sets of the delivery blood reserving portion and the blood delivering drive unit may also be one, or three or more. In addition, although the blood delivering drive units 204a, 204b in this embodiment repeat expansion and contraction through fluid operation, the blood delivering drive unit is not restricted by this driving manner. For example, the blood delivering drive unit may mechanically drive a pressurizing plate to pressurize the blood reserving member.

If two sets of the delivery blood reserving portion and the blood delivering drive unit are provided as in blood delivery mechanism-equipped blood reservoir 200, and furthermore, the individual blood delivering drive units can be separately controlled by a fluid supply machine for blood delivery, it becomes possible to select the form of blood flow for delivery, more specifically, select a pulse flow or a constant flow, considering the conditions of a patient, the type of the oxygenator used or the like. It becomes also possible to change the form of delivery blood flow while in use. More specifically, a substantially constant blood flow can be achieved by shifting the phases of the blood flow out of the individual delivery blood reserving portions 203a, 203b by substantially 180° from each other, that is, by shifting the phases of fluid flowing into and out of the blood delivering drive units 204a, 204b by substantially 180° from each other. A good pulse flow can be achieved by setting the phases of blood flow out of the individual delivery blood reserving portions 203a, 203b to substantially the same phase or within ±30°, that is, by setting the phases of fluid flowing into and out of the blood delivering drive units 204a, 204b to substantially the same phase or within ±30°. If three or more sets of the delivery blood storing member and the blood delivering drive unit are provided, the form of blood flow will become a substantially constant flow by shifting the phases of blood flow out of the individual delivery blood reserving portions by an angle obtained by dividing 360° by the number of the sets provided.

Next, the heat exchange function-equipped oxygenator 205 will be described below.

The heat exchange function-provided oxygenator 205 includes a cylindrical heat exchanger portion 260; a cylindrical hollow fiber bundle 262 composed of a large number of gas exchange hollow fibers and accommodating the cylindrical heat exchanger portion 260 therein; a housing 263 accommodating the cylindrical hollow fiber bundle 262 and the cylindrical heat exchanger portion 260 therein; partitioning walls 285, 286 for fixing either end of the cylindrical hollow fiber bundle 262 to the housing 263 and to the cylindrical heat exchanger portion 260, with either end of the hollow fiber open; a blood chamber 267 formed of an inner surface of the housing 263, an outer surface of the hollow fiber, an outer surface of the cylindrical heat exchanger portion 260, and the partitioning walls 285, 286; and a blood inlet port 268 and a blood outlet port 269 both formed on the housing 263 and communicating with the blood chamber 267; a gas inlet port 271 and a gas outlet port 272 both communicating with an interior of the hollow fiber; and a heating medium inlet port 273 and a heating medium outlet port 274 both communicating with an interior of the cylindrical heat exchanger portion 260.

The housing 263 of the oxygenator 205 of the embodiment shown in Fig. 15 includes a cylindrical housing body 263a; a first header 283 having the gas inlet port 71, the heating medium inlet port 273, and the heating medium outlet port 274; and a second header 282 having the gas outlet port 272. The blood inlet port 268 is formed at a position in the vicinity of the upper end of the side surface of the cylindrical housing body 263a.

The cylindrical hollow fiber bundle 262 is wound on the outer surface of a core 291 having a large number of openings 292 formed thereon. The cylindrical heat exchanger portion 260 is accommodated inside the core 291. A blood communication portion 293 (a part of the blood chamber 267) communicating with the blood inlet port 268 and the blood outlet port 269 is formed between an inner surface of the core 291 and an outer surface of the cylindrical heat exchanger portion 260. The blood chamber 267 is formed between an outer surface of the hollow fiber and an inner surface of the cylindrical housing body 263a. Formed inside the cylindrical heat exchanger portion 260 is a heating medium communication chamber 276 communicating with the heating medium inlet port 273 and the heating medium outlet port 274.

In the oxygenator 205, blood introduced into the oxygenator 205 from the blood inlet port 268 is heat-exchanged while it is flowing between the inner surface of the core 291 and the outer surface of the cylindrical heat exchanger portion 260. Then, the blood flows into the cylindrical hollow fiber bundle 262 (into the blood chamber 267) from the opening 292 of the core 291. Upon contact between the blood and the hollow fiber, gases contained in the blood are exchanged with each other. The blood which has passed through the cylindrical hollow fiber bundle 262 flows into an annular space (a part of the blood chamber 267) formed between the cylindrical hollow fiber bundle 262 and the cylindrical housing body 263a and then flows out from the blood outlet port 269. The annular space may not be provided.

Preferably, the outer diameter of the cylindrical hollow fiber bundle 262 is 30 - 144 mm. Preferably, the filling factor of the cylindrical hollow fiber bundle 262 is 45 - 75%.

A porous fiber is used to make the gas exchange hollow fiber. Favorably, the inner diameter of the porous hollow fiber is favorably, 50 - 1000 $\mu$m, and more favorably, 54.0 - 84.0 $\mu$m. Favorably, the thickness of the porous hollow fiber is 5 - 200 $\mu$m, and more favorably, 10 - 100 $\mu$m. The outer diameter of the porous hollow fiber is favorably, 66.0 - 106.0 $\mu$m. The porosity of the porous hollow fiber is favorably, 20 - 80%, and more favorably, 30 - 60%. The diameter of the pore of the porous hollow fiber is favorably, 0.01 - 5 $\mu$m, and more favorably, 0.01 - 1 $\mu$m. Hydrophobic polymers are used to form the porous hollow fiber. Exemplary hydrophobic polymers include polypropylene, polyethylene, polysulfone, polyacrylonitrile, polytetrafluoroethylene, and cellulose acetate. Preferred among others are polyolefin resins, especially polypropylene. Porous hollow fibers of polypropylene having micropores formed thereon by drawing method or solid-liquid phase separation method are desirable. Preferably, the thickness of the cylindrical hollow fiber bundle 262 is in the range of 5 - 25 mm.

The partitioning walls 285 and 286 are formed of an adhesive and elastic potting material made of such as polyurethane, silicone rubber, and the like. The cylindrical hollow fiber bundle 262, the cylindrical heat exchanger portion 260, and the housing 263 are not necessarily coaxial with one another.

The cylindrical heat exchanger portion 260 of bellows type is preferable. A cylindrical member of bellows type which is a component part of the outer surface of the cylindrical heat exchanger portion 260 is formed of metal such as stainless steel, aluminum or the like or a resinous material such as polyethylene, polycarbonate or the like. These materials are formed in the shape of bellows having fine pleats formed thereon. As the material of the cylindrical member of bellows type, metal such as stainless steel or aluminum is more favorable in consideration of strength and heat exchange effectiveness thereof. The cylindrical heat exchanger portion 260 of the embodiment is formed as a bellow pipe having a large number of convex and concave portions formed repeatedly and substantially perpendicularly to the axial direction thereof. In consideration of the heat exchange effectiveness of the cylindrical heat exchanger portion 260, the difference in height between a mountain of the bellows and a valley thereof is favorably 5.0 - 15.0mm and more favorably 9.0 - 12.0 mm.

The cylindrical heat exchanger portion 260 having an axial length of 70.0 - 150 mm is used, although the axial length thereof is different according to a patient.

The cylindrical heat exchanger portion 260 is constructed of a cylindrical member of bellows type forming the outer surface thereof and an inner cylindrical member 275 accommodated inside the cylindrical member of bellows type. A heating medium communication chamber 276 is formed between the inner cylindrical member 275 and the cylindrical member of bellows type. The inner cylindrical member 275 is closed on a bottom surface thereof. The inner cylindrical member 275 has at the side surface thereof two openings 295a, 295b; the heating medium inlet port 273; and the heating medium outlet port 274. The interior of the inner cylindrical member 275 is divided into first and second inner chambers 277a, 277b. The heating medium inlet port 273 communicates with the first inner chamber 277a. The heating medium outlet port 274 communicates with the second inner chamber 277b. A heat exchange medium introduced into the cylindrical heat exchanger portion 260 from the heating medium inlet port 273 flows into the first inner chamber 277a, passes through the opening 295a, and flows through the space between the cylindrical member of bellows type and the inner cylindrical member 275, flows into the second inner chamber 277b from the opening 295b, and flows out from the heating medium outlet port 274.

The inner cylindrical member 275 is formed of synthetic resin or metal such as stainless steel, aluminum or the like. It is preferable that the heat exchanger portion 60 is cylindrical; that the outer diameter thereof is 20 - 100 mm; and that the length (effective length) thereof is 15 - 765 mm. The cylindrical member of bellow type and the inner cylindrical member constituting the cylindrical heat exchanger portion 260 may be integral with each other. In this case, preferably, the cylindrical heat exchanger portion 260 is formed of metal.

The housing body 263a may be cylindrical, polygonally cylindrical or elliptic in section. Preferably, the cylindrical housing body 263a is cylindrical. Preferably, the inner diameter of the cylindrical housing body 263a is 30 - 150 mm, and the length (effective length) thereof is 30 - 750 mm.

The cylindrical housing body 263a, the inner cylindrical member 275, a lid body, the first header 283, and the second header 282 can be formed of polyolefin (for example, polyethylene, polypropylene), ester resin (for example, polyethylene terephthalate), styrene resin (for example, polystyrene, MS resin, MBS resin), polycarbonate or the like.

It is preferable that the blood contact surface of the blood oxygenating apparatus is formed as an antithrombic surface. In particular, it is preferable that the delivery blood reserving portions 203a, 203b, the blood passages 235a, 35b, the first check valves 233a, 233b, the second check valves 234a, 234b have a blood contact surface, respectively.

The antithrombic surface may be formed by applying and fixing an antithrombin to the surface. Exemplary antithrombins are heparin, urokinase, HEMA-St-HEMA copolymers, and poly-HEMA.

Preferably, the antithrombic surface is formed by treating a substrate with ozone to form functional group-bearing oxides on the substrate surface and applying heparin to the surface so that an amino group of heparin forms a covalent bond with the functional group directly or through a coupling agent. This method permits heparin to be fixed on the blood wetting surface without the use of a solvent, minimizing a change of physical properties (e.g., flexibility, elasticity and strength) of the substrate presenting the blood wetting surface.

Through ozone treatment, oxides are formed on the substrate surface and high reactive functional groups such as aldehyde, ketone, and epoxy groups are generated in the oxides.

Amino groups of heparin can directly bond with these functional groups. For the reason of steric hindrance or other, introducing a spacer or coupling agent into these functional groups prior to fixation of heparin is easy and useful from the standpoint that the surface allows heparin to develop its activity. The coupling agents may be used alone or in admixture of two or more. Compounds having at least two aldehyde or epoxy groups are preferred.

Where two or more coupling agents are used, the preferred sequence is by first bonding a coupling agent (spacer coupling agent) in the form of a compound having at least two amino groups with the functional groups previously introduced in the substrate, to thereby introduce amino acid into the substrate, and thereafter bonding heparin to the substrate with the aid of a coupling agent (heparin-fixing coupling agent) in the form of a compound having at least two aldehyde or epoxy groups. In bonding heparin, the coupling agent is preferably admitted into the reaction system at the same time as or subsequent to heparin admission.

Especially when an amino group is introduced using a spacer coupling agent, it displays substantially the same reactivity as the amino group of heparin in the reaction system so that subsequent fixation of heparin to the substrate by the heparin-fixing coupling agent may take place more effectively.

Where the functional group of a coupling agent to directly bond with heparin or the functional group introduced into the substrate is an aldehyde group, it is preferable to use heparin in which some N-sulfate groups are desulfurized into primary amino groups.

It is preferable that the spacer coupling agent is one that forms a bond (covalent bond) with the functional group introduced on the substrate by ozone treatment and has at least two primary amino groups. Examples of the spacer coupling agent having at least two amino groups include polyethylene imine (PEI), polyethylene glycol diamine, ethylene diamine, and tetramethylene diamine.

Aldehyde and epoxy compounds are preferable as the coupling agent used for fixing heparin to the substrate. Exemplary of the aldehyde compound are glutaraldehyde, glyoxal, and succindialdehyde. Exemplary of the epoxy compound are polyethylene glycol diglycidyl ether, 1,4-butanediol diglycidyl ether, sorbitol diglycidyl ether, and glycerol diglycidyl ether.

Illustrative examples are Denacol EX-421, 521, 611, 612, 614, and 614B where the epoxy compound is sorbitol diglycidyl ether; Denacol EX-313 where the diepoxy compound is glycerol diglycidyl ether; Denacol EX-810, 811, 851, 821, 830, 832, 841, and 861 where the diepoxy compound is polyethylene glycol diglycidyl ether, all commercially available from Nagase Chemicals K.K. Denacol EX-313, 421, 512, 521, 810, 811, 821, and 851 are preferred when the difference of epoxy reactivity is considered. In the above-mentioned heparin fixation, coupling-off of heparin is minimized since the bond between polyethylene imine fixed to the substrate and glutaraldehyde and the bond between glutaraldehyde and heparin are both covalent bonds.

The blood delivery mechanism-equipped blood reservoir of the present invention comprises the blood storing portion; and the blood delivery mechanism including the delivery blood reserving portion communicating with the blood storing portion and reserving blood in an amount proportional to an amount of blood stored in the blood storing portion when the amount of the blood stored in the blood storing portion becomes equal to or less than a predetermined value and capable of flowing out blood stored in the delivery blood reserving portion when the delivery blood reserving portion is pressed from the outside and the blood delivery drive unit operating when the blood in the delivery blood reserving portion is delivered. The blood delivery mechanism-equipped blood reservoir can be connected with a sucking means for sucking an interior of the blood storing portion. The blood delivery mechanism-equipped blood reservoir further comprises the communication passage communicating an upper portion of the interior of the blood storing portion with the space formed between the delivery blood reserving portion formed inside the blood delivery mechanism and the blood delivering drive unit; and the communication mode selection function making it possible to select communication between the space of the blood delivery mechanism and the upper portion of the interior of the blood storing portion or communication between the space of the blood delivery mechanism and outside air.

According to the blood delivery mechanism-equipped blood reservoir, when the amount of blood stored in the blood delivery mechanism-equipped blood reservoir becomes smaller than a predetermined value, blood is delivered in proportion to a residual amount of blood stored in the blood reservoir. As the residual amount of blood becomes smaller, the amount of blood to be delivered approaches to zero. But it does not occur that the amount of the blood becomes zero. That is, the delivery of a small amount of blood is maintained. Thus, stagnation of blood does not occur in the region, of an extracorporeal blood circulation circuit, downstream from the blood reservoir. Further, even though the interior of the blood reservoir having a sucking device installed thereon has a negative-pressure state, the portion accommodating the blood storing member has also a negative-pressure state. This is because the portion accommodating the blood storing member is in communication with the blood reservoir or the sucking device. Thus, no pressure difference is generated between the blood reservoir and the portion accommodating the blood storing member. Therefore, when the sucking device is operated, it is possible to display the above-described operation of the blood storing member securely.

Further, in the blood delivery mechanism-equipped blood reservoir, the space between the delivery blood reserving portion formed in the interior of the blood delivery mechanism and the blood delivery drive unit does not communicate directly with the interior of the blood storing portion. Thus, movement of gas in the space which occurs by the operation of the blood delivery mechanism does not affect the interior of the blood storing portion and the blood storing portion does not suck outside air.

**Claims**

1. A blood oxygenating apparatus having a blood delivery mechanism-equipped blood reservoir comprising:

   a blood delivery mechanism-equipped blood reservoir having a blood storing portion and a blood reserving member having a delivery blood reserving portion communicating with said blood storing portion and reserving blood in an amount proportional to an amount of blood stored in said blood storing portion when the amount of said blood stored in blood storing portion becomes equal to or less than a predetermined value and capable of flowing out blood stored in said delivery blood reserving portion when said delivery blood reserving portion is pressed from outside; and
   an oxygenator connected with a blood outlet port of said blood delivery mechanism-equipped blood reservoir, wherein said blood outlet port of said oxygenator communicates with an upper end or its proximity of a blood chamber provided inside said oxygenator.

2. A blood oxygenating apparatus having a blood delivery mechanism-equipped blood reservoir according to claim 1, wherein said blood storing portion reserves a specified minimum amount of blood; and an upper end of said blood chamber provided inside said oxygenator is located at a position lower than a level of a surface of blood in the state where said specified minimum amount of blood is reserved in said blood storing portion.

3. A blood oxygenating apparatus having a blood delivery mechanism-equipped blood reservoir according to claim 1 or 2, wherein said blood delivery mechanism-equipped blood reservoir has a first check valve positioned between said blood storing portion and said blood reserving member for preventing blood from flowing toward said blood storing portion and a second check valve for preventing blood from flowing from a downstream side toward said blood reserving member.

4. A blood oxygenating apparatus having a blood delivery mechanism-equipped blood reservoir according to claim 2, wherein a level of a surface of blood reserved inside said blood storing portion is almost the same as a level of a lower end of said delivery blood reserving portion of said blood reserving member, with the specified minimum amount of blood is reserved inside said blood storing portion.

5. A blood oxygenating apparatus having a blood delivery mechanism-equipped blood reservoir according to claim 2 or 4, wherein blood delivery mechanism-equipped blood reservoir has an anti-foaming member or a foam-removing member; a lower end of said anti-foaming member or that of said foam-removing member are located below said blood surface, with the specified minimum amount of blood is reserved inside said blood storing portion.

6. A blood oxygenating apparatus having a blood delivery mechanism-equipped blood reservoir according to any one of claims 1 through 5, wherein said oxygenator has a cylindrical housing disposed substantially horizontally, a blood chamber formed inside said cylindrical housing and said blood outlet port is formed on an upper end of a side surface of said cylindrical housing.

7. A blood oxygenating apparatus having a blood delivery mechanism-equipped blood reservoir according to any one of claims 1 through 6, wherein said blood delivery mechanism-equipped blood reservoir has a blood reserving member-pressurizing portion operating in delivering blood reserved in said blood reserving member.

8. A blood oxygenating apparatus having a blood delivery mechanism-equipped blood reservoir according to claim 7, wherein two or more sets of said blood reserving member and said blood reserving member-pressurizing portion are provided.

9. A blood oxygenating apparatus having a blood delivery mechanism-equipped blood reservoir according to any one of claims 1 through 8, wherein said oxygenator has a cylindrical housing disposed substantially horizontally and a blood chamber formed inside said cylindrical housing; and said blood outlet port is so formed as to extend upward from substantially an upper end of a side surface of said cylindrical housing.

10. A blood oxygenating apparatus having a blood delivery mechanism-equipped blood reservoir according to any one of claims 1 through 9, wherein said oxygenator has a heat exchange function.

11. A bubble remover for a blood oxygenating circuit which is used by locating it downstream from an oxygenator of said blood oxygenating circuit comprising a blood delivery mechanism-equipped blood reservoir having a blood storing portion and a blood reserving member having a delivery blood reserving portion communicating with said blood storing portion and reserving blood in an amount proportional to an amount of blood stored in said blood storing portion when the amount of said blood stored in blood storing portion becomes equal to or less than a predetermined value and capable of flowing out blood stored in said delivery blood reserving portion when said delivery blood reserving portion is pressed from outside; and said oxygenator connected with a blood outlet port of said blood delivery mechanism-equipped blood reservoir,
wherein said bubble remover has the following relationship between an internal capacity (ml) and a maximum use flow (L/min):

$$\text{Internal capacity (ml)/Maximum use flow (L/min)} \leqq 15.$$

12. A bubble remover for a blood oxygenating circuit according to claim 11, wherein said bubble remover has a blood inlet port and a blood outlet port, and a each diameter of an opening of the blood inlet port and the blood outlet port is 8 mm or more.

13. A bubble remover for a blood oxygenating circuit according to claim 11 or 12, wherein an internal capacity of said bubble remover is 30 - 120 ml; and said bubble remover has a housing having a blood inlet port so formed as to project in a direction substantially tangent to an inner peripheral surface of said housing to allow blood introduced into said bubble remover to form a spiral current

14. A blood oxygenating circuit apparatus comprising:

a blood delivery mechanism-equipped blood reservoir having a blood storing portion and a blood reserving member having a delivery blood reserving portion communicating with said blood storing portion and reserving blood in an amount proportional to an amount of blood stored in said blood storing portion when the amount of said blood stored in blood storing portion becomes equal to or less than a predetermined value and capable of flowing out blood stored in said delivery blood reserving portion when said delivery blood reserving portion is pressed from outside;
an oxygenator connected with a blood outlet port of said blood delivery mechanism-equipped blood reservoir, and
a bubble remover located downstream from said oxygenator and having a relationship between an internal capacity (ml) and a maximum use flow (L/min):

$$\text{Internal capacity (ml)/Maximum use flow rate (L/min)} \leqq 15.$$

15. A blood oxygenating circuit apparatus according to claim 14, wherein said bubble remover has a blood inlet port and a blood outlet port, and each of diameter of an opening of the blood inlet port and the blood outlet port is 8 mm or more.

**16.** A blood oxygenating circuit apparatus according to claim 14 or 15, wherein an internal capacity of said bubble remover is 30 - 120 ml; and said bubble remover has a housing having a blood inlet port so formed as to project in a direction substantially tangent to an inner peripheral surface of said housing to allow blood introduced into said bubble remover to form a spiral current

**17.** A blood oxygenating circuit apparatus according to any one of claims 14 through 16, wherein said oxygenator has a blood chamber and a blood outlet port communicating with the vicinity of an upper end of said blood chamber.

**18.** A blood oxygenating circuit apparatus according to any one of claims 14 through 17, wherein said blood storing portion of said blood delivery mechanism-equipped blood reservoir reserves a specified minimum amount of blood; and an upper end of said blood chamber provided inside said oxygenator is located at a position lower than a level of a surface of blood in the state where said specified minimum amount of blood is reserved in said blood storing portion.

**19.** A blood oxygenating circuit apparatus according to any one of claims 14 through 18, wherein said blood delivery mechanism-equipped blood reservoir has a first check valve positioned between said blood storing portion and said blood reserving member for preventing blood from flowing toward said blood storing portion and a second check valve for preventing blood from flowing from a downstream side toward said blood reserving member.

**20.** A blood oxygenating circuit apparatus according to any one of claims 14 through 19, wherein said oxygenator has a cylindrical housing disposed substantially horizontally and a blood chamber formed inside said cylindrical housing; and said blood outlet port is so formed as to extend upward from substantially an upper end of a side surface of said cylindrical housing.

**21.** A blood oxygenating circuit apparatus according to any one of claims 14 through 20, wherein said blood delivery mechanism-equipped blood reservoir has a blood reserving member-pressurizing portion operating in delivering blood reserved in said blood reserving member.

**22.** A blood delivery mechanism-equipped blood reservoir comprising:

a blood storing portion; and
a blood delivery mechanism including a delivery blood reserving portion communicating with said blood storing portion and reserving blood in an amount proportional to an amount of blood stored in said blood storing portion when the amount of said blood stored in said blood storing portion becomes equal to or less than a predetermined value and capable of flowing out blood stored in said delivery blood reserving portion when said delivery blood reserving portion is pressed from the outside and a blood delivery drive unit operating when blood in said delivery blood reserving portion is delivered, wherein said blood delivery mechanism-equipped blood reservoir can be connected with a sucking means for sucking an interior of said blood storing portion;
said blood delivery mechanism-equipped blood reservoir further comprising:
a communication passage communicating an upper portion of an interior of said blood storing portion with a space formed inside said blood delivery mechanism and formed between said delivery blood reserving portion and said blood delivering drive unit; and
a communication mode selection function making it possible to select communication between said space of said blood delivery mechanism and said upper portion of said interior of said blood storing portion or communication between said space of said blood delivery mechanism and outside air.

**23.** A blood delivery mechanism-equipped blood reservoir according to claim 22, wherein said communication mode selection function makes it possible to select a state in which said space communicates with the outside air or a state in which said space communicates with only an upper portion of said interior of said blood storing portion without communication between said space of said blood delivery mechanism and said interior of said blood storing portion.

**24.** A blood delivery mechanism-equipped blood reservoir according to claim 22 or 23, wherein said delivery blood reserving portion has a deformable portion formed of a flexible material; and said blood delivery drive unit has a delivery blood reserving portion-pressurizing portion for deforming said deformable portion in a blood delivery operation to force out blood in said delivery blood reserving portion.

**25.** A blood delivery mechanism-equipped blood reservoir according to claim 22 or 23, wherein said blood delivery mechanism comprises a body portion; a flexible diaphragm which is accommodated in said body portion, with a periphery thereof retained; a flexible diaphragm pressurizing portion formed of an elastic sheet material; and a blood delivering fluid flow port used to expand and contract said flexible diaphragm pressurizing portion, wherein said space of said blood delivery mechanism is constructed of said body portion and a space between said diaphragm and said diaphragm pressurizing portion.

**26.** A blood delivery mechanism-equipped blood reservoir according to any one of claims 22 through 25, wherein one end of said communication passage is positioned at an upper portion of the interior of said blood storing portion; said communication mode selection function has a first cap member and a second cap member to be arbitrarily selected; said blood delivery mechanism-equipped blood reservoir has a cap member installing portion; said first cap member has a tubular portion whose one end can be connected with one end of said communication passage and whose other end is open and located outside said blood storing portion when said first cap member is installed; and said second cap member has an installing portion which is installed on said cap member installing portion substantially airtightly and a tubular portion which extends outward and an end of which constitutes a sucking means connection portion.

**27.** A blood delivery mechanism-equipped blood reservoir according to claim 26, wherein said first cap member has an installing portion covering an opening of said the cap member installing portion and not preventing communication between said interior of said blood storing portion and outside air through said cap member installing portion.

**28.** A blood delivery mechanism-equipped blood reservoir according to any one of claims 22 through 25,

wherein said blood delivery mechanism-equipped blood reservoir has a communication mode selection opening,

said communication mode selection function comprises a tubular portion whose one end can be connected with one end of said communication passage and whose other end is located outside said blood storing portion and constituting said sucking means connection portion, a fittable portion provided on a portion of said tubular portion and airtightly fittable into said communication mode selection opening, and a communication mode selection member having a side hole located between said one end of said tubular portion and said fittable portion and communicating said tubular portion and outside with each other,

said communication mode selection member is movable, with said communication mode selection member in connection with said communication passage,

said interior of said blood storing portion communicates with outside air directly in a state where said fittable portion of said communication mode selection member is not fitted in said communication mode selection opening,

said interior of said blood storing portion communicates with an interior of said tubular portion through said side hole in a state where said fittable portion of said communication mode selection member is not airtightly fitted in said communication mode selection opening.

**29.** A blood delivery mechanism-equipped blood reservoir according to any one of claims 22 through 25, wherein said communication mode selection function has a change-over member having an operation portion and an outside air communication port; by operating said operation portion, it is possible to select a state in which said space of said blood delivery mechanism communicates with outside air or a state in which said space communicates with only said upper portion of said blood storing portion.

**30.** A blood delivery mechanism-equipped blood reservoir according to any one of claims 22 through 25, wherein said communication mode selection function has a communication mode change-over member having an operation portion and an outside air communication port/sucking portion; and by operating said operation portion, it is possible to select a state in which said space of said blood delivery mechanism communicates with outside air or a state in which said space of said blood delivery mechanism and said upper portion of said blood storing portion communicate with said outside air communication port/sucking portion.

**31.** A blood delivery mechanism-equipped blood reservoir according to any one of claims 22 through 30, wherein two or more sets of said blood delivery mechanisms are provided.

# FIG. 1

# FIG. 2

# FIG. 3

FIG. 4

# FIG. 5

# FIG. 6

# FIG. 7

# FIG. 8

# FIG. 9

# FIG. 10

EP 0 987 035 A2

# FIG. 11

46

# FIG. 12

# FIG. 13

# FIG. 14

# FIG. 15

# FIG. 16

# FIG. 17

# FIG. 18

# FIG. 19

# FIG. 20

# FIG. 21

# FIG. 22

256

256a

256b

# FIG. 23

256a

256

256b

D

D

# FIG. 24

256

256a

256c

256b

# FIG. 25

# FIG. 26

# FIG. 27

# FIG. 28

# FIG. 29

# FIG. 30

# FIG. 31

# FIG. 32

# FIG. 33

# FIG. 34